(19) Europäisches Patentamt
European Patent Office
Office européen des brevets

(11) EP 2 415 876 B1

(12) EUROPEAN PATENT SPECIFICATION

(45) Date of publication and mention of the grant of the patent:
**02.08.2017 Bulletin 2017/31**

(21) Application number: **10758403.9**

(22) Date of filing: **15.03.2010**

(51) Int Cl.:
***C12Q 1/14*** *(2006.01)* ***A61K 31/7088*** *(2006.01)*
***A61K 38/00*** *(2006.01)* ***A61K 45/00*** *(2006.01)*
***A61P 7/02*** *(2006.01)* ***A61P 7/04*** *(2006.01)*
***C07K 16/12*** *(2006.01)* ***G01N 33/569*** *(2006.01)*
***C12N 15/09*** *(2006.01)*

(86) International application number:
**PCT/JP2010/054364**

(87) International publication number:
**WO 2010/113627 (07.10.2010 Gazette 2010/40)**

(54) **HIGHLY SENSITIVE DETECTION METHOD FOR HIGHLY VIRULENT ORAL CAVITY BACTERIA**

HOCHSENSITIVES NACHWEISVERFAHREN FÜR HOCHVIRULENTE BAKTERIEN DER MUNDHÖHLE

PROCÉDÉ DE DÉTECTION HAUTEMENT SENSIBLE POUR DES BACTÉRIES DE CAVITÉ BUCCALE À VIRULENCE ÉLEVÉE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **31.03.2009 JP 2009088239**

(43) Date of publication of application:
**08.02.2012 Bulletin 2012/06**

(73) Proprietor: **National University Corporation Hamamatsu University School of Medicine**
**Hamamatsu-shi**
**Shizuoka 431-3192 (JP)**

(72) Inventors:
- **UMEMURA, Kazuo**
**Hamamatsu-shi**
**Shizuoka 431-3192 (JP)**
- **HOKAMURA, Kazuya**
**Hamamatsu-shi**
**Shizuoka 431-3192 (JP)**
- **NAKANO, Kazuhiko**
**Suita-shi**
**Osaka 565-0871 (JP)**
- **OOSHIMA, Takashi**
**Suita-shi**
**Osaka 565-0871 (JP)**
- **NOMURA, Ryota**
**Suita-shi**
**Osaka 565-0871 (JP)**
- **WADA, Koichiro**
**Suita-shi**
**Osaka 565-0871 (JP)**

(74) Representative: **Lecca, Patricia S.**
**Cabinet Lecca**
**21, rue de Fécamp**
**75012 Paris (FR)**

(56) References cited:
**EP-A1- 1 710 310 WO-A2-97/43314**
**GB-A- 2 404 981**

- **SATO Y ET AL: "Streptococcus mutans strains harboring collagen-binding adhesin", JOURNAL OF DENTAL RESEARCH, INTERNATIONAL ASSOCIATION FOR DENTAL RESEARCH, US, vol. 83, no. 7, 1 July 2004 (2004-07-01), pages 534-539, XP009163533, ISSN: 0022-0345, DOI: 10.1177/154405910408300705**
- **DATABASE UniProt [Online] 24 March 2009 (2009-03-24), "SubName: Full=Collagen-binding adhesin;", XP002685730, retrieved from EBI accession no. UNIPROT:B9A878 Database accession no. B9A878**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- R. NOMURA ET AL: "Molecular and clinical analyses of the gene encoding the collagen-binding adhesin of Streptococcus mutans", JOURNAL OF MEDICAL MICROBIOLOGY, vol. 58, no. 4, 1 April 2009 (2009-04-01), pages 469-475, XP55040559, ISSN: 0022-2615, DOI: 10.1099/jmm.0.007559-0
- NAHO TANIGUCHI ET AL.: 'Mouse No Shukketsu Model ni Okeru Streptococcus mutans no Byogensei no Kento' THE JAPANESE JOURNAL OF PEDODONTICS vol. 47, no. 2, 15 April 2009, page 392, XP008154480
- NAKANO K ET AL.: 'Protein Antigen in Serotype k Streptococcus mutans Clinical Isolates' J DENT RES vol. 87, no. 10, 2008, pages 964 - 968, XP008154482
- NAKANO K. ET AL.: 'Streptococcus mutans Clonal Variation Revealed by Multilocus Sequence Typing' JOURNAL OF CLINICAL MICROBIOLOGY vol. 45, no. 8, 2007, pages 2616 - 2625, XP008154484
- KAZUYA HOKAMURA ET AL.: 'Kokunai Saikin S. mutans wa No Shukketsu o Zoo saseru' DAI 21 KAI THE JAPANESE SOCIETY OF CEREBRAL BLOOD FLOW AND METABOLISM SOKAI PROGRAM - SHOROKUGO vol. 21, no. 1, 01 October 2009, page 120, XP008154510
- NAKANO K. ET AL.: 'Molecular characterization of Streptococcus mutans strains containing the cnm gene encoding a collagen-binding adhesin' ARCHIVES OF ORAL BIOLOGY vol. 55, January 2010, pages 34 - 39, XP026817270
- W.William Wilson ET AL: "Status of methods for assessing bacterial cell surface charge properties based on zeta potential measurements", Journal of Microbiological Methods, vol. 43, no. 3, 1 January 2001 (2001-01-01), pages 153-164, XP55179472, ISSN: 0167-7012, DOI: 10.1016/S0167-7012(00)00224-4
- WESTERGREN ET AL: "Ionic interaction of oral streptococcal bacteria studied by partition in an aqueous polymer two-phase system", ARCHIVES OF ORAL BIOLOGY, PERGAMON PRESS, OXFORD, GB, vol. 26, no. 12, 1 January 1981 (1981-01-01), pages 1035-1039, XP026167048, ISSN: 0003-9969, DOI: 10.1016/0003-9969(81)90114-X [retrieved on 1981-01-01]
- Fred R. Sattler ET AL: "Impaired hemostasis caused by beta-lactam antibiotics", The American Journal of Surgery, vol. 155, no. 5, 1 May 1988 (1988-05-01), pages 30-39, XP55179446, ISSN: 0002-9610, DOI: 10.1016/S0002-9610(88)80209-5

SEQUENCE LISTING

<110> Hamamatsu University School of Medicine

<120> METHOD FOR DETECTING HIGH-PATHOGENIC ORAL BACTERIUM

<130> PCT2402HI

<150> JP2009-088239
<151> 2009-03-31

<160> 32

<170> PatentIn version 3.1

<210> 1
<211> 1565
<212> PRT
<213> Streptococcus mutans

<400> 1

```
Met Lys Val Lys Lys Thr Tyr Gly Phe Arg Lys Ser Lys Ile Ser Lys
1               5                   10                  15

Thr Leu Cys Gly Ala Val Leu Gly Thr Val Ala Ala Val Ser Val Ala
            20                  25                  30

Gly Gln Lys Val Phe Ala Asp Glu Thr Thr Thr Thr Ser Asp Val Asp
        35                  40                  45

Thr Lys Val Val Gly Thr Gln Thr Gly Asn Pro Ala Thr Asn Leu Pro
    50                  55                  60

Glu Ala Gln Gly Ser Ala Ser Lys Glu Ala Glu Gln Ser Gln Thr Lys
65                  70                  75                  80

Leu Glu Arg Gln Met Val His Thr Ile Glu Val Pro Lys Thr Asp Leu
                85                  90                  95

Asp Gln Ala Ala Lys Asp Ala Lys Ser Ala Gly Val Asn Val Val Gln
            100                 105                 110

Asp Ala Asp Val Asn Lys Gly Thr Val Lys Thr Pro Glu Glu Ala Val
        115                 120                 125

Gln Lys Glu Thr Glu Ile Lys Glu Asp Tyr Thr Lys Gln Ala Glu Asp
    130                 135                 140

Ile Lys Lys Thr Thr Asp Gln Tyr Lys Ser Asp Val Ala Ala His Glu
145                 150                 155                 160

Ala Glu Val Ala Lys Ile Lys Ala Lys Asn Gln Ala Thr Lys Glu Gln
                165                 170                 175
```

Tyr Glu Lys Asp Met Ala Ala His Lys Ala Glu Val Glu Arg Ile Asn
180 185 190

Ala Ala Asn Ala Ala Ser Lys Thr Ala Tyr Glu Ala Lys Leu Ala Gln
195 200 205

Tyr Gln Ala Asp Leu Ala Ala Val Gln Lys Thr Asn Ala Ala Asn Gln
210 215 220

Ala Ala Tyr Gln Lys Ala Leu Ala Ala Tyr Gln Ala Glu Leu Lys Arg
225 230 235 240

Val Gln Glu Ala Asn Ala Ala Ala Lys Ala Ala Tyr Asp Thr Ala Val
245 250 255

Ala Ala Asn Asn Ala Lys Asn Thr Glu Ile Ala Ala Ala Asn Glu Glu
260 265 270

Ile Arg Lys Arg Asn Ala Thr Ala Lys Ala Glu Tyr Glu Thr Lys Leu
275 280 285

Ala Gln Tyr Gln Ala Glu Leu Lys Arg Val Gln Glu Ala Asn Ala Ala
290 295 300

Asn Glu Ala Asp Tyr Gln Ala Lys Leu Thr Ala Tyr Gln Thr Glu Leu
305 310 315 320

Ala Arg Val Gln Lys Ala Asn Ala Asp Ala Lys Ala Thr Tyr Glu Ala
325 330 335

Ala Val Ala Ala Asn Asn Ala Lys Asn Ala Ala Leu Thr Ala Glu Asn
340 345 350

Thr Ala Ile Lys Gln Arg Asn Glu Asn Ala Lys Ala Thr Tyr Glu Ala
355 360 365

Ala Leu Lys Gln Tyr Glu Ala Asp Leu Ala Ala Val Lys Lys Ala Asn
370 375 380

Ala Ala Asn Glu Ala Asp Tyr Gln Ala Lys Leu Thr Ala Tyr Gln Thr
385 390 395 400

Glu Leu Ala Arg Val Gln Lys Ala Asn Ala Asp Ala Lys Ala Ala Tyr
405 410 415

Glu Ala Ala Val Ala Ala Asn Asn Ala Ala Asn Ala Ala Leu Thr Ala
420 425 430

Glu Asn Thr Ala Ile Lys Lys Arg Asn Ala Asp Ala Lys Ala Asp Tyr
435 440 445

Glu Ala Lys Leu Ala Lys Tyr Gln Ala Asp Leu Ala Lys Tyr Gln Lys
450 455 460

Asp Leu Ala Asp Tyr Pro Val Lys Leu Lys Ala Tyr Glu Asp Glu Gln
465 470 475 480

Thr Ser Ile Lys Ala Ala Leu Ala Glu Leu Glu Lys His Lys Asn Glu
485 490 495

Asp Gly Asn Leu Thr Glu Pro Ser Ala Gln Asn Leu Val Tyr Asp Leu
500 505 510

Glu Pro Asn Ala Asn Leu Ser Leu Thr Thr Asp Gly Lys Phe Leu Lys
515 520 525

Ala Ser Ala Val Asp Asp Ala Phe Ser Lys Ser Thr Ser Lys Ala Lys
530 535 540

Tyr Asp Gln Lys Ile Leu Gln Leu Asp Asp Leu Asp Ile Thr Asn Leu
545 550 555 560

Glu Gln Ser Asn Asp Val Ala Ser Ser Met Glu Leu Tyr Gly Asn Phe
565 570 575

Gly Asp Lys Ala Gly Trp Ser Thr Thr Val Ser Asn Asn Ser Gln Val
580 585 590

Lys Trp Gly Ser Val Leu Leu Glu Arg Gly Gln Ser Ala Thr Ala Thr
595 600 605

Tyr Thr Asn Leu Gln Asn Ser Tyr Tyr Asn Gly Lys Lys Ile Ser Lys
610 615 620

Ile Val Tyr Lys Tyr Thr Val Asp Pro Lys Ser Lys Phe Gln Gly Gln
625 630 635 640

Lys Val Trp Leu Gly Ile Phe Thr Asp Pro Thr Leu Gly Val Phe Ala
645 650 655

Ser Ala Tyr Thr Gly Gln Val Glu Lys Asn Thr Ser Ile Phe Ile Lys
660 665 670

Asn Glu Phe Thr Phe Tyr His Glu Asp Glu Lys Pro Ile Asn Phe Asp
675 680 685

Asn Ala Leu Leu Ser Val Thr Ser Leu Asn Arg Glu His Asn Ser Ile

```
        690                 695                 700

Glu Met Ala Lys Asp Tyr Ser Gly Lys Phe Val Lys Ile Ser Gly Ser
705                 710                 715                 720

Ser Ile Gly Glu Lys Asn Gly Met Ile Tyr Ala Thr Asp Thr Leu Asn
                725                 730                 735

Phe Lys Gln Gly Glu Gly Gly Ser Arg Trp Thr Met Tyr Lys Asn Ser
            740                 745                 750

Gln Ala Gly Ser Gly Trp Asp Ser Ser Asp Ala Pro Asn Ser Trp Tyr
        755                 760                 765

Gly Ala Gly Ala Ile Lys Met Ser Gly Pro Asn Asn His Val Thr Val
    770                 775                 780

Gly Ala Thr Ser Ala Thr Asn Val Met Pro Val Ser Asp Met Pro Val
785                 790                 795                 800

Val Pro Gly Lys Asp Asn Thr Asp Gly Lys Lys Pro Asn Ile Trp Tyr
                805                 810                 815

Ser Leu Asn Gly Lys Ile Arg Ala Val Asn Val Pro Lys Val Thr Lys
            820                 825                 830

Glu Lys Pro Thr Pro Pro Val Lys Pro Thr Ala Pro Thr Lys Pro Thr
        835                 840                 845

Tyr Glu Thr Glu Lys Pro Leu Lys Pro Ala Pro Val Ala Pro Asn Tyr
    850                 855                 860

Glu Lys Glu Pro Thr Pro Pro Thr Arg Thr Pro Asp Gln Ala Glu Pro
865                 870                 875                 880

Asn Lys Pro Thr Pro Pro Thr Tyr Glu Thr Glu Lys Pro Leu Glu Pro
                885                 890                 895

Ala Pro Val Glu Pro Ser Tyr Glu Ala Glu Pro Thr Pro Pro Thr Arg
            900                 905                 910

Thr Pro Asp Gln Ala Glu Pro Asn Lys Pro Thr Pro Pro Thr Tyr Glu
        915                 920                 925

Thr Glu Lys Pro Leu Glu Pro Ala Pro Val Glu Pro Ser Tyr Glu Ala
    930                 935                 940

Glu Pro Thr Pro Pro Thr Pro Thr Pro Asp Gln Pro Glu Pro Asn Lys
945                 950                 955                 960
```

Pro Val Glu Pro Thr Tyr Glu Val Ile Pro Thr Pro Pro Thr Asp Pro
965 970 975

Val Tyr Gln Asp Leu Pro Thr Pro Pro Ser Asp Pro Thr Val His Phe
980 985 990

His Tyr Phe Lys Leu Ala Val Gln Pro Gln Val Asn Lys Glu Ile Arg
995 1000 1005

Asn Asn Asn Asp Ile Asn Ile Asp Arg Thr Leu Val Ala Lys Gln
1010 1015 1020

Ser Val Val Lys Phe Gln Leu Lys Thr Ala Asp Leu Pro Ala Gly
1025 1030 1035

Arg Asp Glu Thr Thr Ser Phe Val Leu Val Asp Pro Leu Pro Ser
1040 1045 1050

Gly Tyr Gln Phe Asn Pro Glu Ala Thr Lys Ala Ala Ser Pro Gly
1055 1060 1065

Phe Asp Val Thr Tyr Asp Asn Ala Thr Asn Thr Val Thr Phe Lys
1070 1075 1080

Ala Thr Ala Ala Thr Leu Ala Thr Phe Asn Ala Asp Leu Thr Lys
1085 1090 1095

Ser Val Ala Thr Ile Tyr Pro Thr Val Val Gly Gln Val Leu Asn
1100 1105 1110

Asp Gly Ala Thr Tyr Lys Asn Asn Phe Thr Leu Thr Val Asn Asp
1115 1120 1125

Ala Tyr Gly Ile Lys Ser Asn Val Val Arg Val Thr Thr Pro Gly
1130 1135 1140

Lys Pro Asn Asp Pro Asp Asn Pro Asn Asn Asn Tyr Ile Lys Pro
1145 1150 1155

Thr Lys Val Asn Lys Asn Glu Asn Gly Val Val Ile Asp Gly Lys
1160 1165 1170

Thr Val Leu Ala Gly Ser Thr Asn Tyr Tyr Glu Leu Thr Trp Asp
1175 1180 1185

Leu Asp Gln Tyr Lys Asn Asp Arg Ser Ser Ala Asp Thr Ile Gln
1190 1195 1200

```
Lys Gly Phe Tyr Tyr Val Asp Asp Tyr Pro Glu Glu Ala Leu Glu
    1205                1210                1215

Leu Arg Gln Asp Leu Val Lys Ile Thr Asp Ala Asn Gly Asn Glu
    1220                1225                1230

Val Thr Gly Val Ser Val Asp Asn Tyr Thr Asn Leu Glu Ala Ala
    1235                1240                1245

Pro Gln Glu Ile Arg Asp Val Leu Ser Lys Ala Gly Ile Arg Pro
    1250                1255                1260

Lys Gly Ala Phe Gln Ile Phe Arg Ala Asp Asn Pro Arg Glu Phe
    1265                1270                1275

Tyr Asp Thr Tyr Val Lys Thr Gly Ile Asp Leu Lys Ile Val Ser
    1280                1285                1290

Pro Met Val Val Lys Lys Gln Met Gly Gln Thr Gly Gly Ser Tyr
    1295                1300                1305

Glu Asn Gln Ala Tyr Gln Ile Asp Phe Gly Asn Gly Tyr Ala Ser
    1310                1315                1320

Asn Ile Val Ile Asn Asn Val Pro Lys Ile Asn Pro Lys Lys Asp
    1325                1330                1335

Val Thr Leu Thr Leu Asp Pro Ala Asp Thr Asn Asn Val Asp Gly
    1340                1345                1350

Gln Thr Ile Pro Leu Asn Thr Val Phe Asn Tyr Arg Leu Ile Gly
    1355                1360                1365

Gly Ile Ile Pro Ala Asn His Ser Glu Glu Leu Phe Glu Tyr Asn
    1370                1375                1380

Phe Tyr Asp Asp Tyr Asp Gln Thr Gly Asp His Tyr Thr Gly Gln
    1385                1390                1395

Tyr Lys Val Phe Ala Lys Val Asp Ile Thr Leu Lys Asn Gly Val
    1400                1405                1410

Ile Ile Lys Ser Gly Thr Glu Leu Thr Gln Tyr Thr Thr Ala Glu
    1415                1420                1425

Val Asp Thr Thr Lys Gly Ala Ile Thr Ile Lys Phe Lys Glu Ala
    1430                1435                1440
```

```
Phe Leu Arg Ser Val Ser Ile Asp Ser Ala Phe Gln Ala Glu Ser
    1445                1450                1455

Tyr Ile Gln Met Lys Arg Ile Ala Val Gly Thr Phe Glu Asn Thr
    1460                1465                1470

Tyr Ile Asn Thr Val Asn Gly Val Thr Tyr Ser Ser Asn Thr Val
    1475                1480                1485

Lys Thr Thr Thr Pro Glu Asp Pro Ala Asp Pro Thr Asp Pro Gln
    1490                1495                1500

Asp Pro Ser Ser Pro Arg Thr Ser Thr Val Ile Ile Tyr Lys Pro
    1505                1510                1515

Gln Ser Thr Ala Tyr Gln Pro Ser Ser Val Gln Glu Thr Leu Pro
    1520                1525                1530

Asn Thr Gly Val Thr Asn Asn Ala Tyr Met Pro Leu Leu Gly Ile
    1535                1540                1545

Ile Gly Leu Val Thr Ser Phe Ser Leu Leu Gly Leu Lys Ala Lys
    1550                1555                1560

Lys Asp
    1565
```

<210> 2
<211> 4698
<212> DNA
<213> Streptococcus mutans

<400> 2

atgaaagtca aaaaaactta cggttttcgt aaaagtaaaa ttagtaaaac actgtgtggt 60
gctgttctag gaacagtagc agcagtctct gtagcaggac aaaaggtttt tgccgatgaa 120
acgaccacta ctagtgatgt agatactaaa gtagttggaa cacaaactgg aaatccagcg 180
accaatttgc cagaggctca agggagtgcg agtaaggaag ctgaacaaag tcaaaccaag 240
ctggagagac aaatggttca taccattgaa gtacctaaaa ctgatcttga tcaagcagca 300
aaagatgcta agtctgctgg tgtcaatgtt gtccaagatg ccgatgttaa taaaggaact 360
gttaaaacac ctgaagaagc agtccaaaaa gaaactgaaa ttaaagaaga ttacacaaaa 420
caagctgagg atattaagaa gacaacagat caatataaat cggatgtagc tgctcatgag 480
gcagaagttg ctaaaatcaa agctaaaaat caggcaacta aagaacagta tgaaaaagat 540
atggcagctc ataaagccga ggttgaacgc attaatgctg caaatgctgc cagtaaaaca 600
gcttatgaag ctaaattggc tcaatatcaa gcagatttag cagccgttca aaaaaccaat 660
gctgccaatc aagcagccta tcaaaaagcc cttgctgctt atcaggctga actgaaacgt 720

```
gttcaggaag ctaatgcagc cgccaaagcc gcttatgata ctgctgtagc agcaaataat    780
gccaaaaata cagaaattgc cgctgccaat gaagaaatta gaaaacgcaa tgcaacggcc    840
aaagctgaat atgagactaa gttagctcaa tatcaagctg aactaaagcg tgttcaggaa    900
gctaatgccg caaacgaagc agactatcaa gctaaattga ccgcctatca aacagagctt    960
gctcgtgttc aaaaagccaa tgcggatgct aaagcgacct atgaagcagc tgtagcagca   1020
aataatgcca aaaatgcggc actcacagct gaaaatactg caattaagca acgcaatgag   1080
aatgctaagg cgacttatga agctgcactc aagcaatatg aggccgattt ggcagcggtg   1140
aaaaaagcta atgccgcaaa cgaagcagac tatcaagcta aattgaccgc ctatcaaaca   1200
gagctcgctc gcgttcaaaa agccaatgcg gatgctaaag cggcctatga agcagctgta   1260
gcagcaaata atgccgcaaa tgcagcgctc acagctgaaa atactgcaat taagaagcgc   1320
aatgcggatg ctaaagctga ttacgaagca aaacttgcta agtatcaagc agatcttgcc   1380
aaatatcaaa aagatttagc agactatcca gttaagttaa aggcatacga agatgaacaa   1440
acttctatta aagctgcact ggcagaactt gaaaaacata aaaatgaaga cggaaactta   1500
acagaaccat ctgctcaaaa tttggtctat gatcttgagc caaatgcgaa cttatctttg   1560
acaacagatg ggaagttcct taaggcttct gctgtggatg atgcttttag caaaagcact   1620
tcaaaagcaa aatatgacca aaaaattctt caattagatg atctagatat cactaactta   1680
gaacaatcta atgatgttgc ttcttctatg gagctttatg ggaattttgg tgataaagct   1740
ggctggtcaa cgacagtaag caataactca caggttaaat ggggatcggt acttttagag   1800
cgcggtcaaa gcgcaacagc tacatacact aacctgcaga attcttatta caatggtaaa   1860
aagatttcta aaattgtcta caagtataca gtggacccta agtccaagtt tcaaggtcaa   1920
aaggtttggt taggtatttt taccgatcca actttaggtg tttttgcttc tgcttataca   1980
ggtcaagttg aaaaaaacac ttctattttt attaaaaatg aattcacttt ctatcacgaa   2040
gatgaaaaac caattaattt tgataatgcc cttctctcag tgacttctct taaccgtgaa   2100
cataactcta ttgagatggc taaagattat agtggtaaat ttgtcaaaat ctctggttca   2160
tctattggtg aaaagaatgg catgatttat gctacagata ctcttaactt taaacagggt   2220
gaaggtggct ctcgctggac tatgtataaa aatagtcaag ctggttcagg atgggatagt   2280
tcagatgcgc cgaattcttg gtatggagca ggggctatta aaatgtctgg tccgaataac   2340
catgttactg taggagcaac ttctgcaaca aatgtaatgc cagtttctga catgcctgtt   2400
gttcctggta aggacaatac tgatggcaaa aaaccaaata tttggtattc tttaaatggt   2460
aaaatccgtg cggttaatgt tcctaaagtt actaaggaaa aacccacacc tccggttaaa   2520
ccaacagctc caactaaacc aacttatgaa acagaaaagc cattaaaacc ggcaccagta   2580
gctccaaatt atgaaaagga gccaacaccg ccgacaagga caccggatca agcagagcca   2640
```

aacaaaccca caccgccgac ctatgaaaca gaaaagccgt tggagccagc acctgttgag 2700
ccaagctatg aagcagagcc aacaccgccg acaaggacac cggatcaggc agagccaaat 2760
aaacccacac cgccgaccta tgaaacagaa aagccgttgg agccagcacc tgttgagcca 2820
agctatgaag cagagccaac gccaccgaca ccaacaccag atcaaccaga accaaacaaa 2880
cctgttgagc caacttatga ggttattcca acaccgccga ctgatcctgt ttatcaagat 2940
cttccaacac ctccatctga tccaactgtt catttccatt actttaaact agctgttcag 3000
ccgcaggtta acaaagaaat tagaaacaat aacgatatta atattgacag aactttggtg 3060
gctaaacaat ctgttgttaa gttccagctg aagacagcag atctccctgc tggacgtgat 3120
gaaaccactt cctttgtctt ggtagatccc ctgccatctg gttatcaatt taatcctgaa 3180
gctacaaaag ctgcaagccc tggctttgat gtcacttatg ataatgcaac taatacagtc 3240
accttcaagg caactgcagc aactttggct acgtttaatg ctgatttgac taagtcagtg 3300
gcaacgattt atccaacagt ggtcggacaa gttcttaatg atggcgcaac ttataagaat 3360
aatttcacgc tcacagtcaa tgatgcttat ggcattaaat ccaatgttgt tcgggtgaca 3420
actcctggta aaccaaatga tccagataat ccaaataata attatattaa accaactaag 3480
gttaataaaa acgaaaatgg cgttgttatt gatggtaaaa cagttcttgc cggttcaacg 3540
aattattatg agctaacttg ggatttggat caatataaaa acgaccgctc ttcagcagat 3600
accattcaaa aaggatttta ctatgtagat gattatccag aagaagcgct tgaattgcgt 3660
caggatttag tgaagattac agatgctaat ggtaatgaag ttactggtgt tagtgtggat 3720
aattatacta atcttgaagc agcccctcaa gaaattagag atgttctttc taaggcagga 3780
attagaccta aaggtgcttt ccaaattttc cgtgccgata atccaagaga attttatgat 3840
acttatgtca aaactggaat tgatttgaag attgtatcac caatggttgt taaaaaacaa 3900
atgggacaaa caggcggcag ttatgaaaat caagcttacc aaattgactt tggtaatggt 3960
tatgcatcaa atatcgttat caataatgtt cctaagatta accctaagaa agatgtgacc 4020
ttaacacttg atccggctga tacaaataat gttgatggtc agactattcc acttaataca 4080
gtctttaatt accgtttgat tggtggcatt atccctgcaa atcactcaga agaactcttt 4140
gaatacaatt tctatgatga ttatgatcaa acaggagatc actatactgg tcagtataaa 4200
gtttttgcca aggttgatat cactcttaaa aacggtgtta ttatcaagtc aggtactgag 4260
ttaactcagt atacgacagc ggaagttgat accactaaag gtgctatcac aattaagttc 4320
aaggaagcct ttctgcgttc tgtttcaatt gattcagcct tccaagctga aagttatatc 4380
caaatgaaac gtattgcggt tggtactttt gaaaatacct atattaatac tgtcaatggg 4440
gtaacttaca gttcaaatac agtgaaaaca actactcctg aggatcctgc agaccctact 4500
gatccgcaag atccatcatc accgcggact tcaactgtaa ttatctacaa acctcaatca 4560
actgcttatc agccaagctc tgttcaagaa acattaccaa atacgggagt aacaaacaat 4620

gcttatatgc ctttacttgg tattattggc ttagttacta gttttagttt gcttggttta 4680

aaggctaaga aagattga 4698

<210> 3
<211> 555
<212> PRT
<213> Streptococcus mutans

<400> 3

```
Met Lys Arg Lys Gly Leu Arg Arg Leu Leu Lys Phe Phe Gly Thr Val
1               5                   10                  15

Ala Ile Ile Leu Pro Met Phe Phe Ile Ala Leu Thr Lys Ala Gln Ala
            20                  25                  30

Ser Asp Val Ser Ser Asn Ile Ser Ser Leu Thr Val Ser Pro Thr Gln
        35                  40                  45

Ile Asn Asp Gly Gly Lys Thr Thr Val Arg Phe Glu Phe Asp Glu His
    50                  55                  60

Ala Gln Asn Ile Lys Ala Gly Asp Thr Ile Thr Val Asn Trp Gln Asn
65                  70                  75                  80

Ser Gly Thr Val Arg Gly Thr Gly Tyr Thr Lys Thr Ile Lys Leu Glu
                85                  90                  95

Val Gln Gly Lys Tyr Val Gly Asp Leu Val Val Thr Gln Asp Lys Ala
            100                 105                 110

Val Val Thr Phe Asn Asp Ser Ile Thr Gly Leu Gln Asn Ile Thr Gly
        115                 120                 125

Trp Gly Glu Phe Glu Ile Glu Gly Arg Asn Phe Thr Asp Thr Thr Thr
    130                 135                 140

Gly Asn Thr Gly Ser Phe Gln Val Thr Ser Gly Gly Lys Thr Ala Glu
145                 150                 155                 160

Val Thr Val Val Lys Ser Ala Ser Gly Thr Thr Gly Val Phe Tyr Tyr
                165                 170                 175

Lys Thr Gly Asp Met Gln Thr Asp Asp Thr Asn His Val Arg Trp Phe
            180                 185                 190

Leu Asn Ile Asn Asn Glu Asn Ala Tyr Val Asp Ser Asp Ile Arg Ile
        195                 200                 205
```

```
Glu Asp Asp Ile Gln Ser Gly Gln Thr Leu Asp Ile Asp Ser Phe Asp
    210                 215                 220

Ile Thr Val Asn Gly Ser Glu Ser Tyr His Gly Gln Glu Gly Ile Asn
225                 230                 235                 240

Gln Leu Ala Gln Arg Tyr Gly Ala Thr Ile Ser Ala Asp Pro Ala Ser
                245                 250                 255

Gly His Ile Ser Val Tyr Ile Pro Gln Gly Tyr Ala Ser Leu Asn Arg
            260                 265                 270

Phe Ser Ile Met Tyr Leu Thr Lys Val Asp Asn Pro Asp Gln Lys Thr
        275                 280                 285

Phe Glu Asn Asn Ser Lys Ala Trp Tyr Lys Glu Asn Gly Lys Asp Ala
    290                 295                 300

Val Asp Gly Lys Glu Phe Asn His Ser Val Ala Asn Val Asn Ala Ala
305                 310                 315                 320

Gly Gly Val Asp Gly Arg Thr Thr Thr Thr Thr Glu Lys Pro Thr Thr
                325                 330                 335

Thr Thr Glu Ala Pro Thr Thr Thr Glu Thr Pro Thr Thr Thr Glu Ala
            340                 345                 350

Pro Thr Thr Glu Ala Pro Thr Thr Thr Glu Ala Pro Thr Thr Thr Glu
        355                 360                 365

Ala Pro Thr Thr Thr Glu Ala Pro Thr Thr Thr Glu Ala Pro Thr Thr
    370                 375                 380

Thr Glu Ala Pro Thr Thr Thr Glu Ala Pro Thr Thr Thr Glu Ala Pro
385                 390                 395                 400

Thr Thr Thr Glu Ala Pro Thr Thr Thr Glu Ala Pro Thr Thr Thr Glu
                405                 410                 415

Ala Pro Thr Thr Thr Glu Ala Pro Thr Thr Thr Glu Ala Pro Thr Thr
            420                 425                 430

Thr Glu Ala Pro Thr Thr Thr Glu Ala Pro Thr Thr Thr Glu Ala Pro
        435                 440                 445

Thr Thr Thr Glu Ala Pro Thr Thr Thr Glu Ala Pro Thr Thr Thr Glu
    450                 455                 460

Ala Pro Thr Thr Thr Glu Ala Pro Thr Thr Thr Glu Val Ser Ser Glu
```

465 470 475 480

Thr Thr Lys Ala Glu Glu Thr Thr Thr Lys Val Lys Glu Pro Glu Lys
485 490 495

Thr Thr Thr Ser Val Pro Ala Gly Thr Thr Ser Asn Lys Pro Asn Lys
500 505 510

Pro Ser Gly Lys Gln Gly Ala Gly Thr Lys Gly Leu Pro Ser Thr Gly
515 520 525

Glu Glu Ser Gly Ile Val Leu Ser Leu Leu Gly Leu Ala Thr Val Ser
530 535 540

Val Thr Gly Leu Val Tyr Arg Lys Tyr His Ser
545 550 555

<210> 4
<211> 1668
<212> DNA
<213> Streptococcus mutans

<400> 4

```
atgaaaagaa aaggtttacg aagactatta aagtttttttg gaaccgttgc catcattttg     60
ccaatgtttt tcatagcttt aacgaaagct caggcaagtg atgtcagcag taacatttca    120
tcgctgacgg tatcaccgac tcagattaat gatggcggta agaccaccgt tcgctttgag    180
tttgatgagc atgctcaaaa tattaaagca ggcgacacca ttactgttaa ctggcagaat    240
tcaggaacag tcagaggaac aggttatacg aaaaccatta agctggaggt tcagggcaag    300
tatgttggtg atttggtagt tacgcaagac aaagcagttg ttactttcaa tgacagtatt    360
actggcttgc agaatatcac cggctggggt gaatttgaaa tcgaaggccg gaattttact    420
gacactacta ccggaaatac tggcagcttc caagttacca gcggcggcaa gacagctgag    480
gttactgtcg ttaaatctgc ttcagggact accggcgttt tctactataa gactggggat    540
atgcagacag atgacaccaa tcatgtgcgc tggtttttga atatcaacaa tgagaatgct    600
tatgtagaca gtgatattcg tattgaagat gacattcagt ctggtcaaac tttggatata    660
gacagttttg atattactgt aaatggcagt gagtcttatc acggtcaaga aggtattaat    720
cagcttgccc aaagatatgg tgcaactatt tcagctgatc cggctagtgg ccatatcagt    780
gtttatattc ctcaaggcta tgcttctttg aatcgcttta gcatcatgta cttgactaaa    840
gttgacaatc ctgatcaaaa gacgtttgaa aataacagta aggcttggta taaggaaaac    900
ggtaaagatg ctgttgatgg taaggaattt aaccattctg tagctaatgt taatgccgcc    960
ggcggtgtgg acggaagaac aaccactact acagaaaagc caacaacgac gacagaggct   1020
ccaacaacaa cggaaactcc aacgacaaca gaggctccaa caacggaagc tccaacgaca   1080
acagaggctc caacgacaac agaggctcca acaacaacgg aagctccaac gacaacagaa   1140
gctccaacaa caacggaagc tccaacgaca acagaggctc caacgacaac agaggctcca   1200
acaacaacgg aagctccaac gacaacagag gctccaacaa caacggaagc tccaacgaca   1260
acagaagctc caacaacaac ggaagctcca acgacaacag aggctccaac aacaacggaa   1320
gctccaacga caacagaggc tccaacaaca acggaagctc caacgacaac agaggctcca   1380
acaacaacgg aagctccaac aacaacggaa gctccaacaa caacggaagt atcttcagaa   1440
acaactaaag ctgaagaaac aactactaaa gttaaggaac cagaaaaaac aacgacatca   1500
gttccagcag gtacaacttc aaacaaacct aataagccat caggcaaaca aggtgctggt   1560
accaagggac ttccaagcac aggcgaagaa agcggtattg ttttgtcact tctcggtctt   1620
gcaactgtct cagtgactgg tctagtttac cgtaaatatc atagctga                1668
```

<210> 5
<211> 165
<212> PRT
<213> Streptococcus mutans

<400> 5

```
Val Thr Ser Gly Gly Lys Thr Ala Glu Val Thr Val Val Lys Ser Ala
1               5                   10                  15

Ser Gly Thr Thr Gly Val Phe Tyr Tyr Lys Thr Gly Asp Met Gln Thr
            20                  25                  30

Asp Asp Thr Asn His Val Arg Trp Phe Leu Asn Ile Asn Asn Glu Asn
        35                  40                  45

Ala Tyr Val Asp Ser Asp Ile Arg Ile Glu Asp Asp Ile Gln Ser Gly
    50                  55                  60

Gln Thr Leu Asp Ile Asp Ser Phe Asp Ile Thr Val Asn Gly Ser Glu
65                  70                  75                  80

Ser Tyr His Gly Gln Glu Gly Ile Asn Gln Leu Ala Gln Arg Tyr Gly
                85                  90                  95

Ala Thr Ile Ser Ala Asp Pro Ala Ser Gly His Ile Ser Val Tyr Ile
            100                 105                 110

Pro Gln Gly Tyr Ala Ser Leu Asn Arg Phe Ser Ile Met Tyr Leu Thr
        115                 120                 125

Lys Val Asp Asn Pro Asp Gln Lys Thr Phe Glu Asn Asn Ser Lys Ala
    130                 135                 140

Trp Tyr Lys Glu Asn Gly Lys Asp Ala Val Asp Gly Lys Glu Phe Asn

145                 150                 155                 160

His Ser Val Ala Asn
                165
```

<210> 6
<211> 495
<212> DNA
<213> Streptococcus mutans

<400> 6

```
gttaccagcg gcggcaagac agctgaggtt actgtcgtta aatctgcttc agggactacc     60
ggcgttttct actataagac tggggatatg cagacagatg acaccaatca tgtgcgctgg    120
tttttgaata tcaacaatga gaatgcttat gtagacagtg atattcgtat tgaagatgac    180
attcagtctg gtcaaacttt ggatatagac agttttgata ttactgtaaa tggcagtgag    240
tcttatcacg gtcaagaagg tattaatcag cttgcccaaa gatatggtgc aactatttca    300
gctgatccgg ctagtggcca tatcagtgtt tatattcctc aaggctatgc ttctttgaat    360
cgctttagca tcatgtactt gactaaagtt gacaatcctg atcaaaagac gtttgaaaat    420
aacagtaagg cttggtataa ggaaaacggt aaagatgctg ttgatggtaa ggaatttaac    480
cattctgtag ctaat                                                     495
```

<210> 7
<211> 549
<212> PRT
<213> Streptococcus mutans

<400> 7

```
Met Lys Arg Lys Gly Leu Arg Arg Leu Leu Lys Phe Phe Gly Thr Val
1               5                   10                  15

Ala Ile Ile Leu Pro Met Phe Phe Ile Ala Leu Thr Lys Ala Gln Ala
            20                  25                  30

Ser Asp Val Ser Ser Asn Ile Ser Ser Leu Thr Val Ser Pro Thr Gln
        35                  40                  45

Ile Asn Asp Gly Gly Lys Thr Thr Val Arg Phe Glu Phe Asp Glu His
    50                  55                  60

Ala Gln Asn Ile Lys Ala Gly Asp Thr Ile Thr Val Asn Trp Gln Asn
65                  70                  75                  80

Ser Gly Thr Val Arg Gly Thr Gly Tyr Thr Lys Thr Ile Lys Leu Glu
                85                  90                  95

Val Gln Gly Lys Tyr Val Gly Asp Leu Val Val Thr Gln Asp Lys Ala
            100                 105                 110
```

```
Val Val Thr Phe Asn Asp Ser Ile Thr Gly Leu Gln Asn Ile Thr Gly
        115                 120                 125

Trp Gly Glu Phe Glu Ile Glu Gly Arg Asn Phe Thr Asp Thr Thr Thr
    130                 135                 140

Gly Ser Thr Gly Ser Phe Gln Val Thr Ser Gly Gly Lys Thr Ala Glu
145                 150                 155                 160

Val Thr Val Val Lys Ser Ala Ser Gly Thr Thr Gly Val Phe Tyr Tyr
                165                 170                 175

Lys Thr Gly Asp Met Gln Thr Asp Asp Thr Asn His Val Arg Trp Phe
            180                 185                 190

Leu Asn Ile Asn Asn Glu Asn Ala Tyr Val Asp Ser Asp Ile Arg Ile
        195                 200                 205

Glu Asp Asp Ile Gln Ser Gly Gln Thr Leu Asp Ile Asp Ser Phe Asp
    210                 215                 220

Ile Thr Val Asn Gly Ser Glu Ser Tyr His Gly Gln Glu Gly Ile Asn
225                 230                 235                 240

Gln Leu Ala Gln Arg Tyr Gly Ala Thr Ile Ser Ala Asp Pro Ala Ser
                245                 250                 255

Gly His Asn Ser Val Tyr Ile Pro Gln Gly Tyr Ala Ser Leu Asn Arg
            260                 265                 270

Phe Ser Ile Met Tyr Leu Thr Lys Val Asp Asn Pro Asp Gln Lys Thr
        275                 280                 285

Phe Glu Asn Asn Ser Lys Ala Trp Tyr Lys Glu Asn Gly Lys Asp Ala
    290                 295                 300

Val Asp Gly Lys Glu Phe Asn His Ser Val Ala Asn Val Asn Ala Ala
305                 310                 315                 320

Gly Gly Val Asp Gly Arg Thr Thr Thr Thr Thr Glu Lys Pro Thr Thr
                325                 330                 335

Thr Thr Glu Ala Pro Thr Thr Thr Glu Thr Pro Thr Thr Thr Glu Ala
            340                 345                 350

Pro Thr Thr Glu Ala Pro Thr Thr Thr Glu Ala Pro Thr Thr Thr Glu
        355                 360                 365
```

```
Ala Pro Thr Thr Thr Glu Ala Pro Thr Thr Thr Glu Ala Pro Thr Thr
    370                 375                 380

Thr Glu Ala Pro Thr Thr Thr Glu Ala Pro Thr Thr Thr Glu Ala Pro
385                 390                 395                 400

Thr Thr Thr Glu Ala Pro Thr Thr Thr Glu Ala Pro Thr Thr Thr Glu
                405                 410                 415

Ala Pro Thr Thr Thr Glu Ala Pro Thr Thr Thr Glu Ala Pro Thr Thr
            420                 425                 430

Thr Glu Ala Pro Thr Thr Thr Glu Ala Pro Thr Thr Thr Glu Ala Pro
        435                 440                 445

Thr Thr Thr Glu Ala Pro Thr Thr Thr Glu Ala Pro Thr Thr Thr Glu
    450                 455                 460

Ala Pro Thr Thr Thr Glu Val Ser Ser Glu Thr Thr Lys Ala Glu Glu
465                 470                 475                 480

Thr Thr Thr Lys Val Lys Glu Pro Glu Lys Thr Thr Thr Ser Val Pro
                485                 490                 495

Ala Gly Thr Thr Ser Asn Lys Pro Asn Lys Pro Ser Gly Lys Gln Gly
            500                 505                 510

Ala Gly Thr Lys Gly Leu Pro Ser Thr Gly Glu Glu Ser Gly Ile Val
        515                 520                 525

Leu Ser Leu Leu Gly Leu Ala Thr Val Ser Val Thr Gly Leu Val Tyr
    530                 535                 540

Arg Lys Tyr His Ser
545
```

<210> 8
<211> 1650
<212> DNA
<213> Streptococcus mutans

<400> 8

```
atgaaaagaa aaggtttacg aagactatta aagtttttttg gaaccgttgc catcattttg      60
ccaatgtttt tcatagcttt aacgaaagct caggcaagtg atgtcagcag taacatttca     120
tcgctgacgg tatcaccgac tcagattaat gatggcggta agaccaccgt tcgctttgag     180
tttgatgagc atgctcaaaa tattaaagca ggcgacacca ttactgttaa ctggcagaat     240
tcaggaacag tcagaggaac aggttatacg aaaaccatta agctggaggt tcagggcaag     300
tatgttggtg atttggtagt tacgcaagac aaagcagttg ttactttcaa tgacagtatt     360
actggcttgc agaatatcac cggctggggt gaatttgaaa tcgaaggccg gaattttact     420
gacactacta ccggaagtac tggcagcttc caagttacca gcggcggcaa gacagctgag     480
gttactgtcg ttaaatctgc ttcagggact accggcgttt tctactataa gactggggat     540
atgcagacag atgacaccaa tcatgtgcgc tggtttttga atatcaacaa tgagaatgct     600
tatgtagaca gtgatattcg tattgaagat gacattcagt ctggtcaaac tttggatata     660
gacagttttg atattactgt aaatggcagt gagtcttatc acggtcaaga aggtattaat     720
cagcttgccc aaagatatgg tgcaactatt tcagctgatc cggctagtgg ccataacagt     780
gtttatattc ctcaaggcta tgcttctttg aatcgcttta gcatcatgta cttgactaaa     840
gttgacaatc ctgatcaaaa gacgtttgaa aataacagta aggcttggta taaggaaaac     900
ggtaaagatg ctgttgatgg taaggaattt aaccattctg tagctaatgt taatgccgcc     960
ggcggtgtgg acggaagaac aaccactact acagaaaagc caacaacgac gacagaggct    1020
ccaacaacaa cggaaactcc aacgacaaca gaggctccaa caacggaagc tccaacgaca    1080
acagaggctc caacaacaac ggaagctcca acgacaacag aagctccaac aacaacggaa    1140
gctccaacga caacagaggc tccaacaaca acggaagctc caacgacaac agaagctcca    1200
acaacaacgg aagctccaac gacaacagag gctccaacaa caacggaagc tccaacgaca    1260
acagaagctc caacgacaac agaggctcca acgacaacag aagctccaac aacaacggaa    1320
gctccaacga caacagaggc tccaacaaca acggaagctc caacgacaac agaggctcca    1380
acaacaacgg aagctccaac aacaacggaa gtatcttcag aaacaactaa agctgaagaa    1440
acaactacta aagttaagga accagaaaaa acaacgacat cagttccagc aggtacaact    1500
tcaaacaaac ctaataagcc atcaggcaaa caaggtgctg gtaccaaggg acttccaagc    1560
acaggcgaag aaagcggtat tgttttgtca cttctcggtc ttgcaactgt ctcagtgact    1620
ggtctagttt accgtaaata tcatagctga                                     1650
```

<210> 9
<211> 165
<212> PRT
<213> Streptococcus mutans

<400> 9

```
Val Thr Ser Gly Gly Lys Thr Ala Glu Val Thr Val Val Lys Ser Ala
1               5                   10                  15

Ser Gly Thr Thr Gly Val Phe Tyr Tyr Lys Thr Gly Asp Met Gln Thr
            20                  25                  30

Asp Asp Thr Asn His Val Arg Trp Phe Leu Asn Ile Asn Asn Glu Asn
        35                  40                  45

Ala Tyr Val Asp Ser Asp Ile Arg Ile Glu Asp Asp Ile Gln Ser Gly
    50                  55                  60

Gln Thr Leu Asp Ile Asp Ser Phe Asp Ile Thr Val Asn Gly Ser Glu
65                  70                  75                  80

Ser Tyr His Gly Gln Glu Gly Ile Asn Gln Leu Ala Gln Arg Tyr Gly
                85                  90                  95

Ala Thr Ile Ser Ala Asp Pro Ala Ser Gly His Asn Ser Val Tyr Ile
            100                 105                 110

Pro Gln Gly Tyr Ala Ser Leu Asn Arg Phe Ser Ile Met Tyr Leu Thr
        115                 120                 125

Lys Val Asp Asn Pro Asp Gln Lys Thr Phe Glu Asn Asn Ser Lys Ala
    130                 135                 140

Trp Tyr Lys Glu Asn Gly Lys Asp Ala Val Asp Gly Lys Glu Phe Asn
145                 150                 155                 160

His Ser Val Ala Asn
                165
```

<210> 10
<211> 495
<212> DNA
<213> Streptococcus mutans

<400> 10

```
gttaccagcg gcggcaagac agctgaggtt actgtcgtta aatctgcttc agggactacc      60
ggcgttttct actataagac tggggatatg cagacagatg acaccaatca tgtgcgctgg     120
tttttgaata tcaacaatga gaatgcttat gtagacagtg atattcgtat tgaagatgac     180
attcagtctg gtcaaacttt ggatatagac agttttgata ttactgtaaa tggcagtgag     240
tcttatcacg gtcaagaagg tattaatcag cttgcccaaa gatatggtgc aactatttca     300
gctgatccgg ctagtggcca taacagtgtt tatattcctc aaggctatgc ttctttgaat     360
cgctttagca tcatgtactt gactaaagtt gacaatcctg atcaaaagac gtttgaaaat     420
aacagtaagg cttggtataa ggaaaacggt aaagatgctg ttgatggtaa ggaatttaac     480
cattctgtag ctaat                                                      495
```

<210> 11
<211> 27
<212> DNA
<213> Artificial

<220>
<223> S.mutans specific primer F

<400> 11
ggcaccacaa cattgggaag ctcagtt 27

<210> 12
<211> 25
<212> DNA
<213> Artificial

<220>
<223> S.mutans specific primer R

<400> 12
ggaatggccg ctaagtcaac aggat 25

<210> 13
<211> 20
<212> DNA
<213> Artificial

<220>
<223> S.mutans CBP primer (cnm1F)

<400> 13
gacaaagaaa tgaaagatgt 20

<210> 14
<211> 20
<212> DNA
<213> Artificial

<220>
<223> S.mutans CBP primer (cnm1R)

<400> 14
gcaaagactc ttgtccctgc 20

<210> 15
<211> 30
<212> DNA
<213> Artificial

<220>
<223> S.mutans PAC primer (pac-F)

<400> 15
gcgcgcatgc tttattcaga tttggaggat 30

<210> 16
<211> 30
<212> DNA
<213> Artificial

<220>
<223> S.mutans PAC primer (pac-R)

<400> 16
gcgaaagcgc atgctgtgat ttatcgcttc 30

<210> 17
<211> 1566
<212> PRT
<213> Streptococcus mutans

<400> 17

```
Met Lys Val Lys Lys Thr Tyr Gly Phe Arg Lys Ser Lys Ile Ser Lys
1               5                   10                  15

Thr Leu Cys Gly Ala Val Leu Gly Thr Val Ala Ala Val Ser Val Ala
            20                  25                  30

Gly Gln Lys Val Phe Ala Asp Glu Thr Thr Thr Thr Ser Asp Val Asp
        35                  40                  45

Thr Lys Val Val Gly Thr Gln Thr Gly Asn Pro Ala Thr Asn Leu Pro
    50                  55                  60

Glu Ala Gln Gly Ser Ala Ser Lys Glu Ala Glu Gln Ser Gln Asn Gln
65                  70                  75                  80

Ala Gly Glu Thr Asn Gly Ser Ile Pro Val Glu Val Pro Lys Thr Asp
                85                  90                  95

Leu Asp Gln Ala Ala Lys Asp Ala Lys Ser Ala Gly Val Asn Val Val
            100                 105                 110

Gln Asp Ala Asp Val Asn Lys Gly Thr Val Lys Thr Ala Glu Glu Ala
        115                 120                 125

Val Gln Lys Glu Thr Glu Ile Lys Glu Asp Tyr Thr Lys Gln Ala Glu
    130                 135                 140

Asp Ile Lys Lys Thr Thr Asp Gln Tyr Lys Ser Asp Val Ala Ala His
145                 150                 155                 160

Glu Ala Glu Val Ala Lys Ile Lys Ala Lys Asn Gln Ala Thr Lys Glu
                165                 170                 175

Gln Tyr Glu Lys Asp Met Ala Ala His Lys Ala Glu Val Glu Arg Ile
            180                 185                 190

Asn Ala Ala Asn Ala Ala Ser Lys Thr Ala Tyr Glu Ala Lys Leu Ala
        195                 200                 205

Gln Tyr Gln Ala Asp Leu Ala Ala Val Gln Lys Thr Asn Ala Ala Asn
    210                 215                 220

Gln Ala Ala Tyr Gln Lys Ala Leu Ala Ala Tyr Gln Ala Glu Leu Lys
225                 230                 235                 240
```

```
Arg Val Gln Glu Ala Asn Ala Ala Ala Lys Ala Ala Tyr Asp Thr Ala
                245                 250                 255

Val Ala Ala Asn Asn Ala Lys Asn Thr Glu Ile Ala Ala Ala Asn Glu
            260                 265                 270

Glu Ile Arg Lys Arg Asn Ala Thr Ala Lys Ala Glu Tyr Glu Thr Lys
        275                 280                 285

Leu Ala Gln Tyr Gln Ala Glu Leu Lys Arg Val Gln Glu Ala Asn Ala
    290                 295                 300

Ala Asn Glu Ala Asp Tyr Gln Ala Lys Leu Thr Ala Tyr Gln Thr Glu
305                 310                 315                 320

Leu Ala Arg Val Gln Lys Ala Asn Ala Asp Ala Lys Ala Ala Tyr Glu
                325                 330                 335

Ala Ala Val Ala Ala Asn Asn Ala Lys Asn Ala Ala Leu Thr Ala Glu
            340                 345                 350

Asn Thr Ala Ile Lys Gln Arg Asn Glu Asn Ala Lys Ala Thr Tyr Glu
        355                 360                 365

Ala Ala Leu Lys Gln Tyr Glu Ala Asp Leu Ala Ala Val Lys Lys Ala
    370                 375                 380

Asn Ala Ala Asn Glu Ala Asp Tyr Gln Ala Lys Leu Thr Ala Tyr Gln
385                 390                 395                 400

Thr Glu Leu Ala Arg Val Gln Lys Ala Asn Ala Asp Ala Lys Ala Ala
                405                 410                 415

Tyr Glu Ala Ala Val Ala Ala Asn Asn Ala Ala Asn Ala Ala Leu Thr
            420                 425                 430

Ala Glu Asn Thr Ala Ile Lys Lys Arg Asn Ala Asp Ala Lys Ala Asp
        435                 440                 445

Tyr Glu Ala Lys Leu Ala Lys Tyr Gln Ala Asp Leu Ala Lys Tyr Gln
    450                 455                 460

Lys Asp Leu Ala Asp Tyr Pro Val Lys Leu Lys Ala Tyr Glu Asp Glu
465                 470                 475                 480

Gln Ala Ser Ile Lys Ala Ala Leu Ala Glu Leu Glu Lys His Lys Asn
                485                 490                 495
```

```
Glu Asp Gly Asn Leu Thr Glu Pro Ser Ala Gln Asn Leu Val Tyr Asp
            500                 505                 510

Leu Glu Pro Asn Ala Asn Leu Ser Leu Thr Thr Asp Gly Lys Phe Leu
        515                 520                 525

Lys Ala Ser Ala Val Asp Asp Ala Phe Ser Lys Ser Thr Ser Lys Ala
    530                 535                 540

Lys Tyr Asp Gln Lys Ile Leu Gln Leu Asp Asp Leu Asp Ile Thr Asn
545                 550                 555                 560

Leu Glu Gln Ser Asn Asp Val Ala Ser Ser Met Glu Leu Tyr Gly Asn
                565                 570                 575

Phe Gly Asp Lys Ala Gly Trp Ser Thr Thr Val Ser Asn Asn Ser Gln
            580                 585                 590

Val Lys Trp Gly Ser Val Leu Leu Glu Arg Gly Gln Ser Ala Thr Ala
        595                 600                 605

Thr Tyr Thr Asn Leu Gln Asn Ser Tyr Tyr Asn Gly Lys Lys Ile Ser
    610                 615                 620

Lys Ile Val Tyr Lys Tyr Thr Val Asp Pro Lys Ser Lys Phe Gln Gly
625                 630                 635                 640

Gln Lys Val Trp Leu Gly Ile Phe Thr Asp Pro Thr Leu Gly Val Phe
                645                 650                 655

Ala Ser Ala Tyr Thr Gly Gln Val Glu Lys Asn Thr Ser Ile Phe Ile
            660                 665                 670

Lys Asn Glu Phe Thr Phe Tyr Asp Glu Asp Gly Lys Pro Ile Asp Phe
        675                 680                 685

Asp Asn Ala Leu Leu Ser Val Ala Ser Leu Asn Arg Glu His Asn Ser
    690                 695                 700

Ile Glu Met Ala Lys Asp Tyr Ser Gly Lys Phe Val Lys Ile Ser Gly
705                 710                 715                 720

Ser Ser Ile Gly Glu Lys Asn Gly Met Ile Tyr Ala Thr Asp Thr Leu
                725                 730                 735

Asn Phe Lys Gln Gly Glu Gly Gly Ser Arg Trp Thr Met Tyr Lys Asn
            740                 745                 750
```

Ser Gln Ala Gly Ser Gly Trp Asp Ser Ser Asp Ala Pro Asn Ser Trp
755 760 765

Tyr Gly Ala Gly Ala Ile Lys Met Ser Gly Pro Asn Asn His Val Thr
770 775 780

Val Gly Ala Thr Ser Ala Thr Asn Val Met Pro Val Ser Asp Met Pro
785 790 795 800

Val Val Pro Gly Lys Asp Asn Thr Asp Gly Lys Lys Pro Asn Ile Trp
805 810 815

Tyr Ser Leu Asn Gly Lys Ile Arg Ala Val Asn Val Pro Lys Val Thr
820 825 830

Lys Glu Lys Pro Thr Pro Pro Val Lys Pro Thr Ala Pro Thr Lys Pro
835 840 845

Thr Tyr Glu Thr Glu Lys Pro Leu Lys Pro Ala Pro Val Ala Pro Asn
850 855 860

Tyr Glu Lys Glu Pro Thr Pro Pro Thr Arg Thr Pro Asp Gln Ala Glu
865 870 875 880

Pro Asn Lys Pro Thr Pro Pro Thr Tyr Glu Thr Glu Lys Pro Leu Glu
885 890 895

Pro Ala Pro Val Glu Pro Ser Tyr Glu Ala Glu Pro Thr Pro Pro Thr
900 905 910

Arg Thr Pro Asp Gln Ala Glu Pro Asn Lys Pro Thr Pro Pro Thr Tyr
915 920 925

Glu Thr Glu Lys Pro Leu Glu Pro Ala Pro Val Glu Pro Ser Tyr Glu
930 935 940

Ala Glu Pro Thr Pro Pro Thr Pro Thr Pro Asp Gln Pro Glu Pro Asn
945 950 955 960

Lys Pro Val Glu Pro Thr Tyr Glu Val Ile Pro Thr Pro Pro Thr Asp
965 970 975

Pro Val Tyr Gln Asp Leu Pro Thr Pro Pro Ser Val Pro Thr Val His
980 985 990

Phe His Tyr Phe Lys Leu Ala Val Gln Pro Gln Val Asn Lys Glu Ile
995 1000 1005

Arg Asn Asn Asn Asp Val Asn Ile Asp Arg Thr Leu Val Ala Lys

1010 1015 1020

Gln Ser Val Val Lys Phe Gln Leu Lys Thr Ala Asp Leu Pro Ala
1025 1030 1035

Gly Arg Asp Glu Thr Thr Ser Phe Val Leu Val Asp Pro Leu Pro
1040 1045 1050

Ser Gly Tyr Gln Phe Asn Pro Glu Ala Thr Lys Ala Ala Ser Pro
1055 1060 1065

Gly Phe Asp Val Ala Tyr Asp Asn Ala Thr Asn Thr Val Thr Phe
1070 1075 1080

Lys Ala Thr Ala Ala Thr Leu Ala Thr Phe Asn Ala Asp Leu Thr
1085 1090 1095

Lys Ser Val Ala Thr Ile Tyr Pro Thr Val Val Gly Gln Val Leu
1100 1105 1110

Asn Asp Gly Ala Thr Tyr Lys Asn Asn Phe Thr Leu Thr Val Asn
1115 1120 1125

Asp Ala Tyr Gly Ile Lys Ser Asn Val Val Arg Val Thr Thr Pro
1130 1135 1140

Gly Lys Pro Asn Asp Pro Asp Asn Pro Asn Asn Asn Tyr Ile Lys
1145 1150 1155

Pro Thr Lys Val Asn Lys Asn Glu Asn Gly Val Val Ile Asp Gly
1160 1165 1170

Lys Thr Val Leu Ala Gly Ser Thr Asn Tyr Tyr Glu Leu Thr Trp
1175 1180 1185

Asp Leu Asp Gln Tyr Lys Asn Asp Arg Ser Ser Ala Asp Thr Ile
1190 1195 1200

Gln Gln Gly Phe Tyr Tyr Val Asp Asp Tyr Pro Glu Glu Ala Leu
1205 1210 1215

Glu Leu Arg Gln Asp Leu Val Lys Ile Thr Asp Ala Asn Gly Asn
1220 1225 1230

Glu Val Thr Gly Val Ser Val Asp Asn Tyr Thr Ser Leu Glu Ala
1235 1240 1245

Ala Pro Gln Glu Ile Arg Asp Val Leu Ser Lys Ala Gly Ile Arg
1250 1255 1260

```
Pro Lys Gly Ala Phe Gln Ile Phe Arg Ala Asn Asn Pro Arg Glu
    1265                1270                1275

Phe Tyr Asp Thr Tyr Val Lys Thr Gly Ile Asp Leu Lys Ile Val
    1280                1285                1290

Ser Pro Met Val Val Lys Lys Gln Met Gly Gln Thr Gly Gly Ser
    1295                1300                1305

Tyr Glu Asn Gln Ala Tyr Gln Ile Asp Phe Gly Asn Gly Tyr Ala
    1310                1315                1320

Ser Asn Ile Val Ile Asn Asn Val Pro Lys Ile Asn Pro Lys Lys
    1325                1330                1335

Asp Val Thr Leu Thr Leu Asp Pro Ala Asp Thr Asn Asn Val Asp
    1340                1345                1350

Gly Gln Thr Ile Pro Leu Asn Thr Val Phe Asn Tyr Arg Leu Ile
    1355                1360                1365

Gly Gly Ile Ile Pro Ala Asn His Ser Glu Glu Leu Phe Glu Tyr
    1370                1375                1380

Asn Phe Tyr Asp Asp Tyr Asp Gln Thr Gly Asp His Tyr Thr Gly
    1385                1390                1395

Gln Tyr Lys Val Phe Ala Lys Val Asp Ile Thr Leu Lys Asn Gly
    1400                1405                1410

Val Ile Ile Lys Ser Gly Thr Glu Leu Thr Gln His Thr Thr Ala
    1415                1420                1425

Glu Val Asp Thr Thr Lys Gly Ala Ile Thr Ile Lys Phe Lys Glu
    1430                1435                1440

Ala Phe Leu Arg Ser Val Ser Ile Asp Ser Ala Phe Gln Ala Glu
    1445                1450                1455

Ser Tyr Ile Gln Met Lys Arg Ile Ala Val Gly Thr Phe Glu Asn
    1460                1465                1470

Thr Tyr Ile Asn Thr Val Asn Gly Val Thr Tyr Ser Ser Asn Thr
    1475                1480                1485

Val Lys Thr Thr Thr Pro Glu Asp Pro Thr Asp Pro Thr Asp Pro
    1490                1495                1500
```

Gln Asp Pro Ser Ser Pro Arg Thr Ser Thr Val Ile Asn Tyr Lys
1505 1510 1515

Pro Gln Ser Thr Ala Tyr Gln Pro Ser Ser Val Gln Lys Thr Leu
1520 1525 1530

Pro Asn Thr Gly Val Thr Asn Asn Ala Tyr Met Pro Leu Leu Gly
1535 1540 1545

Ile Ile Gly Leu Val Thr Ser Phe Ser Leu Leu Gly Leu Lys Ala
1550 1555 1560

Lys Lys Asp
1565

<210> 18
<211> 4701
<212> DNA
<213> Streptococcus mutans

<400> 18

```
atgaaagtca aaaaaactta cggttttcgt aaaagtaaaa ttagtaaaac actgtgtggt      60
gctgttctag gaacagtagc agcagtctct gtagcgggac aaaaggtttt tgccgatgaa     120
acgaccacta ctagtgatgt agatactaaa gtagttggaa cacaaactgg aaatccagcg     180
accaatttgc cagaggctca agggagtgcg agtaaggaag ctgaacaaag tcaaaaccaa     240
gctggagaga caaatggttc aataccagtt gaagtaccta aaactgatct tgatcaagca     300
gcaaaagatg ctaagtctgc tggtgtcaat gttgtccaag atgccgatgt taataaagga     360
actgttaaaa cagctgaaga agcagtccaa aaagaaactg aaattaaaga agattacaca     420
aaacaagctg aggatattaa gaagacaaca gatcaatata aatcggatgt agctgctcat     480
gaggcagaag ttgctaaaat caaagctaaa aatcaggcga ctaaagaaca gtatgaaaaa     540
gatatggcag ctcataaagc cgaggttgaa cgcattaatg ctgcaaatgc tgccagtaaa     600
acagcttatg aagctaaatt ggctcaatat caagcagatt tagcagccgt tcaaaaaacc     660
aatgctgcca atcaagcagc ctatcaaaaa gcccttgctg cctatcaggc tgaactgaaa     720
cgtgttcagg aagctaatgc agccgccaaa gctgcttatg atactgctgt agcagcaaat     780
aatgccaaaa atacagaaat tgccgctgcc aatgaagaaa ttagaaaacg caatgcaacg     840
gccaaagctg aatatgagac taagttagct caatatcaag ctgaactaaa gcgtgttcag     900
gaagctaatg ccgcaaacga agcagactat caagctaaat tgaccgccta tcaaacagag     960
ctcgctcgcg ttcaaaaggc taatgcggat gctaaagcgg cctatgaagc agctgtagca    1020
gcaaataatg ccaaaaatgc ggcactcaca gctgaaaata ctgcaattaa gcaacgcaat    1080
gagaatgcta aggcgactta tgaagctgca ctcaagcaat atgaggccga tttggcagcg    1140
```

gtgaaaaaag ctaatgccgc aaacgaagca gactatcaag ctaaattgac cgcctatcaa 1200
acagagctcg ctcgcgttca aaaagccaat gcggatgcta aagcggccta tgaagcagct 1260
gtagcagcaa ataatgccgc aaatgcagcg ctcacagctg aaaatactgc aattaagaag 1320
cgcaatgcgg atgctaaagc tgattacgaa gcaaaacttg ctaagtatca agcagatctt 1380
gccaaatatc agaaagattt agcagactat ccagttaagt taaaggcata cgaagatgaa 1440
caagcttcta ttaaagctgc actggcagaa cttgaaaaac ataaaaatga agacggaaac 1500
ttaacagaac catctgctca aaatttggtc tatgatcttg agccaaatgc gaacttatct 1560
ttgacaacag atgggaagtt ccttaaggct tctgctgtgg atgatgcttt tagcaaaagc 1620
acttcaaaag caaaatatga ccaaaaaatt cttcaattag atgacctaga tataactaac 1680
ttagaacaat ctaatgatgt tgcttcttct atggagcttt atgggaattt tggtgataaa 1740
gctggctggt caacaacagt aagcaataac tcacaggtta aatggggatc ggtactttta 1800
gagcgcggtc aaagcgcaac agctacatac actaacctgc agaattctta ttacaatggt 1860
aaaaagattt ctaaaattgt ctacaagtat acagtggacc ctaagtccaa gtttcaaggt 1920
caaaaggttt ggttaggtat ttttaccgat ccaactttag gtgtttttgc ttccgcttat 1980
acaggtcaag ttgaaaaaaa cacttctatt tttattaaaa atgaattcac tttctatgac 2040
gaagatggaa aaccaattga ttttgataat gcccttctct cagtagcttc tcttaaccgt 2100
gaacataact ctattgagat ggctaaagat tatagtggta aatttgtcaa aatctctggt 2160
tcatctattg gtgaaaagaa tggcatgatt tatgctacag atactcttaa ctttaaacag 2220
ggtgaaggcg gctctcgctg gactatgtat aaaaatagtc aagctggttc aggatgggat 2280
agttcagatg cgccgaattc ttggtatgga gcaggggcta ttaaaatgtc tggtccgaat 2340
aaccatgtta ctgtaggagc aacttctgca acaaatgtga tgccagtttc tgacatgcct 2400
gttgttcctg gtaaggacaa tactgatggc aaaaaaccaa atatttggta ttctttaaat 2460
ggtaaaatcc gtgcggttaa tgttcctaaa gttactaagg aaaaacccac acctccggtt 2520
aaaccaacag ctccaactaa accaacttat gaaacagaaa agccattaaa accggcacca 2580
gtagctccaa attatgaaaa ggagccaaca ccgccgacaa ggacaccgga tcaagcagag 2640
ccaaataaac ccacaccgcc gacctatgaa acagaaaagc cgttggagcc agcacctgtt 2700
gagccaagct atgaagcaga gccaacaccg ccgacaagga caccggatca ggcagagcca 2760
aataaaccca caccgccgac ctatgaaaca gaaaagccgt tggagccagc acctgttgag 2820
ccaagctatg aagcagagcc aacgccaccg acaccaacac cagatcaacc agaaccaaac 2880
aaacctgttg agccaactta tgaggttatt ccaacaccgc cgactgatcc tgtttatcaa 2940
gatcttccaa cacctccatc tgtaccaact gttcatttcc attactttaa actagctgtt 3000
cagccgcagg ttaacaaaga aattagaaac aataacgatg ttaatattga cagaactttg 3060
gtggctaaac aatctgttgt taagttccag ctgaagacag cagatctccc tgctggacgt 3120

gatgaaacaa cttcctttgt cttggtagat cccctgccat ctggttatca atttaatcct 3180
gaagctacaa aagctgccag ccctggcttt gatgtcgctt atgataatgc aactaataca 3240
gtcaccttca aggcaactgc agcaactttg gctacgttta atgctgattt gactaagtca 3300
gtggcaacga tttatccaac agtggtcgga caagttctta acgatggcgc aacttataag 3360
aataatttca cactcacagt caatgatgct tatggcatta aatccaatgt tgttcgggtg 3420
acaactcctg gtaaaccaaa tgatccagat aacccaaata ataattatat taaaccaact 3480
aaggttaata aaaacgaaaa tggcgttgtt attgatggta aaacagttct tgccggttca 3540
acgaattatt atgagctaac ttgggatttg gatcaatata agaacgaccg ctcttcagca 3600
gataccattc aacaaggatt ttactatgta gatgattatc cagaagaagc gcttgaattg 3660
cgtcaggatt tagtgaagat tacagatgct aatggtaatg aagttactgg tgttagtgtg 3720
gataattata ctagtcttga agcagcccct caagaaatta gagatgttct ttctaaggca 3780
ggaattagac ctaaaggtgc tttccaaatt ttccgtgcca ataatccaag agaattttat 3840
gatacttatg tcaaaactgg aattgatttg aagattgtat caccaatggt tgttaaaaaa 3900
caaatgggac aaacaggtgg cagttatgaa aatcaagctt accaaattga ctttggtaat 3960
ggttatgcat caaatatcgt tatcaataat gttcctaaga ttaaccctaa gaaagatgtg 4020
accttaacac ttgatccggc tgatacaaat aatgttgatg gtcagactat tccacttaat 4080
acagtcttta attaccgttt gattggtggc attatccctg caaatcactc agaagaactc 4140
tttgaataca atttctatga tgattatgat caaacaggag atcactatac tggtcagtat 4200
aaagtttttg ccaaggttga tatcactctt aaaaacggtg ttattattaa gtcaggtact 4260
gaattgactc agcatacgac agcggaagtt gataccacta aaggtgctat cacaattaag 4320
ttcaaggaag cctttctgcg ttctgtttca attgattcag ccttccaagc tgaaagttat 4380
atccaaatga aacgtattgc ggttggtact tttgaaaata cttatattaa tactgtcaat 4440
ggggtaactt acagttcaaa tacagtgaaa acaactactc ctgaggatcc tacagaccct 4500
actgatccgc aagatccatc atcaccgcgg acttcaactg taattaacta caaacctcaa 4560
tcaactgctt atcaaccaag ctctgtccaa aaaacgttac caaatacggg agtaacaaac 4620
aatgcttata tgcctttact tggtattatt ggcttagtta ctagttttag tttgcttggc 4680
ttaaaggcta agaaagattg a 4701

<210> 19
<211> 1564
<212> PRT
<213> Streptococcus mutans

<400> 19

```
Met Lys Val Lys Lys Thr Tyr Gly Phe Arg Lys Ser Lys Ile Ser Lys
1               5                   10                  15
```

```
Thr Leu Cys Gly Ala Val Leu Gly Thr Val Ala Ala Val Ser Val Ala
            20                  25                  30

Gly Gln Lys Val Phe Ala Asp Glu Thr Thr Thr Thr Ser Asp Val Asp
        35                  40                  45

Thr Lys Val Val Gly Thr Gln Thr Gly Asn Pro Ala Thr Asn Leu Pro
    50                  55                  60

Glu Ala Gln Gly Ser Ala Ser Lys Glu Ala Glu Gln Ser Gln Asn Gln
65                  70                  75                  80

Ala Gly Glu Thr Asn Gly Ser Ile Pro Ile Glu Val Pro Lys Thr Asp
                85                  90                  95

Leu Asp Gln Thr Ala Lys Asp Ala Lys Ser Ala Gly Val Asn Val Val
            100                 105                 110

Gln Asp Ala Asp Val Asn Lys Gly Thr Val Lys Thr Ala Glu Ala Ala
        115                 120                 125

Val Gln Lys Glu Thr Glu Ile Lys Glu Asp Tyr Thr Lys Gln Ala Glu
    130                 135                 140

Asp Ile Lys Lys Thr Thr Asp Gln Tyr Lys Ser Asp Val Ala Ala His
145                 150                 155                 160

Glu Ala Glu Val Ala Lys Ile Lys Ala Lys Asn Gln Ala Thr Lys Glu
                165                 170                 175

Gln Tyr Glu Lys Asp Met Ala Ala His Lys Ala Glu Val Glu Arg Ile
            180                 185                 190

Asn Ala Ala Asn Ala Ala Ser Lys Thr Ala Tyr Glu Ala Lys Leu Ala
        195                 200                 205

Gln Tyr Gln Ala Asp Leu Ala Ala Val Gln Lys Thr Asn Ala Ala Asn
    210                 215                 220

Gln Ala Ala Tyr Gln Lys Ala Leu Ala Ala Tyr Gln Ala Glu Leu Lys
225                 230                 235                 240

Arg Val Gln Glu Ala Asn Ala Ala Ala Lys Ala Ala Tyr Asp Thr Ala
                245                 250                 255

Val Ala Ala Asn Asn Ala Lys Asn Thr Glu Ile Thr Ala Ala Asn Glu
            260                 265                 270
```

Glu Ile Arg Lys Arg Asn Ala Thr Ala Lys Ala Glu Tyr Glu Thr Lys
275 280 285

Leu Ala Gln Tyr Gln Ala Glu Leu Lys Arg Val Gln Glu Ala Asn Ala
290 295 300

Ala Asn Glu Ala Asp Tyr Gln Ala Lys Leu Thr Ala Tyr Gln Thr Glu
305 310 315 320

Leu Ala Arg Val Gln Lys Ala Asn Ala Asp Ala Lys Ala Ala Tyr Glu
325 330 335

Ala Ala Val Ala Ala Asn Asn Ala Lys Asn Ala Ala Leu Thr Ala Glu
340 345 350

Asn Thr Ala Ile Lys Gln Arg Asn Glu Asn Ala Lys Ala Thr Tyr Glu
355 360 365

Ala Ala Leu Lys Gln Tyr Glu Ala Asp Leu Ala Ala Ala Lys Lys Ala
370 375 380

Asn Ala Ala Asn Glu Ala Asp Tyr Gln Ala Lys Leu Thr Ala Tyr Gln
385 390 395 400

Thr Glu Leu Ala Arg Val Gln Lys Thr Asn Ala Asp Ala Lys Ala Ala
405 410 415

Tyr Glu Ala Ala Val Ala Ala Asn Asn Ala Ala Asn Ala Ala Leu Thr
420 425 430

Ala Glu Asn Thr Ala Ile Lys Lys Arg Asn Ala Asp Ala Lys Ala Asp
435 440 445

Tyr Glu Ala Lys Leu Ala Lys Tyr Gln Ala Asp Leu Ala Lys Tyr Gln
450 455 460

Lys Asp Leu Ala Asp Tyr Pro Val Lys Leu Lys Ala Tyr Glu Asp Glu
465 470 475 480

Gln Ala Ser Ile Lys Ala Ala Leu Ala Glu Leu Glu Lys His Lys Asn
485 490 495

Glu Asp Gly Asn Leu Thr Glu Pro Ser Ala Gln Asn Leu Val Tyr Asp
500 505 510

Leu Glu Pro Asn Ala Asn Leu Ser Leu Thr Thr Asp Gly Lys Phe Leu
515 520 525

Lys Ala Ser Ala Val Asp Asn Ala Phe Lys Gln Asp Thr Asn Gln Tyr
    530                 535                 540

Ser Lys Lys Asn Leu Gln Leu Asp Asn Leu Asn Val Lys Tyr Leu Glu
545                 550                 555                 560

Asn Ala Gly Ala Thr Ala Ser Ser Met Glu Leu Tyr Gly Asn Ile Gly
                565                 570                 575

Asp Lys Ser Ser Trp Thr Thr Asn Val Gly Asn Lys Thr Glu Val Lys
            580                 585                 590

Trp Gly Ser Val Leu Leu Glu Arg Gly Gln Ser Ala Thr Ala Thr Tyr
        595                 600                 605

Thr Asn Leu Gln Asn Ser Tyr Tyr Asn Gly Lys Lys Ile Ser Lys Ile
    610                 615                 620

Val Tyr Lys Tyr Thr Val Asp Pro Lys Ser Lys Phe Gln Gly Gln Lys
625                 630                 635                 640

Val Trp Leu Gly Ile Phe Thr Asp Pro Thr Leu Gly Val Phe Ala Ser
                645                 650                 655

Ala Tyr Thr Gly Gln Val Glu Lys Asn Thr Ser Ile Phe Ile Lys Asn
            660                 665                 670

Glu Phe Thr Phe Tyr Asp Glu Asp Gly Lys Pro Ile Asp Phe Asp Asn
        675                 680                 685

Ala Leu Leu Ser Val Ala Ser Leu Asn Arg Glu His Asn Ser Ile Glu
    690                 695                 700

Met Ala Lys Asp Tyr Ser Gly Lys Phe Val Lys Ile Ser Gly Ser Ser
705                 710                 715                 720

Ile Gly Glu Lys Asn Gly Met Ile Tyr Ala Thr Asp Thr Leu Asn Phe
                725                 730                 735

Lys Gln Gly Glu Gly Gly Ser Arg Trp Thr Met Tyr Lys Asn Ser Gln
            740                 745                 750

Ala Gly Ser Gly Trp Asp Ser Ser Asp Ala Pro Asn Ser Trp Tyr Gly
        755                 760                 765

Ala Gly Ala Ile Lys Met Ser Gly Pro Asn Asn His Val Thr Val Gly
    770                 775                 780

Ala Thr Ser Ala Thr Asn Val Met Pro Val Ser Asp Met Pro Val Val

```
785                 790                 795                 800

Pro Gly Lys Asp Asn Thr Asp Gly Lys Lys Pro Asn Ile Trp Tyr Ser
                805                 810                 815

Leu Asn Gly Lys Ile Arg Ala Val Asn Val Pro Lys Val Thr Lys Glu
            820                 825                 830

Lys Pro Thr Pro Pro Val Lys Pro Thr Ala Pro Thr Lys Pro Thr Tyr
        835                 840                 845

Glu Thr Glu Lys Pro Leu Lys Pro Ala Pro Val Ala Pro Asn Tyr Glu
    850                 855                 860

Lys Glu Pro Thr Pro Pro Thr Arg Thr Pro Asp Gln Ala Glu Pro Asn
865                 870                 875                 880

Lys Pro Thr Pro Pro Thr Tyr Glu Thr Glu Lys Pro Leu Glu Pro Ala
                885                 890                 895

Pro Val Glu Pro Ser Tyr Glu Ala Glu Pro Thr Pro Pro Thr Arg Thr
            900                 905                 910

Pro Asp Gln Ala Glu Pro Asn Lys Pro Thr Pro Pro Thr Tyr Glu Thr
        915                 920                 925

Glu Lys Pro Leu Glu Pro Ala Pro Val Glu Pro Ser Tyr Glu Ala Glu
    930                 935                 940

Pro Thr Pro Pro Thr Pro Thr Pro Asp Gln Pro Glu Pro Asn Lys Pro
945                 950                 955                 960

Val Glu Pro Thr Tyr Glu Val Ile Pro Thr Pro Pro Thr Asp Pro Val
                965                 970                 975

Tyr Gln Asp Leu Pro Thr Pro Pro Ser Val Pro Thr Val His Phe His
            980                 985                 990

Tyr Phe Lys Leu Ala Val Gln Pro  Gln Val Asn Lys Glu  Ile Arg Asn
        995                 1000                1005

Asn Asn  Asp Val Asn Ile Asp  Arg Thr Leu Val Ala  Lys Gln Ser
    1010                 1015                 1020

Val Val  Lys Phe Gln Leu Lys  Thr Ala Asp Leu Pro  Ala Gly Arg
    1025                 1030                 1035

Asp Glu  Thr Thr Ser Phe Val  Leu Val Asp Pro Leu  Pro Ser Gly
    1040                 1045                 1050
```

Tyr Gln Phe Asn Pro Glu Ala Thr Lys Ala Ala Ser Pro Gly Phe
1055 1060 1065

Asp Val Thr Tyr Asp Asn Ala Thr Asn Thr Val Thr Phe Lys Ala
1070 1075 1080

Thr Ala Ala Thr Leu Ala Thr Phe Asn Ala Asp Leu Thr Lys Ser
1085 1090 1095

Val Ala Thr Ile Tyr Pro Thr Val Val Gly Gln Val Leu Asn Asp
1100 1105 1110

Gly Ala Thr Tyr Lys Asn Asn Phe Thr Leu Thr Val Asn Asp Ala
1115 1120 1125

Tyr Gly Ile Lys Ser Asn Val Val Arg Val Thr Thr Pro Gly Lys
1130 1135 1140

Pro Asn Asp Pro Asp Asn Pro Asn Asn Asn Tyr Ile Lys Pro Thr
1145 1150 1155

Lys Val Asn Lys Asn Glu Asn Gly Val Val Ile Asp Gly Lys Thr
1160 1165 1170

Val Leu Ala Gly Ser Thr Asn Tyr Tyr Glu Leu Thr Trp Asp Leu
1175 1180 1185

Asp Gln Tyr Lys Asn Asp Arg Ser Ser Ala Asp Thr Ile Gln Lys
1190 1195 1200

Gly Phe Tyr Tyr Val Asp Asp Tyr Pro Glu Glu Ala Leu Glu Leu
1205 1210 1215

Arg Gln Asp Leu Val Lys Ile Thr Asp Ala Asn Gly Asn Glu Val
1220 1225 1230

Thr Gly Val Ser Val Asp Asn Tyr Thr Ser Leu Glu Ala Ala Pro
1235 1240 1245

Gln Glu Ile Arg Asp Val Leu Ser Lys Ala Gly Ile Arg Pro Lys
1250 1255 1260

Gly Ala Phe Gln Ile Phe Arg Ala Asp Asn Pro Arg Glu Phe Tyr
1265 1270 1275

Asp Thr Tyr Val Lys Thr Gly Ile Asp Leu Lys Ile Val Ser Pro
1280 1285 1290

```
Met Val Val Lys Lys Gln Met Gly Gln Thr Gly Gly Ser Tyr Glu
    1295                1300                1305

Asn Gln Ala Tyr Gln Ile Asp Phe Gly Asn Gly Tyr Ala Ser Asn
    1310                1315                1320

Ile Val Ile Asn Asn Val Pro Lys Ile Asn Pro Lys Lys Asp Val
    1325                1330                1335

Thr Leu Thr Leu Asp Pro Ala Asp Thr Asn Asn Val Asp Gly Gln
    1340                1345                1350

Thr Ile Pro Leu Asn Thr Val Phe Asn Tyr Arg Leu Ile Gly Gly
    1355                1360                1365

Ile Ile Pro Ala Asn His Ser Glu Glu Leu Phe Glu Tyr Asn Phe
    1370                1375                1380

Tyr Asp Asp Tyr Asp Gln Thr Gly Asp His Tyr Thr Gly Gln Tyr
    1385                1390                1395

Lys Val Phe Ala Lys Val Asp Ile Thr Phe Lys Asp Gly Ser Ile
    1400                1405                1410

Ile Lys Ser Gly Ala Glu Leu Thr Gln Tyr Thr Thr Ala Glu Val
    1415                1420                1425

Asp Thr Thr Lys Gly Ala Ile Thr Ile Lys Phe Lys Glu Ala Phe
    1430                1435                1440

Leu Arg Ser Val Ser Ile Asp Ser Val Phe Gln Ala Glu Ser Tyr
    1445                1450                1455

Ile Gln Met Lys Arg Ile Ala Val Gly Thr Phe Glu Asn Thr Tyr
    1460                1465                1470

Ile Asn Thr Val Asn Gly Val Thr Tyr Ser Ser Asn Thr Val Lys
    1475                1480                1485

Thr Thr Thr Pro Glu Asp Pro Thr Asp Pro Thr Asp Pro Gln Asp
    1490                1495                1500

Pro Ala Ser Pro Arg Thr Ser Thr Val Ile Asn Tyr Lys Pro Gln
    1505                1510                1515

Ser Thr Ala Tyr Gln Pro Ser Ser Val Gln Lys Thr Leu Pro Asn
    1520                1525                1530
```

```
Thr Gly  Val Thr Asn Asn Ala  Tyr Met Pro Leu Leu  Gly Ile Ile
    1535                 1540                 1545

Gly Leu  Val Thr Ser Phe Ser  Leu Leu Gly Leu Lys  Ala Lys Lys
    1550                 1555                 1560

Asp
```

<210> 20
<211> 4695
<212> DNA
<213> Streptococcus mutans

<400> 20

```
atgaaagtca aaaaaactta cggttttcgt aaaagtaaaa ttagtaaaac actgtgtggt     60
gctgttctag gaacagtagc agcagtctct gtagcaggac aaaaggtttt tgccgatgaa    120
acgaccacta ctagtgatgt agatactaaa gtagttggaa cacaaactgg aaatccagcg    180
accaatttgc cagaggctca agggagtgcg agtaaggaag ctgaacaaag tcaaaaccaa    240
gctggagaga caaatggttc aataccaatt gaagtaccta aaactgatct tgatcaaaca    300
gcaaaagatg ctaagtctgc tggtgtcaat gttgtccaag atgccgatgt taataaagga    360
actgttaaaa cagctgaagc agcagtccaa aaagaaactg aaattaaaga agattacaca    420
aaacaagctg aggatattaa gaagacaaca gatcaatata aatcggatgt agctgctcat    480
gaggcagaag ttgctaaaat caaagctaaa aatcaggcaa ctaaagaaca gtatgaaaaa    540
gatatggcag ctcataaagc cgaggttgaa cgcattaatg ctgcaaatgc tgccagtaaa    600
acagcttatg aagctaaatt ggctcaatat caagcagatt tagcagccgt tcaaaaaacc    660
aatgctgcca atcaagcagc ctatcaaaaa gcccttgctg cttatcaggc tgaactgaag    720
cgtgttcagg aagctaatgc agccgccaaa gccgcttatg atactgctgt agcagcaaat    780
aatgccaaaa atacagaaat taccgctgcc aatgaagaaa ttagaaaacg caatgcaacg    840
gccaaagctg aatatgagac taagttagct caatatcaag ctgaactaaa gcgtgttcag    900
gaagctaatg cagcaaacga agcagactat caagctaaat tgactgctta tcaaacagag    960
ctcgctcgcg ttcaaaaggc caatgcggat gctaaagcgg cctatgaagc agctgtagca   1020
gcaaataatg ccaaaaatgc ggcactcaca gctgaaaata ctgcaattaa gcaacgcaat   1080
gagaatgcta aggcgactta tgaagctgca ctcaagcaat atgaggccga tttggcagca   1140
gcgaaaaaag ctaatgcagc aaacgaagca gactatcaag ctaaattgac cgcttatcaa   1200
acagagctcg ctcgcgttca aaagaccaat gcggatgcta aagcggccta tgaagcagct   1260
gtagcagcaa ataatgccgc aaatgcagcg ctcacagctg aaaatactgc aattaagaag   1320
cgcaatgcgg atgctaaagc tgattacgaa gcaaaacttg ctaagtatca agcagatctt   1380
gccaaatatc aaaaagattt agcagactat ccagttaagt taaaggcata cgaagatgaa   1440
```

caagcttcta ttaaagctgc actggcagaa cttgaaaaac ataaaaatga agacggaaac 1500
ttaacagaac catctgctca aaatttggtc tatgatcttg agccaaatgc gaacttatct 1560
ttgacaacag atgggaagtt ccttaaggct tctgctgtgg ataacgcatt taagcaagat 1620
acaaatcaat atagtaaaaa gaaccttcaa ttagataacc ttaatgttaa atatctagaa 1680
aacgcaggag ccactgcctc atctatggaa ttatacggaa atataggtga taaatcgagt 1740
tggacaacaa atgtaggcaa caaaacagaa gttaaatggg gatcggtact tttagagcgc 1800
ggtcaaagcg caacagctac atacactaac ctgcagaatt cttattacaa tggtaaaaag 1860
atttctaaaa ttgtctacaa gtatacagtg gaccctaagt ccaagtttca aggtcaaaag 1920
gtttggttag gtatttttac cgatccaact ttaggtgttt ttgcttccgc ttatacaggt 1980
caagttgaaa aaaacacttc tatttttatt aaaaatgaat tcactttcta tgacgaagat 2040
ggaaaaccaa ttgattttga taatgccctt ctctcagtag cttctcttaa ccgtgaacat 2100
aactctattg agatggctaa agattatagt ggtaaatttg tcaaaatctc tggttcatct 2160
attggtgaaa agaatggcat gatttatgct acagatactc ttaactttaa acagggtgaa 2220
ggcggctctc gctggactat gtataaaaat agtcaagctg gttcaggatg ggatagttca 2280
gatgcgccga attcttggta tggagcaggg gctattaaaa tgtctggtcc gaataaccat 2340
gttactgtag gagcaacttc tgcaacaaat gtgatgccag tttctgacat gcctgttgtt 2400
cctggtaagg acaatactga tggcaaaaaa ccaaatattt ggtattcttt aaatggtaaa 2460
atccgtgcgg ttaatgttcc taaagttact aaggaaaaac ccacacctcc ggttaaacca 2520
acagctccaa ctaaaccaac ttatgaaaca gaaaagccat taaaaccggc accagtagct 2580
ccaaattatg aaaaggagcc aacaccgccg acaaggacac cggatcaagc agagccaaat 2640
aaacccacac cgccgaccta tgaaacagaa aagccgttgg agccagcacc tgttgagcca 2700
agctatgaag cagagccaac accgccgaca aggacaccgg atcaggcaga gccaaataaa 2760
cccacaccgc cgacctatga aacagaaaag ccgttggagc cagcacctgt tgagccaagc 2820
tatgaagcag agccaacgcc accgacacca acaccagatc aaccagaacc aaacaaacct 2880
gttgagccaa cttatgaggt tattccaaca ccgccgactg atcctgttta tcaagatctt 2940
ccaacacctc catctgtacc aactgttcat ttccattact ttaaactagc tgttcagccg 3000
caggttaaca aagaaattag aaacaataac gatgttaata ttgacagaac tttggtggct 3060
aaacaatctg ttgttaagtt ccagctgaag acagcagatc tccctgctgg acgtgatgaa 3120
acaacttcct ttgtcttggt agatcccctg ccatctggtt atcaatttaa tcctgaagct 3180
acaaaagctg caagccctgg ctttgatgtc acttatgata atgcaactaa tacagtcacc 3240
ttcaaggcaa ctgcagcaac tttggctacg tttaatgctg atttgactaa gtcagtggca 3300
acgatttatc caacagtggt cggacaagtt cttaatgatg gcgcaactta taagaataat 3360

ttcacgctca cagtcaatga tgcttatggc attaaatcca atgttgttcg ggtgacaact 3420

cctggtaaac caaatgatcc agataatcca aataataatt atattaaacc aactaaggtt 3480

aataaaaacg aaaatggcgt tgttattgat ggtaaaacag ttcttgccgg ttcaacgaat 3540

tattatgagc taacttggga tttggatcaa tataaaaacg accgctcttc agcagatacc 3600

attcaaaaag gattttacta tgtagatgat tatccagaag aagcgcttga attgcgtcag 3660

gatttagtga agattacaga tgctaatggt aatgaagtta ctggtgttag tgtggataat 3720

tatactagtc ttgaagcagc ccctcaagaa attagagatg ttctttctaa ggcaggaatt 3780

agacctaaag gtgctttcca aattttccgt gccgataatc caagagaatt ttatgatact 3840

tatgtcaaaa ctggaattga tttgaagatt gtatcaccaa tggttgttaa aaaacaaatg 3900

ggacaaacag gcggcagtta tgaaaatcaa gcttaccaaa ttgactttgg taatggttat 3960

gcatcaaata tcgttatcaa taatgttcct aagattaacc ctaagaaaga tgtgacctta 4020

acacttgatc cggctgatac aaataatgtt gatggtcaga ctattccact taatacagtc 4080

tttaattacc gtttgattgg tggcattatc cctgcaaatc actcagaaga actctttgaa 4140

tacaatttct atgatgatta tgatcaaaca ggagatcact atactggtca gtataaagtt 4200

tttgccaagg ttgatatcac ttttaaagac ggttctatta tcaagtcagg tgctgagtta 4260

actcagtata cgacagcgga agttgatacc actaaaggtg ctatcacaat taagttcaag 4320

gaagcctttc tgcgttctgt ttcaattgat tcagtcttcc aagctgaaag ttatatccaa 4380

atgaaacgta ttgcggttgg tacttttgaa aatacttata ttaatactgt caatggggta 4440

acttacagtt caaatacagt gaaaacaact actcctgagg atcctacaga ccctactgat 4500

ccgcaagatc cagcatcacc gcggacttca actgtaatta actacaaacc tcaatcaact 4560

gcttatcaac caagctctgt ccaaaaaacg ttaccaaata cgggagtaac aaacaatgct 4620

tatatgcctt tacttggtat tattggctta gttactagtt ttagtttgct tggcttaaag 4680

gctaagaaag attga 4695

<210> 21
<211> 1561
<212> PRT
<213> Streptococcus mutans

<400> 21

```
Met Lys Val Lys Lys Thr Tyr Gly Phe Arg Lys Ser Lys Ile Ser Lys
1               5                   10                  15

Thr Leu Cys Gly Ala Val Leu Gly Thr Val Ala Ala Val Ser Val Ala
            20                  25                  30

Gly Gln Lys Val Phe Ala Asp Glu Thr Thr Thr Thr Ser Asp Val Asp
        35                  40                  45
```

```
Thr Lys Val Val Gly Thr Gln Thr Gly Asn Pro Ala Thr Asn Leu Pro
    50                  55                  60

Glu Ala Gln Gly Ser Ala Ser Lys Gln Ala Glu Gln Ser Gln Thr Lys
65                  70                  75                  80

Leu Glu Arg Gln Met Val His Thr Ile Glu Val Pro Lys Thr Asp Leu
                85                  90                  95

Asp Gln Ala Ala Lys Asp Ala Lys Ser Ala Gly Val Asn Val Val Gln
            100                 105                 110

Asp Ala Asp Val Asn Lys Gly Thr Val Lys Thr Ala Glu Glu Ala Val
        115                 120                 125

Gln Lys Glu Thr Glu Ile Lys Glu Asp Tyr Thr Lys Gln Ala Glu Asp
    130                 135                 140

Ile Lys Lys Thr Thr Asp Gln Tyr Lys Ser Asp Val Ala Ala His Glu
145                 150                 155                 160

Ala Glu Val Ala Lys Ile Lys Ala Lys Asn Gln Ala Thr Lys Glu Gln
                165                 170                 175

Tyr Gly Lys Asp Met Val Ala His Lys Ala Glu Val Glu Arg Ile Asn
            180                 185                 190

Ala Ala Asn Ala Ala Ser Lys Thr Ala Tyr Glu Ala Lys Leu Ala Gln
        195                 200                 205

Tyr Gln Ala Asp Leu Ala Ala Val Gln Lys Thr Asn Ala Ala Asn Gln
    210                 215                 220

Ala Ser Tyr Gln Lys Ala Leu Ala Ala Tyr Gln Ala Glu Leu Lys Arg
225                 230                 235                 240

Val Gln Glu Ala Asn Ala Ala Ala Lys Ala Ala Tyr Asp Thr Ala Val
                245                 250                 255

Ala Ala Asn Asn Ala Lys Asn Thr Glu Ile Ala Ala Ala Asn Glu Glu
            260                 265                 270

Ile Arg Lys Arg Asn Ala Thr Ala Lys Ala Glu Tyr Glu Thr Lys Leu
        275                 280                 285

Ala Gln Tyr Gln Ala Glu Leu Lys Arg Val Gln Glu Ala Asn Ala Ala
    290                 295                 300
```

```
Asn Glu Ala Asp Tyr Gln Ala Lys Leu Thr Ala Tyr Gln Thr Glu Leu
305                 310                 315                 320

Ala Arg Val Gln Lys Ala Asn Ala Asp Ala Lys Ala Ala Tyr Glu Ala
                325                 330                 335

Ala Val Ala Ala Asn Asn Ala Lys Asn Ala Ala Leu Thr Ala Glu Asn
            340                 345                 350

Thr Ala Ile Lys Gln Arg Asn Glu Asn Ala Lys Ala Thr Tyr Glu Ala
        355                 360                 365

Ala Leu Lys Gln Tyr Glu Ala Asp Leu Ala Ala Val Lys Lys Ala Asn
    370                 375                 380

Ala Ala Asn Glu Ala Asp Tyr Gln Ala Lys Leu Thr Ala Tyr Gln Thr
385                 390                 395                 400

Glu Leu Ala Arg Val Gln Lys Ala Asn Ala Asp Ala Lys Ala Ala Tyr
                405                 410                 415

Glu Ala Ala Val Ala Ala Asn Asn Ala Ala Asn Ala Ala Leu Thr Ala
            420                 425                 430

Glu Asn Thr Ala Ile Lys Lys Arg Asn Ala Asp Ala Lys Ala Asp Tyr
        435                 440                 445

Glu Ala Lys Leu Ala Lys Tyr Gln Ala Asp Leu Ala Lys Tyr Gln Lys
    450                 455                 460

Asp Leu Ala Asp Tyr Pro Val Lys Leu Lys Ala Tyr Glu Asp Glu Gln
465                 470                 475                 480

Ala Ser Ile Lys Ala Ala Leu Ala Glu Leu Glu Lys His Lys Asn Glu
                485                 490                 495

Asp Gly Asn Leu Thr Glu Pro Ser Ala Gln Asn Leu Val Tyr Asp Leu
            500                 505                 510

Glu Pro Asn Ala Asn Leu Ser Leu Thr Thr Asp Gly Lys Phe Leu Lys
        515                 520                 525

Ala Ser Ala Val Asp Asp Ala Phe Ser Lys Ser Thr Ser Lys Ala Lys
    530                 535                 540

Tyr Asp Gln Lys Ile Leu Gln Leu Asp Asp Leu Asp Ile Thr Asn Leu
545                 550                 555                 560

Glu Gln Ser Asn Asp Val Ala Ser Ser Met Glu Leu Tyr Gly Asn Phe
```

565 570 575

Gly Asp Lys Ala Gly Trp Ser Thr Thr Val Ser Asn Asn Ser Gln Val
580 585 590

Lys Trp Gly Ser Val Leu Leu Glu Arg Gly Gln Ser Ala Thr Ala Thr
595 600 605

Tyr Thr Asn Leu Gln Asn Ser Tyr Tyr Asn Gly Lys Lys Ile Ser Lys
610 615 620

Ile Val Tyr Lys Tyr Thr Val Asp Pro Lys Ser Lys Phe Gln Gly Gln
625 630 635 640

Lys Val Trp Leu Gly Ile Phe Thr Asp Pro Thr Leu Gly Val Phe Ala
645 650 655

Ser Ala Tyr Thr Gly Gln Val Glu Lys Asn Thr Ser Ile Phe Ile Lys
660 665 670

Asn Glu Phe Thr Phe Tyr Asp Glu Asp Gly Lys Pro Ile Asn Phe Asp
675 680 685

Asn Ala Leu Leu Ser Val Ala Ser Leu Asn Arg Glu Asn Asn Ser Ile
690 695 700

Glu Met Ala Lys Asp Tyr Thr Gly Lys Phe Val Lys Ile Ser Gly Ser
705 710 715 720

Ser Ile Gly Glu Lys Asn Gly Met Ile Tyr Ala Thr Asp Thr Leu Asn
725 730 735

Phe Arg Gln Gly Gln Gly Gly Ala Arg Trp Thr Met Tyr Thr Arg Ala
740 745 750

Ser Glu Pro Gly Ser Gly Trp Asp Ser Ser Asp Ala Pro Asn Ser Trp
755 760 765

Tyr Gly Ala Gly Ala Ile Arg Met Ser Gly Pro Asn Asn Ser Val Thr
770 775 780

Leu Gly Ala Ile Ser Ser Thr Leu Val Val Pro Ala Asp Pro Thr Met
785 790 795 800

Ala Ile Glu Thr Gly Lys Lys Pro Asn Ile Trp Tyr Ser Leu Asn Gly
805 810 815

Lys Ile Arg Ala Val Asn Leu Pro Lys Val Thr Lys Glu Lys Pro Thr
820 825 830

Pro Pro Val Lys Pro Thr Ala Pro Thr Lys Pro Thr Tyr Glu Thr Glu
835 840 845

Lys Pro Leu Lys Pro Ala Pro Val Ala Pro Asn Tyr Glu Lys Glu Pro
850 855 860

Thr Pro Pro Thr Arg Thr Pro Asp Gln Ala Glu Pro Lys Lys Pro Thr
865 870 875 880

Pro Pro Thr Tyr Glu Thr Glu Lys Pro Leu Glu Pro Ala Pro Val Glu
885 890 895

Pro Ser Tyr Glu Ala Glu Pro Thr Pro Pro Thr Arg Thr Pro Asp Gln
900 905 910

Ala Glu Pro Asn Lys Pro Thr Pro Pro Thr Tyr Glu Thr Glu Lys Pro
915 920 925

Leu Glu Pro Ala Pro Val Glu Pro Ser Tyr Glu Ala Glu Pro Thr Pro
930 935 940

Pro Thr Pro Thr Pro Asp Gln Pro Glu Pro Asn Lys Pro Val Glu Pro
945 950 955 960

Thr Tyr Glu Val Ile Pro Thr Pro Pro Thr Asp Pro Val Tyr Gln Asp
965 970 975

Leu Pro Thr Pro Pro Ser Ile Pro Thr Val His Phe His Tyr Phe Lys
980 985 990

Leu Ala Val Gln Pro Gln Val Asn Lys Glu Ile Arg Asn Asn Asn Asp
995 1000 1005

Val Asn Ile Asp Arg Thr Leu Val Ala Lys Gln Ser Val Val Lys
1010 1015 1020

Phe Gln Leu Lys Thr Ala Asp Leu Pro Ala Gly Arg Asp Glu Thr
1025 1030 1035

Thr Ser Phe Val Leu Val Asp Pro Leu Pro Ser Gly Tyr Gln Phe
1040 1045 1050

Asn Pro Glu Ala Thr Lys Ala Ala Ser Pro Gly Phe Asp Val Ala
1055 1060 1065

Tyr Asp Asn Ala Thr Asn Thr Val Thr Phe Lys Ala Thr Ala Ala
1070 1075 1080

Thr Leu Ala Thr Phe Asn Ala Asp Leu Thr Lys Ser Val Ala Thr
1085 1090 1095

Ile Tyr Pro Thr Val Val Gly Gln Val Leu Asn Asp Gly Ala Thr
1100 1105 1110

Tyr Lys Asn Asn Phe Ser Leu Thr Val Asn Asp Ala Tyr Gly Ile
1115 1120 1125

Lys Ser Asn Val Val Arg Val Thr Thr Pro Gly Lys Pro Asn Asp
1130 1135 1140

Pro Asp Asn Pro Asn Asn Asn Tyr Ile Lys Pro Thr Lys Val Asn
1145 1150 1155

Lys Asn Glu Asn Gly Val Val Ile Asp Gly Lys Thr Val Leu Ala
1160 1165 1170

Gly Ser Thr Asn Tyr Tyr Glu Leu Thr Trp Asp Leu Asp Gln Tyr
1175 1180 1185

Lys Asn Asp Arg Ser Ser Ala Asp Thr Ile Gln Gln Gly Phe Tyr
1190 1195 1200

Tyr Val Asp Asp Tyr Pro Glu Glu Ala Leu Glu Leu Arg Gln Asp
1205 1210 1215

Leu Val Lys Ile Thr Asp Ala Asn Gly Asn Glu Val Thr Gly Val
1220 1225 1230

Ser Val Asp Asn Tyr Thr Ser Leu Glu Ala Ala Pro Gln Glu Ile
1235 1240 1245

Arg Asp Val Leu Ser Lys Ala Gly Ile Arg Pro Lys Gly Ala Phe
1250 1255 1260

Gln Ile Phe Arg Ala Asp Asn Pro Arg Glu Phe Tyr Asp Thr Tyr
1265 1270 1275

Val Lys Thr Gly Ile Asp Leu Lys Ile Val Ser Pro Met Val Val
1280 1285 1290

Lys Lys Gln Met Gly Gln Thr Gly Gly Ser Tyr Glu Asp Gln Ala
1295 1300 1305

Tyr Gln Ile Asp Phe Gly Asn Gly Tyr Ala Ser Asn Ile Val Ile
1310 1315 1320

```
Asn Asn  Val Pro Lys Ile Asn  Pro Lys Lys Asp Val  Thr Leu Thr
    1325                 1330                 1335

Leu Asp  Pro Ala Asp Thr Asn  Asn Val Asp Gly Gln  Thr Ile Pro
    1340                 1345                 1350

Leu Asn  Thr Val Phe Asn Tyr  Arg Leu Ile Gly Gly  Ile Ile Pro
    1355                 1360                 1365

Ala Asn  His Ser Glu Glu Leu  Phe Glu Tyr Asn Phe  Tyr Asp Asp
    1370                 1375                 1380

Tyr Asp  Gln Thr Gly Asp His  Tyr Thr Gly Gln Tyr  Lys Val Phe
    1385                 1390                 1395

Ala Lys  Val Asp Ile Thr Leu  Lys Asn Gly Val Ile  Ile Lys Ser
    1400                 1405                 1410

Gly Thr  Glu Leu Thr Gln Tyr  Thr Thr Ala Glu Val  Asp Thr Thr
    1415                 1420                 1425

Lys Gly  Ala Ile Thr Ile Lys  Phe Lys Glu Ala Phe  Leu Arg Ser
    1430                 1435                 1440

Val Ser  Ile Asp Ser Ala Phe  Gln Ala Glu Ser Tyr  Ile Gln Met
    1445                 1450                 1455

Lys Arg  Ile Ala Val Gly Thr  Phe Glu Asn Thr Tyr  Ile Asn Thr
    1460                 1465                 1470

Val Asn  Gly Val Thr Tyr Ser  Ser Asn Thr Val Lys  Thr Thr Thr
    1475                 1480                 1485

Pro Glu  Asp Pro Ala Asp Pro  Thr Asp Pro Gln Asp  Pro Ser Ser
    1490                 1495                 1500

Pro Arg  Thr Ser Thr Val Ile  Ile Tyr Lys Pro Gln  Ser Thr Ala
    1505                 1510                 1515

Tyr Gln  Pro Ser Ser Val Gln  Lys Thr Leu Pro Asn  Thr Gly Val
    1520                 1525                 1530

Thr Asn  Asn Ala Tyr Met Pro  Leu Leu Gly Ile Ile  Gly Leu Val
    1535                 1540                 1545

Thr Ser  Phe Ser Leu Leu Gly  Leu Lys Ala Lys Lys  Asp
    1550                 1555                 1560
```

<210>  22

<211> 4865
<212> DNA
<213> Streptococcus mutans

<400> 22

```
atttcagcaa aaattgacaa atcaaatcaa ttatattaca attttttaac gtatattaca      60
aaaatatatt tggaagattt attcagattt ggaggattta tgaaagtcaa aaaaacttac     120
ggttttcgta aaagtaaaat tagtaaaaca ctgtgtggtg ctgttctagg aacagtagca     180
gcagtctctg tagcaggaca aaaggttttt gccgatgaaa cgaccactac tagtgatgta     240
gatactaaag tagttggaac acaaactgga aatccagcga ccaatttgcc agaggctcaa     300
ggaagtgcga gtaagcaagc tgaacaaagt caaaccaagc tggagagaca aatggttcat     360
accattgaag tacctaaaac tgatcttgat caagcagcaa aagatgctaa gtctgctggt     420
gtcaatgttg tccaagatgc cgatgttaat aaaggaactg ttaaaacagc tgaagaagca     480
gtccaaaaag aaactgaaat taaagaagat tacacaaaac aagctgagga tattaagaag     540
acaacagatc aatataaatc ggatgtagct gctcatgagg cagaagttgc taaaatcaaa     600
gctaaaaatc aggcaactaa agaacagtat ggaaaagata tggtagctca taaagccgag     660
gttgaacgca ttaatgctgc aaatgctgcc agtaaaacag cttatgaagc taaattggct     720
caatatcaag cagatttagc agccgttcaa aaaaccaatg ctgccaatca agcatcctat     780
caaaaagccc ttgctgctta tcaggctgaa ctgaaacgtg ttcaggaagc taatgcagcc     840
gccaaagccg cttatgatac tgctgtagca gcaaataatg ccaaaaatac agaaattgcc     900
gctgccaatg aagaaattag aaaacgcaat gcaacggcca aagctgaata tgagactaag     960
ttagctcaat atcaagctga actaaagcgt gttcaggaag ctaatgccgc aaacgaagca    1020
gactatcaag ctaaattgac cgcctatcaa acagagcttg ctcgcgttca gaaagccaat    1080
gcagatgcta aagcggccta tgaagcagct gtagcagcaa ataatgccaa aaatgcggca    1140
cttacagctg aaaatactgc aattaagcaa cgcaatgaga atgctaaggc gacttatgaa    1200
gctgcactca agcaatatga ggctgatttg gcagcggtga aaaaagctaa tgccgcaaac    1260
gaagcagact atcaagctaa attgaccgcc tatcaaacag agctcgctcg cgttcaaaag    1320
gccaatgcgg atgctaaagc ggcctatgaa gcagctgtag cagcaaataa tgccgcaaat    1380
gcagcgctca cagctgaaaa tactgcaatt aagaagcgca atgcggatgc taaagctgat    1440
tacgaagcaa aacttgctaa gtatcaagca gatcttgcca aatatcaaaa agatttagca    1500
gactatccag ttaagttaaa ggcatacgaa gatgaacaag cttctattaa agctgcactg    1560
gcagaacttg aaaaacataa aaatgaagac ggaaacttaa cagaaccatc tgctcaaaat    1620
ttggtctatg atcttgagcc aaatgcgaac ttatctttga caacagatgg gaagttcctt    1680
aaggcttctg ctgtggatga tgcttttagc aaaagcactt caaaagcaaa atatgaccaa    1740
aaaattcttc aattagatga tctagatatc actaacttag aacaatctaa tgatgttgct    1800
```

tcttctatgg agctttatgg caattttggt gataaagctg gctggtcaac gacagtaagc 1860
aataactcac aggttaaatg gggatcggta cttttagagc gcggtcaaag cgcaacagct 1920
acatacacta acctgcagaa ttcttattac aatggtaaaa agatttctaa aattgtctac 1980
aagtatacag tggaccctaa gtccaagttt caaggtcaaa aggtttggtt aggtattttt 2040
accgatccaa ctttaggtgt tttgcttcc gcttatacag gtcaagttga aaaaaacact 2100
tctattttta ttaaaaatga attcactttc tatgacgaag atggaaaacc aattaatttt 2160
gataatgccc ttctatcagt agcttctctt aaccgagaaa ataattctat tgagatggcc 2220
aaagattata cgggtaaatt tgtcaaaatc tctggatcat ctatcggtga aaagaatggc 2280
atgatttatg ctacagatac tctcaacttt aggcagggtc aaggtggtgc tcgttggacc 2340
atgtatacca gagctagcga accgggatct ggctgggata gttcagatgc gcctaactct 2400
tggtatggtg ctggtgctat ccgcatgtct ggtcctaata acagtgtgac tttgggtgct 2460
atctcatcaa cacttgttgt gcctgctgat cctacaatgg caattgaaac cggcaaaaaa 2520
ccaaatattt ggtattcatt aaatggtaaa atccgtgcgg ttaatcttcc taaagttact 2580
aaggaaaaac ccacacctcc ggttaaacca acagctccaa ctaaaccaac ttatgaaaca 2640
gaaaagccat taaaaccggc accagtagct ccaaattatg aaaaggagcc aacaccaccg 2700
acaagaacac cggatcaagc agagccaaag aaacccactc cgccgaccta tgaaacagaa 2760
aagccgttgg agccagcacc tgttgagcca agctatgaag cagagccaac accgccgaca 2820
aggacaccgg atcaggcaga gccaaataaa cccacaccgc cgacctatga aacagaaaag 2880
ccgttggagc cagcacctgt tgagccaagc tatgaagcag agccaacgcc accgacacca 2940
acaccagatc aaccagaacc aaacaaacct gttgagccaa cttatgaggt tattccaaca 3000
ccgccgactg atcctgttta tcaagatctt ccaacacctc catctatacc aactgttcat 3060
ttccattact ttaaactagc tgttcagccg caggttaaca aagaaattag aaacaataac 3120
gatgttaata ttgacagaac tttggtggct aaacaatctg ttgttaagtt ccagctgaag 3180
acagcagatc tccctgctgg acgtgatgaa acaacttcct ttgtcttggt agatcccctg 3240
ccatctggtt atcaatttaa tcctgaagct acaaaagctg ccagccctgg ctttgatgtc 3300
gcttatgata atgcaactaa tacagtcacc ttcaaggcaa ctgcagcaac tttggctacg 3360
tttaatgctg atttgactaa gtcagtggca acgatttatc caacagtggt cggacaagtt 3420
cttaatgatg gcgcaactta taagaataat ttctcgctca cagtcaatga tgcttatggc 3480
attaaatcca atgttgttcg ggtgacaact cctggtaaac caaatgatcc agataaccca 3540
aataataatt acattaagcc aactaaggtt aataaaaatg aaaatggcgt tgttattgat 3600
ggtaaaacag ttcttgccgg ttcaacgaat tattatgagc taacttggga tttggatcaa 3660
tataaaaacg accgctcttc agcagatacc attcaacaag gattttacta tgtagatgat 3720
tatccagaag aagcgcttga attgcgtcag gatttagtga agattacaga tgctaatggc 3780

aatgaagtta ctggtgttag tgtggataat tatactagtc ttgaagcagc ccctcaagaa 3840
attagagatg ttctttctaa ggcaggaatt agacctaaag gtgctttcca aattttccgt 3900
gccgataatc caagagaatt ttatgatact tatgtcaaaa ctggaattga tttgaagatt 3960
gtatcaccaa tggttgttaa aaaacaaatg ggacaaacag gcgggagtta tgaagatcaa 4020
gcttaccaaa ttgactttgg taatggttat gcatcaaata tcgttatcaa taatgttcct 4080
aagattaacc ctaagaaaga tgtgacctta acacttgatc cggctgatac aaataatgtt 4140
gatggtcaga ctattccact taatacagtc tttaattacc gtttgattgg tggcattatc 4200
cctgcaaatc actcagaaga actctttgaa tacaatttct atgatgatta tgatcaaaca 4260
ggagatcact atactggtca gtataaagtt tttgccaagg ttgatatcac tcttaaaaac 4320
ggtgttatta tcaagtcagg tactgagtta actcagtata cgacagcgga agttgatacc 4380
actaaaggtg ctatcacaat taagttcaag gaagcctttc tgcgttctgt ttcaattgat 4440
tcagccttcc aagctgaaag ttatatccaa atgaaacgta ttgcggttgg tacttttgaa 4500
aatacctata ttaatactgt caatggggta acttacagtt caaatacagt gaaaacaact 4560
actcctgagg atcctgcaga ccctactgat ccgcaagatc catcatcacc gcggacttca 4620
actgtaatta tctacaaacc tcaatcaact gcttatcaac caagctctgt ccaaaaaacg 4680
ttaccaaata cgggagtaac aaacaatgct tatatgcctt tacttggtat tattggctta 4740
gttactagtt ttagtttgct tggcttaaag gctaagaaag attgacagca tagatattac 4800
attagaatta aaaagtgaga tgaagcgata aatcacagat tgagctttta tctcattttt 4860
tgatt 4865

<210> 23
<211> 330
<212> PRT
<213> Neisseria meningitidis

<400> 23

```
Met Lys Thr Ser Ile Arg Tyr Ala Leu Leu Ala Ala Ala Leu Thr Ala
1               5                   10                  15

Ala Thr Pro Ala Leu Ala Asp Ile Thr Val Tyr Asn Gly Gln His Lys
            20                  25                  30

Glu Ala Ala Gln Ala Val Ala Asp Ala Phe Thr Arg Ala Thr Gly Ile
        35                  40                  45

Lys Val Lys Leu Asn Ser Ala Lys Gly Asp Gln Leu Ala Gly Gln Ile
    50                  55                  60

Lys Glu Glu Gly Ser Arg Ser Pro Ala Asp Val Phe Tyr Ser Glu Gln
65                  70                  75                  80
```

```
Ile Pro Ala Leu Ala Thr Leu Ser Ala Ala Asn Leu Leu Glu Pro Leu
                85                  90                  95

Pro Ala Ser Thr Ile Asn Glu Thr Arg Gly Lys Gly Val Pro Val Ala
            100                 105                 110

Ala Lys Lys Asp Trp Val Ala Leu Ser Gly Arg Ser Arg Val Val Val
        115                 120                 125

Tyr Asp Thr Arg Lys Leu Ser Glu Lys Asp Leu Glu Lys Ser Val Leu
    130                 135                 140

Asn Tyr Ala Thr Pro Lys Trp Lys Asn Arg Ile Gly Tyr Ala Pro Thr
145                 150                 155                 160

Ser Gly Ala Phe Leu Glu Gln Val Val Ala Ile Val Lys Leu Lys Gly
                165                 170                 175

Glu Ala Ala Ala Leu Lys Trp Leu Lys Ala Leu Lys Glu Tyr Gly Lys
            180                 185                 190

Pro Tyr Ala Lys Asn Ser Val Ala Leu Gln Ala Val Glu Asn Gly Glu
        195                 200                 205

Ile Asp Ala Ala Leu Ile Asn Asn Tyr Tyr Trp His Ala Phe Ala Arg
    210                 215                 220

Glu Lys Gly Val Gln Asn Val His Thr Arg Leu Asn Phe Val Arg His
225                 230                 235                 240

Arg Asp Pro Gly Ala Leu Val Thr Tyr Ser Gly Ala Val Leu Lys Ser
                245                 250                 255

Ser Gln Asn Lys Asp Glu Ala Lys Lys Phe Val Ala Phe Leu Ala Gly
            260                 265                 270

Lys Glu Gly Gln Arg Ala Leu Thr Ala Val Arg Ala Glu Tyr Pro Leu
        275                 280                 285

Asn Pro His Val Val Ser Thr Phe Asn Leu Glu Pro Ile Ala Lys Leu
    290                 295                 300

Glu Ala Pro Gln Val Ser Ala Thr Thr Val Ser Glu Lys Glu His Ala
305                 310                 315                 320

Thr Arg Leu Leu Glu Gln Ala Gly Met Lys
                325                 330
```

<210> 24
<211> 1072
<212> DNA
<213> Neisseria meningitidis

<400> 24

```
atgaaaacat ctatccgata cgcactgctt gccgcagccc tgaccgccgc aacccccgcg      60
ctggcagaca ttaccgtgta caacggccaa cacaaagaag cagcacaagc cgttgcagat     120
gcctttaccc gggctaccgg catcaaagtc aaactcaaca gtgccaaagg cgaccagctt     180
gccggtcaaa tcaaagaaga aggcagccga agccccgccg acgtattcta ttccgaacaa     240
atcccggcac tcgccaccct ttccgcagcc aacctcctag agcccctgcc cgcctccacc     300
atcaacgaaa cacgcggcaa aggcgtgccg gttgccgcca aaaaagactg ggtggcactg     360
agcggacgtt cgcgcgtcgt cgtttacgac acccgcaaac tgtctgaaaa agatttggaa     420
aaatccgtcc tgaattacgc cacgccgaaa tggaaaaacc gcatcggtta cgcccccact     480
tccggcgcgt tcttggaaca ggttgtcgcc atcgtcaaac tgaaaggcga agcggccgca     540
ttgaaatggc tcaaagcact gaaagaatac ggcaagcctt acgctaaaaa ctccgtcgcc     600
cttcaagcgg ttgaaaacgg cgaaatcgat gccgccctca tcaacaacta ctactggcac     660
gctttcgcgc gtgaaaaagg cgtacaaaat gtccacaccc gcctgaattt cgtccgccac     720
agagatcccg gcgcactcgt tacctattcc ggcgcagtgt taaaatcctc ccaaaacaag     780
gatgaggcga aaaaattcgt cgccttcctc gccggcaagg aaggacagcg cgccctgacc     840
gccgtccgtg ccgaatatcc tttgaatccg cacgtggtat ccactttcaa tttggaaccc     900
atcgccaagt tggaagcacc ccaagtgtcc gccaccactg tttccgaaaa agaacacgcc     960
acccggctgc ttgagcaagc cggtatgaaa taagccgttt tcggattgtc aaacgggtgg    1020
acatttatac tgtccgcccg ttttgccgat gaaaaacact atgtctccta aa            1072
```

<210> 25
<211> 556
<212> PRT
<213> Streptococcus mutans

<400> 25

```
Met Lys Arg Lys Gly Leu Arg Arg Leu Leu Lys Phe Phe Gly Thr Val
1               5                   10                  15

Ala Ile Ile Leu Pro Met Phe Phe Ile Ala Leu Thr Lys Ala Gln Ala
            20                  25                  30

Ser Asp Val Ser Ser Asn Ile Ser Ser Leu Thr Val Ser Pro Thr Gln
        35                  40                  45

Ile Asn Asp Gly Gly Lys Thr Thr Val Arg Phe Glu Phe Asp Glu His
    50                  55                  60
```

```
Ala Gln Asn Ile Lys Ala Gly Asp Thr Ile Thr Val Asn Trp Gln Asn
65                  70                  75                  80

Ser Gly Thr Val Arg Gly Thr Gly Tyr Thr Lys Thr Ile Lys Leu Glu
                85                  90                  95

Val Gln Gly Lys Tyr Val Gly Asp Leu Val Val Thr Gln Asp Lys Ala
            100                 105                 110

Val Val Thr Phe Asn Asp Ser Ile Thr Gly Leu Gln Asn Ile Thr Gly
        115                 120                 125

Trp Gly Glu Phe Glu Ile Glu Gly Arg Asn Phe Thr Asp Thr Thr Thr
    130                 135                 140

Gly Asn Thr Gly Ser Phe Gln Val Thr Ser Gly Gly Lys Thr Ala Glu
145                 150                 155                 160

Val Thr Val Val Lys Ser Ala Ser Gly Thr Thr Gly Val Phe Tyr Tyr
                165                 170                 175

Lys Thr Gly Asp Met Gln Thr Asp Asp Thr Asn His Val Arg Trp Phe
            180                 185                 190

Leu Asn Ile Asn Asn Glu Asn Ala Tyr Val Asp Ser Asp Ile Arg Ile
        195                 200                 205

Glu Asp Asp Ile Gln Ser Gly Gln Thr Leu Asp Ile Asp Ser Phe Asp
    210                 215                 220

Ile Thr Val Asn Gly Ser Glu Ser Tyr His Gly Gln Glu Gly Ile Asn
225                 230                 235                 240

Gln Leu Ala Gln Arg Tyr Gly Ala Thr Ile Ser Ala Asp Pro Ala Ser
                245                 250                 255

Gly His Ile Ser Val Tyr Ile Pro Gln Gly Tyr Ala Ser Leu Asn Arg
            260                 265                 270

Phe Ser Ile Met Tyr Leu Thr Lys Val Asp Asn Pro Asp Gln Lys Thr
        275                 280                 285

Phe Glu Asn Asn Ser Lys Ala Trp Tyr Lys Glu Asn Gly Lys Asp Ala
    290                 295                 300

Val Asp Gly Lys Glu Phe Asn His Ser Val Ala Asn Val Asn Ala Ala
305                 310                 315                 320
```

```
Gly Gly Val Asp Gly Arg Thr Thr Thr Thr Thr Glu Lys Pro Thr Thr
                325                 330                 335

Thr Thr Glu Ala Pro Thr Thr Thr Glu Thr Pro Thr Thr Thr Glu Ala
            340                 345                 350

Pro Thr Thr Thr Glu Ala Pro Thr Thr Thr Glu Thr Pro Thr Thr Thr
        355                 360                 365

Glu Ala Pro Thr Thr Glu Ala Pro Thr Thr Thr Glu Ala Pro Thr Thr
    370                 375                 380

Thr Glu Ala Pro Thr Thr Thr Glu Ala Pro Thr Thr Thr Glu Ala Pro
385                 390                 395                 400

Thr Thr Thr Glu Ala Pro Thr Thr Thr Glu Ala Pro Thr Thr Thr Glu
                405                 410                 415

Ala Pro Thr Thr Thr Glu Ala Pro Thr Thr Thr Glu Ala Pro Thr Thr
            420                 425                 430

Thr Glu Ala Pro Thr Thr Thr Glu Ala Pro Thr Thr Thr Glu Ala Pro
        435                 440                 445

Thr Thr Thr Glu Ala Pro Thr Thr Thr Glu Ala Pro Thr Thr Thr Gly
    450                 455                 460

Ala Pro Thr Thr Thr Glu Ala Pro Thr Thr Thr Glu Ala Ser Ser Glu
465                 470                 475                 480

Thr Thr Lys Ala Glu Glu Lys Thr Thr Glu Val Lys Glu Pro Glu Lys
                485                 490                 495

Thr Thr Thr Thr Ala Pro Ala Gly Lys Thr Ser Asn Lys Pro Asn Lys
            500                 505                 510

Pro Ser Gly Lys Gln Asn Ala Gly Ala Lys Gly Leu Pro Ser Thr Gly
        515                 520                 525

Glu Glu Ser Gly Thr Val Leu Ser Leu Leu Gly Leu Ala Ala Val Ser
    530                 535                 540

Met Thr Gly Leu Phe Tyr Tyr Arg Lys His His Asn
545                 550                 555
```

<210> 26
<211> 1782

<212> DNA
<213> Streptococcus mutans

<400> 26

```
tacaaagaaa tgaaagatgt tataatagat ttgtaatatt cttgttacaa gaaaggacta     60
aaaatatgaa aagaaaaggt ttacgaagac tattaaagtt ttttggaacc gttgccatca    120
ttttgccaat gtttttcata gctttaacga aagctcaggc aagtgatgtc agcagtaaca    180
tttcatcgct gacggtatca ccgactcaga ttaatgatgg cggtaagacc accgttcgct    240
ttgagtttga tgagcatgct caaaatatta aagcaggcga caccattact gttaactggc    300
agaattcagg aacagtcaga ggaacaggtt atacgaaaac cattaagctg gaggttcagg    360
gcaagtatgt tggtgatttg gtagttacgc aagacaaagc agttgttact ttcaatgaca    420
gtattactgg cttgcagaat atcaccggct ggggtgaatt tgaaatcgaa ggccggaatt    480
ttactgacac tactaccgga aatactggca gcttccaagt taccagcggc ggcaagacag    540
ctgaggttac tgtcgttaaa tctgcttcag ggactaccgg cgttttctac tataagactg    600
gggatatgca gacagatgac accaatcatg tgcgctggtt tttgaatatc aacaatgaga    660
atgcttatgt agacagtgat attcgtattg aagatgacat tcagtctggt caaactttgg    720
atatagacag ttttgatatt actgtaaatg gcagtgagtc ttatcacggt caagaaggta    780
ttaatcagct tgcccaaaga tatggtgcaa ctatttcagc tgatccggct agtggccata    840
tcagtgttta tattcctcaa ggctatgctt ctttgaatcg ctttagcatc atgtacttga    900
ctaaagttga caatcctgat caaaagacgt ttgaaaataa cagtaaggct tggtataagg    960
aaaacggtaa agatgctgtt gatggtaagg aatttaacca ttctgtagct aatgttaatg   1020
ccgccggcgg tgtggacgga agaacaacca ctactacaga aaagccaaca acgacgacag   1080
aggctccaac aacaacggaa actccaacga caacagaggc tccaacgacg acagaggctc   1140
caacaacaac ggaaactcca acgacaacag aggctccaac aacggaagct ccaacgacaa   1200
cagaggctcc aacgacaaca gaggctccaa caacaacgga agctccaacg acaacagaag   1260
ctccaacgac aacagaagct ccaacaacaa cggaagctcc aacgacaaca gaggctccaa   1320
cgacaacaga agctccaaca acaacggaag ctccaacgac aacagaggct ccaacaacaa   1380
cggaagctcc aacaacaacg gaagctccaa caacaacaga ggctccaaca acgacggaag   1440
ctccaacgac aacaggggct ccaacaacaa cggaagctcc aacgacgaca gaggcatctt   1500
cagaaacaac aaaagctgaa gaaaagacta ctgaagttaa ggaaccagaa aaaacaacga   1560
caacagctcc agcaggtaag acttcaaaca aacctaataa gccatcaggc aaacaaaatg   1620
ctggtgctaa gggacttcca agcacaggcg aagaaagcgg cactgttttg tcacttctcg   1680
gtcttgcagc tgtctcaatg actggtctat tctattaccg taaacatcat aactgatatt   1740
gattaaaaat aggatgaaag aggcagggac aagagtcttt gc                       1782
```

<210> 27
<211> 165
<212> PRT
<213> Streptococcus mutans

<400> 27

```
Val Thr Ser Gly Gly Lys Thr Ala Glu Val Thr Val Val Lys Ser Ala
1               5                   10                  15

Ser Gly Thr Thr Gly Val Phe Tyr Tyr Lys Thr Gly Asp Met Gln Thr
            20                  25                  30

Asp Asp Thr Asn His Val Arg Trp Phe Leu Asn Ile Asn Asn Glu Asn
        35                  40                  45

Ala Tyr Val Asp Ser Asp Ile Arg Ile Glu Asp Asp Ile Gln Ser Gly
    50                  55                  60

Gln Thr Leu Asp Ile Asp Ser Phe Asp Ile Thr Val Asn Gly Ser Glu
65                  70                  75                  80

Ser Tyr His Gly Gln Glu Gly Ile Asn Gln Leu Ala Gln Arg Tyr Gly
                85                  90                  95

Ala Thr Ile Ser Ala Asp Pro Ala Ser Gly His Ile Ser Val Tyr Ile
            100                 105                 110

Pro Gln Gly Tyr Ala Ser Leu Asn Arg Phe Ser Ile Met Tyr Leu Thr
        115                 120                 125

Lys Val Asp Asn Pro Asp Gln Lys Thr Phe Glu Asn Asn Ser Lys Ala
    130                 135                 140

Trp Tyr Lys Glu Asn Gly Lys Asp Ala Val Asp Gly Lys Glu Phe Asn
145                 150                 155                 160

His Ser Val Ala Asn
                165
```

<210> 28
<211> 495
<212> DNA
<213> Streptococcus mutans

<400> 28

```
gttaccagcg gcggcaagac agctgaggtt actgtcgtta aatctgcttc agggactacc     60
ggcgttttct actataagac tggggatatg cagacagatg acaccaatca tgtgcgctgg    120
tttttgaata tcaacaatga gaatgcttat gtagacagtg atattcgtat tgaagatgac    180
attcagtctg gtcaaacttt ggatatagac agttttgata ttactgtaaa tggcagtgag    240
tcttatcacg gtcaagaagg tattaatcag cttgcccaaa gatatggtgc aactatttca    300
gctgatccgg ctagtggcca tatcagtgtt tatattcctc aaggctatgc ttctttgaat    360
cgctttagca tcatgtactt gactaaagtt gacaatcctg atcaaaagac gtttgaaaat    420
aacagtaagg cttggtataa ggaaaacggt aaagatgctg ttgatggtaa ggaatttaac    480
cattctgtag ctaat                                                     495
```

<210> 29
<211> 538
<212> PRT
<213> Streptococcus mutans

<400> 29

```
Met Lys Arg Lys Gly Leu Arg Arg Leu Leu Lys Phe Phe Gly Thr Val
1               5                   10                  15

Ala Ile Ile Leu Pro Met Phe Phe Ile Ala Leu Thr Lys Ala Gln Ala
            20                  25                  30

Ser Asp Val Ser Ser Asn Ile Ser Ser Leu Thr Val Ser Pro Thr Gln
        35                  40                  45

Ile Asn Asp Gly Gly Lys Thr Thr Val Arg Phe Glu Phe Asp Glu His
    50                  55                  60

Ala Gln Asn Ile Lys Ala Gly Asp Thr Ile Thr Val Asn Trp Gln Asn
65                  70                  75                  80

Ser Gly Thr Val Arg Gly Thr Gly Tyr Thr Lys Thr Ile Lys Leu Glu
                85                  90                  95

Val Gln Gly Lys Tyr Val Gly Asp Leu Val Val Thr Gln Asp Lys Ala
            100                 105                 110

Val Val Thr Phe Asn Asp Ser Ile Thr Gly Leu Gln Asn Ile Thr Gly
        115                 120                 125

Trp Gly Glu Phe Glu Ile Glu Gly Arg Asn Phe Thr Asp Thr Thr Thr
    130                 135                 140

Gly Asn Thr Gly Ser Phe Gln Val Thr Ser Gly Gly Lys Thr Ala Glu
145                 150                 155                 160

Val Thr Val Val Lys Ser Ala Ser Gly Thr Thr Gly Val Phe Tyr Tyr
                165                 170                 175

Lys Thr Gly Asp Met Gln Thr Asp Asp Thr Asn His Val Arg Trp Phe
            180                 185                 190

Leu Asn Ile Asn Asn Glu Asn Ala Tyr Val Asp Ser Asp Ile Arg Ile
```

```
        195                 200                 205

Glu Asp Asp Ile Gln Ser Gly Gln Thr Leu Asp Ile Asp Ser Phe Asp
    210                 215                 220

Ile Thr Val Asn Gly Ser Glu Ser Tyr Arg Gly Gln Glu Gly Ile Asn
225                 230                 235                 240

Gln Leu Ala Gln Arg Tyr Gly Ala Thr Ile Ser Ala Asp Pro Ala Ser
                245                 250                 255

Gly His Ile Ser Val Tyr Ile Pro Gln Gly Tyr Ala Ser Leu Asn Arg
            260                 265                 270

Phe Ser Ile Met Tyr Leu Thr Lys Val Asp Asn Pro Asp Gln Lys Thr
        275                 280                 285

Phe Glu Asn Asn Ser Lys Ala Trp Tyr Lys Glu Asn Gly Lys Asp Ala
    290                 295                 300

Val Asp Gly Lys Glu Phe Asn His Ser Val Ala Asn Val Asn Ala Ala
305                 310                 315                 320

Gly Gly Val Asp Gly Arg Thr Thr Thr Thr Thr Glu Lys Pro Thr Thr
                325                 330                 335

Thr Thr Glu Ala Pro Thr Thr Thr Glu Thr Pro Thr Thr Thr Glu Ala
            340                 345                 350

Pro Thr Thr Thr Glu Ser Pro Thr Thr Thr Glu Ala Pro Thr Thr Thr
        355                 360                 365

Glu Ala Pro Thr Thr Thr Glu Ala Pro Thr Thr Thr Glu Ala Pro Thr
    370                 375                 380

Thr Thr Glu Ala Pro Thr Thr Thr Glu Ala Pro Thr Thr Thr Glu Ala
385                 390                 395                 400

Pro Thr Thr Thr Glu Ala Pro Thr Thr Thr Glu Ala Pro Thr Thr Thr
                405                 410                 415

Glu Ala Pro Thr Thr Thr Glu Ala Pro Thr Thr Thr Glu Ala Pro Thr
            420                 425                 430

Thr Thr Glu Ala Pro Thr Thr Thr Glu Ala Pro Thr Thr Thr Glu Ser
        435                 440                 445

Pro Thr Thr Thr Glu Ala Pro Thr Thr Thr Glu Val Ser Ser Glu Thr
    450                 455                 460
```

```
Thr Lys Ala Glu Glu Thr Thr Thr Lys Val Lys Glu Pro Glu Lys Thr
465                 470                 475                 480

Thr Thr Ser Val Pro Ala Gly Thr Thr Ser Asn Lys Pro Asn Lys Pro
                485                 490                 495

Ser Gly Lys Gln Gly Ala Gly Thr Lys Gly Leu Pro Ser Thr Gly Glu
            500                 505                 510

Glu Ser Gly Ile Val Leu Ser Leu Leu Gly Leu Ala Thr Val Ser Val
        515                 520                 525

Thr Gly Leu Val Tyr Arg Lys Tyr His Ser
    530                 535
```

<210> 30
<211> 1617
<212> DNA
<213> Streptococcus mutans

<400> 30

```
atgaaaagaa aaggtttacg aagactatta aagtttttg gaaccgttgc catcattttg      60
ccaatgtttt tcatagcttt aacgaaagct caggcaagtg atgtcagcag taacatttca     120
tcgctgacgg tatcaccgac tcagattaat gatggcggta agaccaccgt tcgctttgag     180
tttgatgagc atgctcaaaa tattaaagca ggcgacacca ttactgttaa ctggcagaat     240
tcaggaacag tcagaggaac aggttatacg aaaaccatta agctggaggt tcagggcaag     300
tatgttggtg atttggtagt tacgcaagac aaagcagttg ttactttcaa tgacagtatt     360
actggcttgc agaatatcac cggctggggt gaatttgaaa tcgaaggccg gaattttact     420
gacactacta ccggaaatac tggcagcttc caagttacca gcggcggcaa gacagctgag     480
gttactgtcg ttaaatctgc ttcagggact accggcgttt tctactataa gactggggat     540
atgcagacag atgacaccaa tcatgtgcgc tggtttttga atatcaacaa tgagaatgct     600
tatgtagaca gtgatattcg tattgaagat gacattcagt ctggtcaaac tttggatata     660
gacagttttg atattactgt aaatggcagt gagtcttatc gcggtcaaga aggtattaat     720
cagcttgccc aaagatatgg tgcaactatt tcagctgatc cggctagtgg ccatatcagt     780
gtttatattc ctcaaggcta tgcttctttg aatcgcttta gcatcatgta cttgactaaa     840
gttgacaatc ctgatcaaaa gacgtttgaa aataacagta aggcttggta taaggaaaac     900
ggtaaagatg ctgttgatgg taaggaattt aaccattctg tagctaatgt taatgccgcc     960
ggcggtgtgg acggaagaac aaccactact acagaaaagc caacaacgac gacagaggct    1020
ccaacaacaa cggaaactcc aacgacaaca gaggctccaa cgacaacaga gtctccaaca    1080
acaacggaag ctccaacgac aacagaagct ccaacaacaa cggaagctcc aacgacaaca    1140
gaggctccaa cgacaacaga ggctccaaca acaacggaag ctccaacgac aacagaggct    1200
ccaacaacaa cggaagctcc aacgacaaca gaagctccaa cgacaacaga ggctccaaca    1260
acaacggaag ctccaacgac aacagaagct ccaacgacaa cagaagctcc aacaacaacg    1320
gaagctccaa cgacaacaga gtctccaaca acaacggaag ctccaacaac aacggaagta    1380
tcttcagaaa caactaaagc tgaagaaaca actactaaag ttaaggaacc agaaaaaaca    1440
acgacatcag ttccagcagg tacaacttca aacaaaccta ataagccatc aggcaaacaa    1500
ggtgctggta ccaagggact tccaagcaca ggcgaagaaa gcggtattgt tttgtcactt    1560
ctcggtcttg caactgtctc agtgactggt ctagtttacc gtaaatatca tagctga       1617
```

<210> 31
<211> 165
<212> PRT
<213> Streptococcus mutans

<400> 31

Val Thr Ser Gly Gly Lys Thr Ala Glu Val Thr Val Val Lys Ser Ala
1               5                   10                  15

Ser Gly Thr Thr Gly Val Phe Tyr Tyr Lys Thr Gly Asp Met Gln Thr
            20                  25                  30

Asp Asp Thr Asn His Val Arg Trp Phe Leu Asn Ile Asn Asn Glu Asn
        35                  40                  45

Ala Tyr Val Asp Ser Asp Ile Arg Ile Glu Asp Asp Ile Gln Ser Gly
    50                  55                  60

Gln Thr Leu Asp Ile Asp Ser Phe Asp Ile Thr Val Asn Gly Ser Glu
65                  70                  75                  80

Ser Tyr Arg Gly Gln Glu Gly Ile Asn Gln Leu Ala Gln Arg Tyr Gly
                85                  90                  95

Ala Thr Ile Ser Ala Asp Pro Ala Ser Gly His Ile Ser Val Tyr Ile
            100                 105                 110

Pro Gln Gly Tyr Ala Ser Leu Asn Arg Phe Ser Ile Met Tyr Leu Thr
        115                 120                 125

Lys Val Asp Asn Pro Asp Gln Lys Thr Phe Glu Asn Asn Ser Lys Ala
    130                 135                 140

Trp Tyr Lys Glu Asn Gly Lys Asp Ala Val Asp Gly Lys Glu Phe Asn
145                 150                 155                 160

His Ser Val Ala Asn
                165

<210> 32
<211> 495
<212> DNA
<213> Streptococcus mutans

<400> 32

```
gttaccagcg gcggcaagac agctgaggtt actgtcgtta aatctgcttc agggactacc     60
ggcgttttct actataagac tggggatatg cagacagatg acaccaatca tgtgcgctgg    120
tttttgaata tcaacaatga gaatgcttat gtagacagtg atattcgtat tgaagatgac    180
attcagtctg gtcaaacttt ggatatagac agttttgata ttactgtaaa tggcagtgag    240
tcttatcgcg gtcaagaagg tattaatcag cttgcccaaa gatatggtgc aactatttca    300
gctgatccgg ctagtggcca tatcagtgtt tatattcctc aaggctatgc ttctttgaat    360
cgctttagca tcatgtactt gactaaagtt gacaatcctg atcaaaagac gtttgaaaat    420
aacagtaagg cttggtataa ggaaaacggt aaagatgctg ttgatggtaa ggaatttaac    480
cattctgtag ctaat                                                     495
```

## Description

### TECHNICAL FIELD

The present invention is directed to a method of detecting an oral bacterium that causes hemorrhage aggravation, a method of screening for a subject at a high risk of hemorrhage aggravation, a method of determining the risk of hemorrhage aggravation in a subject, as well as detection reagents and kits for the use in these methods.

### BACKGROUND

Conditions which involve hemorrhage through vascular injuries include such as hemorrhage by a rupture of a blood vessel that caused by a traumatic injury or pressure, hemorrhage at delivery and intracerebral hemorrhage. In a case of intracerebral hemorrhage, for instance, a severe disorder may be brought about by an injury of the neuronal tissue due to compression or necrosis of the brain associated with hemorrhage, or by neurologic symptoms due to a vascular spasm in cerebrum induced by bleeding in a case of subarachnoid hemorrhage etc.. In order to improve the prognosis of hemorrhage, an effective treatment of hemorrhage (hemostasis) as well as the prevention of hemorrhage aggravation is necessary, and diagnosis of the risk of hemorrhage aggravation is important.

Markers used in the diagnosis of a disease which involves hemorrhage include, for example, Apo C-III, serum amyloid A, Apo C-I, antithrombin III fragment and Apo A-I (Patent literature 1) for the diagnoses of the possibility of a stroke, cerebrospinal fluid markers of cerebral ischemia such as adenylate kinase as well as β-thromboglobulin, vascular cell adhesion molecule (VCAM) and atriuretic peptide for the diagnoses of the prognosis of a stroke and cerebral injury, and von Willebrand factor (vWF), vascular endothelial growth factor (VEGF) and matrix-metalloprotease-9 (MMP-9) for the prediction of cerebral vascular spasm which occurs later (Patent literature 2). However, these are all markers for detecting already-happening bleeding in vivo, and cannot diagnose the risk of hemorrhage aggravation.

Accordingly, there have been needs for the establishment of a method of determining or screening a risk of causing aggravation of hemorrhage or a subject with such a risk, and a method of preventing or treating.

### PRIOR ART LITERATURES

### PATENT LITERATURES

[Patent literature 1] JP A No. 2007-502401
[Patent literature 2] JP A No. 2005-522669

Sato Y et al., (Journal of Dental Resarch, International Association for Dental Research, US, Vol. 83, No. 7, 2004-07-01, pages 534-539) described that *S. mutans* was regarded as the primary ethiologic agent of human dental caries, as it resides in the oral biofilm dental plaque. These authors have further charactherized a new gene designated *cnm* which encodes a new strain-specific member of the collagen-binding adhesion family.

### SUMMARY OF INVENTION

### PROBLEMS TO BE SOLVED BY THE INVENTION

Accordingly, an object of the invention is to identify the responsible factor that causes aggravation of hemorrhage, and to construct a system for rapidly and readily specifying a patient having a risk of hemorrhage aggravation. Another object of the invention is to prevent the aggravation of hemorrhage in an individual having such a risk.

### MEANS FOR SOLVING THE PROBLEMS

The inventors carried out an intensive study to achieve the aforementioned objects and found that hemorrhage is aggravated in a subject who has been infected with a particular strain of *S. mutans.* By additional studies the inventors found that the most severe virulence is exerted by bacterial strains that do not carry a protein antigen PA (Protein Antigen, also known as PAc, SpaP, antigen I/II, antigen B, SR, IF, P1, MSL-1), i.e., a major bacterial surface protein having a molecular weight of about 190kDa, and that carry a collagen binding protein CBP (Collagen Binding Protein, also known as Cnm) having a molecular weight of about 120kDa, and also discovered that all these virulent bacterial strains have low cell surface charge. The influences of *S. mutans* on hemorrhage has never been reported so far, and the findings that particular strains of *S. mutans* exacerbate the prognosis of hemorrhage and that PA and CBP as well as cell surface charge are involved in such virulence were therefore surprising results. Based on these findings, the inventors further proceeded with the study, and found that CBP-positive bacterium has an ability to inhibit platelet aggregation, thereby completed the invention.

Accordingly, the present invention relates to a method of detecting a hemorrhage aggravating oral bacterium, the method comprising detecting PA and/or CBP and/or cell surface charge of oral bacteria in a sample, wherein the presence of the hemorrhage aggravating oral bacterium is determined if PA is not detected and/or CBP is detected and/or the cell surface charge is negative.

Moreover, the present invention relates to a method of screening a subject at a high risk of hemorrhage aggravation, the method comprising detecting PA and/or CBP and/or cell surface charge of oral bacteria in a biological sample obtained from the subject, wherein a high risk of hemorrhage aggravation is determined if PA is not detected and/or CBP is detected and/or the cell surface charge is negative.

Alternatively, the present invention relates to a method of judging the risk of hemorrhage aggravation in a subject, the method comprising detecting PA and/or CBP and/or cell surface charge of oral bacteria in a biological sample obtained from the subject, wherein a high risk of hemorrhage aggravation in the subject is determined if PA is not detected and/or CBP is detected and/or the cell surface charge is negative.

Furthermore, the present invention relates to any one of said methods wherein the hemorrhage is hemorrhage by diabrosis.

The present invention also relates to any one of said methods wherein the oral bacterium is *Streptococcus mutans.*

The present invention further relates to any one of said methods wherein PA is selected from the group consisting of

(1) a polypeptide comprising an amino acid sequence according to SEQ ID NO.: 1, 17, 19, 21 or 23;
(2) a polypeptide comprising one or more mutations in the polypeptide of (1) but having an equal function to the polypeptide of (1);
(3) a polypeptide comprising an amino acid sequence encoded by a nucleic acid sequence that hybridizes with a nucleic acid sequence according to SEQ ID NO.: 2, 18, 20, 22 or 24 or its complementary sequence or its fragment under stringent condition, and having an equal function to the polypeptide of (1); and
(4) a polypeptide comprising an amino acid sequence having 70% or more homology with an amino acid sequence according to SEQ ID NO.: 1, 17, 19, 21 or 23.

The present invention further relates to any one of said methods wherein PA comprises a polypeptide consisting of an amino acid sequence according to SEQ ID NO.: 1, 17, 19, 21 or 23.

The present invention also relates to any one of said methods wherein CBP is selected from the group consisting of

(1) a polypeptide comprising an amino acid sequence according to SEQ ID NO.: 5, 9, 27 or 31;
(2) a polypeptide comprising one or more mutations in the polypeptide of (1) but having an equal function to the polypeptide of (1);
(3) a polypeptide comprising an amino acid sequence encoded by a nucleic acid sequence that hybridizes with a nucleic acid sequence according to SEQ ID NO.: 6, 10, 28 or 32 or its complementary sequence or its fragment under stringent condition, and having an equal function to the polypeptide of (1);
(4) a polypeptide comprising an amino acid sequence having 70% or more homology with an amino acid sequence according to SEQ ID NO.: 5, 9, 27 or 31.

The present invention also relates to any one of said methods wherein CBP comprises a polypeptide consisting of an amino acid sequenceaccording to SEQ ID NO.: 5, 9, 27 or 31.

Also, the present invention relates to a reagent for detection of a hemorrhage-aggravating oral bacterium, the reagent comprising an oral bacterial PA-detecting agent and/or CBP-detecting agent.

Furthermore, the present invention relates to an oral bacterial PA-specific antibody for detection of a hemorrhage-aggravating oral bacterium.

Moreover, the present invention relates to a kit for detection of a hemorrhage-aggravating oral bacterium and/or for screening of a subject at a high risk of hemorrhage aggravation and/or for determination of the risk of hemorrhage aggravation in the subject, the kit comprising at least:

a PA-detecting reagent, and
a CBP-detecting reagent.

Also, the present invention relates to a hemostatic agent comprising PA protein of an oral bacterium or nucleic acid encoding the PA protein.

Also, the present invention relates to an inhibitor of platelet aggregation comprising a substance that binds to an oral bacterium PA protein or to a nucleic acid encoding the PA protein.

Also, the present invention relates to a hemorrhage aggravation inhibitor comprising a substance that binds to an oral bacterium CBP or to a nucleic acid encoding the CBP protein.

Alternatively, the present invention relates to an agent for detecting collagen-denuded site in tissue comprising CBP of an oral bacterium.

Also, the present invention relates to a carrier for delivering a substance to the collagen-denuded site comprising CBP of an oral bacterium.

The present invention also relates to a therapeutic agent for hemorrhage comprising CBP of an oral bacterium and a hemostatic agent.

Moreover, the present invention relates to said therapeutic agent for hemorrhage for a subject having low sensitivity of platelet to collagen.

Also, the present invention relates to a prophylactic agent for hemorrhage aggravation comprising an oral bacterium-removing agent.

## THE EFFECTS OF THE INVENTION

The present invention allows rapidly and easily diagnosing the risk of causing hemorrhage aggravation in a subject. Also, the method of the present invention enables detecting responsible factors of hemorrhage aggravation using readily-available biological samples such as saliva and plaque without employing any special analyzers. As such, the present invention allows to specify a high-risk population of hemorrhage aggravation, to treat the individuals belonging to this population with a regimen such as removing virulent bacteria and advising dental hygiene, and thereby to effectively prevent a hemorrhage aggravation.

## BRIEF DESCRIPTION OF THE DRAWINGS

**Figure 1** is a flow-chart of a system to detect a *S. mutans* strain which may cause hemorrhage aggravation.

**Figure 2** is a scheme of methods of culturing and detecting *S. mutans.*

**Figure 3** is a scheme of methods of detecting PA-deleted *S. mutans* and CBP-carrying *S. mutans.*

**Figure 4** is a diagram showing the results of infecting mice with *S. mutans* strains and inducing an aggravation of cerebral hemorrhage. (a-b) Representative macro images of (a) mouse whole blains and (b) coronal slices 24 hours after inducing cerebral hemorrhage and administering either PBS (control) or *S. mutans* TW295 strain. Arrowheads indicate the breeding sites. (c) A graph showing the differences in breeding areas between various *S. mutans* strains used for infection. Each column represents the mean ± SEM from 10 to 21 independent experiments (*P<0.05, **P<0.01). (d) Typical microscopic photographs of the vessel-damaged hemisphere from the control or TW295-administered mouse isolated 3 hours after the induction of hemorrhage. The enlarged view indicates microvascular hemorrhages. (e) Samples were collected 24 hours after administration of the bacteria, and the MMP-9 activity was detected by gelatin gel zymography. The MMP-9 activity was consistently high in the vessel-damaged hemisphere in TW295-administered group compared with those in the control group (Ips: vessel-damaged hemisphere of the brain, Ctr: contralateral hemisphere).

**Figure 5** is a diagram showing the accumulation of administered bacteria to the damaged vessel and their interaction with collagen surrounding the vessel. (a) 24 hours after infecting mice with TW295 strain, cellular DNA of the infected bacteria was detected in each organ by PCR using specific primers. Labels on each lane is: M: molecular marker (100 bp ladder), Std: positive control (genomic DNA extracted from TW295 strain), Ips: vessel-damaged hemisphere of the brain, Ctr: contralateral hemisphere of the brain, respectively. (b) Tissues isolated from damaged and undamaged area are plated onto culture dishes to harvest *S. mutans.* Germ numbers of isolated bacteria are expressed in CFU/mg protein. (c) Representative *in vivo* scanning electron microscopic images showing interaction between an injured blood vessel and infecting bacterial cells observed in a sample prepared from cerebral tissue resected 3 hours after the administration of TW295 strain. The right panel is enlarged image of the boxed part. White arrowheads indicate leaking of accumulated bacteria at the damaged site. (d) An *in vivo* SEM image showing the interaction between the bacteria and collagen fibers surrounding the vessel in damaged hemisphere. White arrowheads indicate the bacteria interacting with collagen fibers.

**Figure 6** is a diagram showing the detection of Gram staining of bacteria accumulating in the damaged brain area. White arrowheads indicate accumulated bacteria. Scale bar: 25 μm.

**Figure 7** is a diagram summarizing the effects of the presence of collagen binding protein (CBP) and the expression of protein antigen (PA) on the virulence.

**Figure 8** is a diagram showing the impacts of CBP-expressing *S. mutans* on collagen binding activity and cerebral hemorrhage. (a) Collagen binding activities of various *S. mutans* strains were assessed under certain condition using 2 mg Type I collagen and 1 x $10^{10}$ bacterial cells. The result from each strain was expressed as a percentage to that from TW871 strain. (b) The areas of hemorrhage in mice infected either with TW295, a TW295CND strain generated from TW295, or a MT8141PD strain generated from MT8141 or MT8141. Each column represents the mean ± SEM from 11 to 16 independent experiments. (c) Correlation between collagen binding activity and total hemorrhage area.

**Figure 9** is a diagram showing the relationship between collagen binding activity and platelet aggregation in various *S. mutans* strains. (a) Platelet aggregation activity of various *S. mutans* strains. The assay was performed using mouse whole blood and an aggregometer under certain conditions using 4 μg collagen and 1 x $10^7$ bacterial cells. The result from each strain was expressed as a percentage to that of the case wherein collagen was included but no bacterial cells were included. (b) Correlation between collagen binding activity and platelet aggregation.

**Figure 10** is a diagram showing the effects of various *S. mutans* strain on platelet aggregation. (a) Platelet aggregation rates after the addition of either standard strain MT8148 or virulent strain TW871 in various cell numbers. Aggregation in mouse whole blood was measured after the addition of *S. mutans* and expressed as a percentage. (b-c) Platelet aggregation rates for 58 clinical strains were assessed by the same method as (a). Results are expressed (b) by their serotypes (c; n=20, e; n=15, f; n=10, and k; n=13), or (c) by the sources of the isolated strains (oral cavity; n=45, blood; n=13). White and black circles indicate the strain isolated from oral cavity and blood, respectively. Bold horizontal bars indicate the mean value of each group (*p<0.05, **p<0.01, ***p<0.001). (d) A typical chart of the platelet aggregation assay using human platelet-rich plasma. MT8141 or TW871 bacterial cells ($10^7$ CFU) and human platelet-rich plasma were incubated, then collagen (4 μg) was added after 5 minutes. (e) The effect of bacteria on arachidonic acid-induced platelet aggregation. In whole blood aggregation, collagen was substituted by arachidonic acid as aggregating reagent. Each column represents the mean ± SEM of 8 to 14 independent experiments.

**Figure 11** is a diagram showing the relationship between the difference in bacterial cell surface conditions and collagen-induced platelet aggregation. (a) Representative observation of scanning electron microscopy of the reaction of *S. mutans* strain and platelets to collagen. Platelet fraction was collected after reacting with collagen, and observed with scanning electron microscope. (b) Zeta potential values of *S. mutans* strain. Zeta potential values of the standard strain MT8148 and its isogenic mutant strain MT8148PD were measured and expressed in mV. Moreover, 7 oral cavity-isolated strains and 7 blood isolated strains were subjected to the measurement. Each column represents the data from 3 to 5 independent experiments. (c) Transmission electron microscopy observation of bacterial surface. (d) Scanning electron microscopy observation of bacterial surface.

**Figure 12** is a diagram showing correlation of platelet aggregation and zeta potential value. Each point represents one bacterial strain.

**Figure 13** is a diagram showing three-dimensional reconstructed images of the bacterial surface. Bacterial membranes were compared using three-dimensional reconstructed images generated by TEM computerized tomography. (a) MT8148 peptidoglycan layer is observed as a transparent and very smooth layer in the three-dimensional image obtained by TEM. (b) The bacterial surface peptidoglycan of the virulent strain TW295 is opaque and its outer shape is obscure (arrowheads).

**Figure 14** is a diagram showing the effects of the bacteria isolated from human stroke patients on collagen binding activity and platelet aggregation. (a) Collagen binding activity of *S. mutans* strains isolated from stroke patients (SMH2 and SMH4). The activity was assessed under certain conditions using 2mg Type I collagen and 1 x $10^{10}$ bacterial cells. The result for each strain is expressed as a percentage to that for TW871. (b) Platelet aggregation activity of *S. mutans* strains isolated from stroke patients. Assay was performed by impedance method on aggregometer using mouse whole blood under certain condition using 4 μg Type I collagen and 1 x $10^7$ bacterial cells. The result for each strain is expressed as a percentage to that for the case where collagen was added but no bacterial cells are added.

**Figure 15** is a diagram showing the effects of CBP-expressing *S. mutans* isolated from stroke patients on cerebral hemorrhage. (a) A representative macro image of whole brain of a mouse to which SMH4 isolated from a stroke patient was administered, 24 hours after the onset of cerebral hemorrhage. (b) Representative macro images of brain sections of a mouse to which SMH4 was administered. (c) Sizes of cerebral hemorrhage regions in groups of mice infected with CBP-expressing *S. mutans* isolated from stroke patients (SMH2 and SMH4). Each column represents the mean ± SEM of 11 to 16 independent experiments (*p<0.05).

**Figure 16** is a schematic diagram showing a putative mechanism of aggravation of cerebral hemorrhage by *S. mutans* cells. (a) Normal hemostasis induced by platelet aggregation at damaged site of an endothelial cell. (b) *S. mutans* cells with a high negative charge accumulate onto denuded collagen with a positive charge. Moreover, *S. mutans* cells carrying collagen binding protein have a high affinity to denuded collagen. Both these factors result in an activation of MMP-9 and inhibition of platelet aggregation at the damaged site of the endothelial cell, thereby causing a sustained bleeding.

**Figure 17** is a schematic diagram of the experimental protocols to photochemically induce damage onto mouse mesencephalic artery endothelial cells. (a) A schematic diagram of the hypothesis and the experimental protocols. (b) Time schedule of the experimental procedures.

**Figure 18** is a diagram showing examples of the results of the analysis to determine the presences of PA-deleted *S. mutans* and CBP-carrying *S. mutans.*

**Figure 19** is a graph showing the results of the investigation on optimum conditions for culturing *S. mutans* (culture in an aerobic/anaerobic condition, concentration of antibiotics, nutrient concentration).

**Figure 20** is a graph showing the results of the investigation on the possible stock period for saliva to be used for the detection of virulent *S. mutans.*

## DESCRIPTION OF EMBODIMENTS

The present invention provides a method of detecting a hemorrhage-aggravating oral bacterium, the method comprising detecting PA and/or CBP and/or cell surface charge of oral bacteria in a sample, wherein the presence of the hemorrhage-aggravating oral bacterium is determined by that PA is not detected and/or that CBP is detected and/or that the cell surface charge is negative.

The present invention provides, in another embodiment, a method of screening a subject at a high risk of hemorrhage aggravation, the method comprising detecting PA and/or CBP and/or cell surface charge of oral bacteria in a biological sample obtained from a subject, wherein a high risk of hemorrhage aggravation is determined by that PA is not detected and/or that CBP is detected and/or that the cell surface charge is negative.

The present invention further provide, in another embodiment, a method of determining the risk of hemorrhage aggravation in a subject, the method comprising detecting PA and/or CBP and/or cell surface charge of oral bacteria in a biological sample obtained from a subject, wherein a high risk of hemorrhage aggravation is determined in the subject by that PA is not detected and/or that CBP is detected and/or that the cell surface charge is negative.

A mutans streptococci *Streptococcus mutans,* an oral bacterium that is a major pathogenic bacteria of dental caries, are known to have four serotypes (c, e, f and k). *S. mutans* is also known to be a pathogenic bacterium of bacteremia and infective endocarditis, and reported to be relevant to cardiovascular diseases since bacterial DNA of *S. mutans* was detected from the specimens of cardiac valve and aortic aneurysm (Nakano et al., 2008, Japanese Dental Science Review, 44: 29-37). However, association of *S. mutans* to other diseases, for example its impact on cerebrovascular diseases, have never been investigated so far.

Studies by the inventors disclosed herein revealed that the intravenous administration of some of different *S. mutans* strains inhibits spontaneous hemostatic action and induces aggravation of hemorrhage, when mild cerebral hemorrhage has been induced by damaging the middle cerebral artery A MT8148 strain generally isolated from the oral cavity (serotype c) (Minami et al., 1990, Oral Microbiol. Immunol., 5: 189-194) does not cause such effects, thought there are strains among serotype *k* that evokes hemorrhage aggravation. In particular, TW295 strain and TW871 strain (Nakano et al., 2004, Journal of Clinical Microbiology, 42(1): 198-202), SA53 strain (Nakano et al., 2007, J. Clin. Microbiol., 45: 2614-2625), and LJ32 strain (Nakano, K. et al., 2008, J.Dent. Res. 87: 964-968) cause a significant hemorrhage aggravation.

The inventors found that those highly virulent *S. mutans* strains lack PA, a major bacterial surface protein. The inventors also found that among the PA-deficient strains, the virulence of the strains carrying CBP, another bacterial surface protein, was particularly high. The inventors further confirmed that TW295 strain-like hemorrhage aggravation is not exhibited when CBP-encoding gene of TW295 strain has been deleted by genetic engineering; and that a strain in which PA-encoding gene has been deleted from MT8148 strain exhibits hemorrhage aggravation, confirming that CBP and PA are involved in hemorrhage aggravating activity of *S. mutans.* The inventors further found that CBP-carrying *S. mutans* strains are detected in the oral cavity of human patients with hemorrhagic stroke, and further confirmed that CBP-carrying *S. mutans* strains isolated from such patients cause aggravation of cerebral hemorrhage *in vivo.* The inventors further found that the cell surface charge of a highly virulent *S. mutans* strain is negative. Based on these findings, the inventors demonstrated that these bacterial surface protein and cell surface charge can be utilized as useful markers for detection of a *S. mutans* strain that exacerbates hemorrhage, for screening of a subject at a high risk of hemorrhage aggravation, and for determination of the risk of hemorrhage aggravation of a subject.

The oral bacterium detected according to the method of the present invention may exacerbate any bleeding, though, in particular, would exacerbate a hemorrhage by diabrosis caused by the occurrence of damage on the vascular wall due to a traumatic injury, an ulcer or a ruptured aneurysm. Representative examples of hemorrhage by diabrosis include such as cerebral hemorrhage (intracerebral hemorrhage, subarachnoid hemorrhage, chronic subdural hematoma), bleeding due to traumatic injury or compression, hemorrhage after delivery, subcutaneous hemorrhage associated with diseases. Also, diseases which cause bleeding tendency include connective tissue disorders (such as allergic purpura), thrombocytopenia (such as disseminated intravascular coagulation and aplastic anemia) or platelet disorders (such as thrombasthenia), or disorders in coagulation system (such as coagulation disorders associated with liver diseases and vitamin K deficiency). Endogenous or exogenous circulating anti-coagulation substances (such as lupus anticoagulant and VIII factor anti-coagulation substance) may also cause bleeding tendency.

Hemorrhage aggravation herein means that the spontaneous hemostatic action against bleeding caused by such endogenous or exogenous factor is delayed, decreased or lost as compared to a normal subject. Also, a subject at a high risk of hemorrhage aggravation means that, in said subject, the spontaneous hemostatic action by platelets is highly likely to be delayed, decreased or lost as compared to a normal subject upon the bleeding due to an endogenous or exogenous factor.

PA (Protein Antigen) is a surface protein of approximately 190kDa found in MT8148 strain, a *S. mutans* wild-type strain, and also known in various other names such as PAc (Protein Antigen c), SpaP, Antigen I/II and Antigen B, P1 and MSL-1. PA polypeptide comprises 3 alanine-rich repeat domains (A-region) at N-terminal side and 3 proline-rich repeat domains (P-region) at central part, and has cell wall/membrane-spanning domain at C-terminal. It has been reported that the A-regions are involved in the attachment of bacterial cells to teeth (Matsumoto-Nakano et al., 2008, Oral Microbiology and Immunology, 23:265-270). Also, there have been reports that PA is involved in infective endocarditis by *S. mutans* (Nakano et al., 2008, Japanese Dental Science Review, 44: 29-37); that an antibody against PA inhibits the attachment of bacterial cells to a hydroxyapatite substrate (Kawato et al., 2008, Oral Microbiology and Immunology, 23:14-20); and that an antiserum against PA is useful as a vaccine for dental caries (Okahashi et al., 1989, Molecular Microbiology, 3(2): 221-228). Although there is a region between A-region and P-region of PA, in which amino acid sequences are highly variable between strains (for example, in MT8148 strain, residues from 679 to 827), the repeat domain and transmembrane domain are highly conserved among strains.

Also, it is reported that strains of serotype *k*, which are often detected in patients with infective endocarditis, lacks PA in a high percentage, and that both the hydrophobicity of the bacterial body sensitivity to phagocytosis are low in this serotype (Nakano et al., 2008, Journal of Dental Research, 87(10): 964-968).

Known PA includes, for example, PA of serotype c MT8148 (DDBJ Accession No.: X14490, amino acids: SEQ ID NO.: 1, nucleic acids: SEQ ID NO.: 2), PA of LJ23 strain (DDBJ Accession No.: AB364261, amino acids: SEQ ID NO.: 17, nucleic acids: SEQ ID NO.: 18), PA of SA98 strain (DDBJ Accession No.: AB364285, amino acids: SEQ ID NO.: 19, nucleic acids: SEQ ID NO.: 20), as well as *spaP* gene of antigen I/II (DDBJ Accession No.: X17390, Kelly et al., 1989, FEBS Lett. 258(1), 127-132, amino acids: SEQ ID NO.: 21, nucleic acids: SEQ ID NO.: 22) and a meningococcus *Neisseria meningitidis* iron binding protein *fbp* gene (X53469, Berish et al., 1990, Nucleic Acid Research, 18(15): 4596-4596, amino acids: SEQ ID NO.: 23, nucleic acids: SEQ ID NO.: 24).

CBP, i.e., another anchor protein of *S. mutans* (also denoted as Cnm), is a Type I collagen binding protein of approximately 120kDa molecular weight, and has a collagen binding domain (CBD, residues from 152 to 316), B repeat domain (residues from 328 to 455) and LPXTG motif (residues from 507 to 511) (Sato et al., 2004, Journal of Dental Research, 83(7): 534-539). CBP gene-carrying frequency of *S. mutans* is about 10 to 20%, and CBP-positive strain is predominantly expressed in serotype f and k (Nakano et al., 2007, J. Clin. Microbiol., 45: 2616-2625).

The studies by the inventors revealed that, for CBP of serotype *k* TW295 strain (DDBJ Accession No.: AB102689, amino acids: SEQ ID NO.: 3, nucleic acids: SEQ ID NO.: 4), CBD (amino acids: SEQ ID NO.: 5, nucleic acids: SEQ ID NO.: 6) and LPXTG motif are highly conserved between strains, whereas the number of repeats in the B repeat domain varies between strains (Nomura et. al., 2009, J. Med. Microbiol., 58:469-75).

In one embodiment of the present invention, PA is defined as:

(1) a polypeptide comprising an amino acid sequence expressed by SEQ ID NOs.: 1, 17, 19, 21 or 23;
(2) a polypeptide comprising one or more, preferably 1 to 20, 1 to 15, 1 to 10, or one or several mutations in polypeptide of (1), but having an equal function as the polypeptide of (1);
(3) a polypeptide comprising an amino acid sequence encoded by a nucleic acid sequence that hybridizes under stringent condition with a nucleic acid sequence expressed by SEQ ID NOs.: 2, 18, 20, 22 or 24 or its complementary sequence or its fragment, and having an equal function as the polypeptide of (1); or
(4) a polypeptide comprising an amino acid sequence having 70% or more, preferably 80% or more, more preferably 90% or more, still more preferably 95% or more homology to an amino acid sequence expressed by SEQ ID NOs.: 1, 17, 19, 21 or 23, and having an equal function as the polypeptide of (1).

Preferably, PA comprises a polypeptide consisting of an amino acid sequence expressed by SEQ ID NOs.: 1, 17, 19, 21 or 23. More preferably, PA comprises a polypeptide consisting of an amino acid sequence expressed by SEQ ID NO.: 1.

PA that can be used in the method of the present invention may be a polypeptide comprising one or more amino acid mutations (deletions, substitutions and/or additions), as long as it comprises an amino acid sequence encoded by a nucleic acid sequence that hybridizes under stringent condition with a nucleic acid sequence expressed by SEQ ID NOs.: 2, 18, 20, 22 or 24 (nucleic acid encoding the PA protein sequence) or its complementary sequence or its fragment, and has a equal function as a polypeptide comprising an amino acid sequence expressed by SEQ ID NOs.: 1, 17, 19, 21 or 23 (amino acid sequence of PA protein). Mutations may be naturally occurring mutations or mutations generated by any known procedures, e.g., cleavage or insertion of a nucleic acid by restriction enzyme, site-specific mutagenesis, or radiation or ultraviolet irradiation. Moreover, the number of mutated amino acids may be 1 to 20, 1 to 15, 1 to 10, or 1 to several, for example.

Furthermore, in one embodiment of the present invention, CBP is defined as:

(1) a polypeptide comprising an amino acid sequence expressed by SEQ ID NOs.: 5, 9, 27 or 31;
(2) a polypeptide comprising one or more, preferably 1 to 20, 1 to 15, 1 to 10, or one or several mutations in the polypeptide of (1), but having an equal function as the polypeptide of (1);
(3) a polypeptide comprising an amino acid sequence encoded by a nucleic acid sequence that hybridizes under stringent condition with a nucleic acid sequence expressed by SEQ ID NOs.: 6, 10, 28 or 32 or its complementary sequence or its fragment, and an equal function as the polypeptide of (1); or
(4) a polypeptide comprising an amino acid sequence having 70% or more, preferably 80% or more, more preferably 90% or more, still more preferably 95% or more homology with an amino acid sequence expressed by SEQ ID NOs.: 5, 9, 27 or 31, and having an equal function as the polypeptide of (1).

CBP polypeptide that can be used in the method of the present invention may be a polypeptide comprising one or more, e.g., 1 to 20, 1 to 15, 1 to 10, or one or several amino acid mutations (deletions, substitutions and/or additions), as long as it comprises an amino acid sequence encoded by a nucleic acid sequence that hybridizes under stringent condition with a nucleic acid sequence expressed by SEQ ID NOs.: 6, 10, 28 or 32 (nucleic acid sequence encoding CBD of *S. mutans* TW295, TW871, SA53 or LJ32 strains) or its complementary sequence or its fragment, and has an equal function as a polypeptide comprising an amino acid sequence expressed by SEQ ID NOs.: 5, 9, 27 or 31 (CBD amino acid sequence of *S. mutans* TW295, TW871, SA53 or LJ32 strain).

For instance, CBP polypeptide may be a polypeptide comprising an amino acid sequence encoded by a nucleic acid sequence that hybridizes under stringent condition with a nucleic acid sequence expressed by SEQ ID NOs.: 4, 8, 26 or 30 (a nucleic acid sequence encoding CBP of *S. mutans* TW295 strain, TW871 strain (DDBJ Accession No.AB469914), SA53 strain (AB465299) or LJ32 strain (AB465263)) or its complementary sequence or its fragment, and has an equal function as a polypeptide comprising an amino acid sequence expressed by SEQ ID NOs.: 3, 7, 25 or 29 (an amino acid sequence of CBP protein of *S. mutans* TW295, TW871, SA53 or LJ32 strain).

Preferably, CBP comprises a polypeptide consisting of an amino acid sequence expressed by SEQ ID NOs.: 5, 9, 27 or 31.

Whether a PA or CBP mutant has an equal function as PA or CBP or not may be confirmed using any known means. For instance, the ability of PA mutant making the bacterial cell adhere to a hydroxyapatite substrate may be determined by raising a specific antibody against the mutant peptide by a known method, and assaying the inhibition of adhesion of bacteria to the hydroxyapatite by said antibody according to a method described in Kawato et al., 2008, Oral Microbiology and Immunology, 23:14-20. Alternatively, the biding ability of a CBP mutant to Type I collagen may be determined by collagen binding assay described in Nomura et al., 2009, J. Med. Microbiol., 58(4): 469-475. By such means, the ability of a mutant can be assessed in comparison with an appropriate negative control, or with PA or CBP as a positive control. For instance, certain mutant is considered as a functional mutant when at least one function described above is better, e.g., 10% or better, 25% or better, 50% or better, 75% or better, or even 100% or better, than the negative control, and/or when said function is 1/100 or less, 1/50 or less, 1/25 or less, 1/10 or less, 1/5 or less, or even 1/2 or less, than the positive control.

In the method of the present invention, the surface charge of a bacterial cell can be measured by any known method, e.g., zeta potential measuring method. Zeta potential, also called as electrokinetic potential, is a potential difference that arises on the interface between a solid and a liquid contacting to each other in a relative motion, which may be used as an index for the surface charge of a bacterial cell. Zeta potential can be calculated from electrophoretic mobility of bacterial cells using an equation of Smoluchowski:

$$\zeta=\eta u/\varepsilon 0 \varepsilon r$$

wherein, $\zeta$ indicates the zeta potential, $\eta$ indicates the viscosity of the solvent, u indicates the electrophoretic mobility, $\varepsilon 0$ indicates the dielectric constant of a vacuum, er indicates the dielectric constant of the solvent.

Methods of electrophoresis suitable for measuring zeta potential are not particularly limited as long as it can measure the migrating speed of bacterial cells, and include, for example, capillary electrophoresis, microscopic electrophoresis, rotating diffraction gating method and laser Doppler electrophoresis.

In the method of the present invention, a negative surface charge of the bacterial cell is an index for a highly virulent oral bacterium, and is a criterion for the presence of a hemorrhage-aggravating oral bacterium and a risk of hemorrhage aggravation. Namely, collagen fibers denuded within a damaged vessel are positively charged, and if bacterial cell surface is negatively charged, the bacterial cell may easily interact with denuded collagen fibers, thereby resulting in hemorrhage aggravation due to the inhibition of platelet aggregation. Typically, an oral bacterium is determined to be highly virulent when the surface charge measured as zeta potential is -1.0mV or below, more preferably -3.0mV or below, still more preferably -4.0mV or below, even more preferably -5.0mV or below, particularly preferably -8.0mV or below.

In aforementioned methods of the present invention, oral bacterial PA, CBP and cell surface charge may be used either alone or in combination. Accordingly, either PA alone, CBP alone, or cell surface charge alone may be detected, or any combination of PA, CBP and cell surface charge, namely, both PA and CBP, both PA and cell surface charge, both CBP and cell surface charge, or, all of PA, CBP and cell surface charge may be detected. Furthermore, each of the criteria, i.e., that PA is not detected, that CBP is detected and that the cell surface charge is negative, may be used alone or in combination, according to the items to be detected.

Major bacteria species that are identified as hemorrhage-aggravating oral bacteria include mutans streptococci such as *Streptococcus mutans, Streptococcus sobrinus, Streptococcus cricetus, Streptococcus rattus, Streptococcus downei*; *Streptococcus sanguinis, Streptococcus oralis, Streptococcus gordonii,* and *Streptococcus salivarius.* Particularly, *S. mutans* TW295 strain, TW871 strain, SA53 strain, and LJ32 strain would cause severe hemorrhage aggravation.

Screening of other bacteria that could induce hemorrhage aggravation can be carried out utilizing databases such as NCBI GenBank®, DDBJ (DNA Data Bank of Japan, http://www.ddbj.nig.ac.jp/) and EMBL, and publicly available search tools such as BLAST.

The present invention provides, in one embodiment, a reagent for the detection of a hemorrhage-aggravating oral bacterium comprising an oral bacterial PA detecting agent and/or an oral bacterial CBP detecting agent.

In one embodiment, the PA detecting agent comprises an oral bacterial PA-specific antibody. Using the PA-specific antibody developed by the inventors, the presence or absence of a highly virulent *S. mutans* can rapidly and easily detected. The PA-specific antibody is preferably an antibody or its fragment induced from polypeptide comprising an amino acid sequence of SEQ ID NO.: 1 or its immunogenic fragment. Alternatively, the PA-specific antibody may be an antibody or its fragment induced from a polypeptide having 70% or more, preferably 80% or more, more preferably 90% or more, still more preferably 95% or more homology with an amino acid sequence of SEQ ID NOs.: 1, 17, 19, 21 or 23, and having an immunogenicity to induce an antibody production against a polypeptide comprising an amino acid sequence of SEQ ID NOs.: 1, 17, 19, 21 or 23. For example, a recombinant PA comprising the polypeptide (see, e.g., Nakano et al., 2006, Microbes and Infection, 8:114-121) may be used as an antigen to produce a monoclonal or polyclonal antibody.

In one embodiment, CBP detecting agent comprises a substrate (such as a microplate, test tube or slide glass) coated with Type I collagen. The binding affinity of CBP to Type I collagen (Nomura et al., 2009, J. Med. Microbiol., 58(4): 469-475) can be utilized to allow CBP-expressing bacterial cell to attach a substrate coated with Type I collagen, which can easily be detected.

In another embodiment, the CBP detecting agent comprises a specific antibody against an oral bacterial CBP The CBP-specific antibody may be a specific antibody against the collagen binding domain of CBP, preferably, an antibody or its fragment induced from a polypeptide comprising an amino acid sequence of SEQ ID NOs.: 5, 9, 27 or 31 or its immunogenic fragment. Alternatively, the CBP-specific antibody may be an antibody or its fragment induced from a polypeptide having 70% or more, preferably 80% or more, more preferably 90% or more, still more preferably 95% or more homology with an amino acid sequence of SEQ ID NOs.: 5, 9, 27 or 31, and having an immunogenicity to induce an antibody production against a polypeptide comprising an amino acid sequence of SEQ ID NOs.: 5, 9, 27 or 31.

In the present invention, the antibody fragment comprises, for example, various functional fragments such as Fab, Fab', F(ab')2, scFv, dsFv (disulfide-stabilized V region fragment), and CDR-containing fragment.

The present invention provides, in one embodiment, a kit for the detection of a hemorrhage-aggravating oral bacterium, and/or for the screening of a subject at a high risk of hemorrhage aggravation, and/or for the determination of the risk of hemorrhage aggravation in a subject. The kit comprises at least a PA-detecting reagent and a CBP-detecting reagent.

In one embodiment, the kit comprises as a PA-detecting reagent an oral bacterial PA-specific antibody.

In one embodiment, the kit comprises as a CBP-detecting reagent a substrate coated with Type I collagen (such as a microplate, test tube or slide glass).

In another embodiment, the kit comprises as a CBP-detecting reagent a CBP-specific antibody.

The kit of the present invention may further comprise one or more of the followings for culturing *S. mutans*:

- An instrument for collecting saliva such as a spitz for collecting saliva (the material and shape is not particularly limited as long as it is sterilized and suitable for collecting and seeding).
- A collecting instrument such as a dropper capable of collecting saliva of approximately 10 μl.
- *S. mutans* selection medium (Special Medium A). For example, sterile substrate coated with MSB agar medium (Mitis-salivariusagar medium (e.g., Difco Laboratories) supplemented with an antibiotic (e.g., bacitracin (e.g., SIGMA-ALDRICH)) and sucrose (e.g., Wako Pure Chemical Industries, Ltd.)). The substrate is not particularly limited as long as it is such as a dish or well plate, though typically a plate of about 24-well (e.g., 24 well with Lid MICROPLATE (IWAKI)) is used. Bacitracin is preferably used at about 100 unit/ml. Sucrose is preferably used at about 15%.
- A sealing and/or deoxygenating instrument for culturing under an anaerobic condition such as Anaero Pack® or a $CO_2$ chamber.
- A sterile stick for picking up bacterial colonies (such as a toothpick or tip).
- A liquid medium for culturing the picked-up colonies (Special Medium B). For example, sterilized Brain Heart Infusion (BHI) liquid medium (Difco Laboratories) contained in a disposable test tube.

The kit of the present invention may further contain one or more of the followings for detecting *S. mutans*:

- A collecting instrument suitable for collecting bacterial solution of approximately 10 μl such as a dropper.
- A special medium for detecting *S. mutans* (Special Medium C). For example, sucrose (Wako Pure Chemical Industries, Ltd.)-containing BHI solution 100 μl added to a substrate. The substrate is not particularly limited as long as it is such as a well plate or test tube, though typically a 96-well plate (e.g., MULTI WELL PLATE for ELISA (SUMIRON)) is used. Sucrose is used at about 1%.
- A wash buffer (Wash Buffer A: PBS solution or sterile water may be used, though preferably PBS solution is used.)
- A Gram-positive bacteria detecting reagent (Buffer 1: for example, a solution in which to sterile distilled water about 0.5% crystal violet (e.g., Wako Pure Chemical Industries, Ltd.) is added as the Gram-positive bacteria detecting reagent.)
- A mordanting reagent (Buffer 2: a suitable mordanting reagent may be selected depending on the bacteria detecting reagent. For example, 7% acetate (e.g., Wako Pure Chemical Industries, Ltd.) solution or sterile water may be used for crystal violet, though preferably acetate solution is used.)

The kit of the present invention may further comprise one or more of the followings for detecting PA-deleted *S. mutans*:

- A plate for detecting PA-deleted *S. mutans.* It is not particularly limited as long as sterile it is a well plate, though typically a 96-well plate (e.g., MICROTEST U-Bottom (BECTON DICKINSON)) is used.
- A wash buffer (Wash Buffer B: a solution in which to PBS solution or sterile water about 0.05% of a surfactant such as Triton X-100 (e.g., Wako Pure Chemical Industries, Ltd.) are added. Preferably PBS solution is used.)
- A buffer (Buffer 3: a mixture of Tris buffered saline (pH6.8), 100 mM dithiothreitol (e.g., Wako Pure Chemical Industries, Ltd.) and 20% glycerin (e.g., Wako Pure Chemical Industries, Ltd.).)
- A blocking solution (Buffer 4: a PBST solution containing approximately 5% of skimmed milk (e.g., BECTON DICKINSON).)

- A primary antibody (Buffer 5: a PBST solution containing approximately 0.1% of anti-PA antiserum.)
- A secondary antibody (Buffer 6: a PBST solution containing approximately 0.1% of a primary antibody against the immunoglobulin (e.g., Dakopatts).)
- A color-developing reagent (Buffer 7: AP (100mM 2-amino-2-hydroxymethyl-1,3-propanediol, 5mM magnesium chloride, 100mM sodium chloride) buffer supplemented with NBT solution (Wako Pure Chemical Industries, Ltd.) at final concentration of 0.6% and BCIP solution (Wako Pure Chemical Industries, Ltd.) at final concentration of 0.33%.)

The kit of the present invention may further comprise one or more of the followings for detecting CBP-carrying *S. mutans*:

- A special medium for detecting CBP-carrying *S. mutans* (Special Medium D : a mixed solution of 0.6% acetate-containing sterile distilled water and Type I collagen (Sigma) in 9: 1 ratio contained in the Special Plate used in Analysis 3.)
- A wash buffer (Wash Buffer A: PBS solution or sterile water may be used, though preferably PBS solution is used.)
- A buffer (Buffer 8: Wash Buffer A containing approximately 5% bovine albumin (Sigma).)
- A wash buffer (Wash Buffer C: PBS solution or sterile water containing a surfactant such as approximately 0.01% Tween 20 (Wako Pure Chemical Industries, Ltd.). Preferably, PBS solution is used.)
- A fixative solution (Buffer 9: for example, sterile distilled water containing approximately 25% formaldehyde (Wako Pure Chemical Industries, Ltd.).)
- A Gram-positive bacteria detecting reagent (e.g., above Buffer 1: a solution in which to sterile distilled water approximately 0.5% crystal violet (Wako Pure Chemical Industries, Ltd.) is added as a Gram-positive bacteria detecting reagent.)
- A mordanting reagent (e.g., above Buffer 2: 7% acetate (e.g., Wako Pure Chemical Industries, Ltd.) solution or sterile water may be used, though preferably acetate solution is used.)

A skilled person in the art may appropriately adjust the concentration of above-mentioned component, e.g., antiserum, secondary antibody, formaldehyde or crystal violet, to be optimum depending on the experimental condition.

The method of the present invention for the detection of a hemorrhage-aggravating oral bacterium is carried out, specifically, for example in a scheme comprising following four steps as shown in Figs. 1 to 3:

Analysis 1. Culturing of *S. mutans*
Analysis 2. Detection of *S. mutans*
Analysis 3. Detection of PA-deleted *S. mutans*
Analysis 4. Detection of CBP-carrying *S. mutans*

In Analysis 1, culturing of bacteria is carried out by following procedures using for example instruments and reagents in the aforementioned kit for culturing mutans streptococci.

The saliva of the subject is collected in a small amount using a spitz for collecting saliva. 10 μl of the saliva is taken from the spitz using a dropper, plated onto a *S. mutans* selection agar medium (e.g., above-mentioned Special Medium A), and cultured at 37°C for 48 hours, preferably under an anaerobic condition. After culturing, the presence of bacterial colonies are grossly confirmed, colonies are picked up and added to a liquid medium (e.g., above-mentioned Special Medium B) and cultured for 37°C for 18 hours, then used for the following Analysis 2, 3 and 4. Preferably, rough colonies are picked up, since *S. mutans* forms rough colonies, whereas *S. sobrinus* forms smooth colonies.

In Analysis 2, detection of *S. mutans* is carried out by following procedures using for example instruments and reagents in the aforementioned kit for detecting *S. mutans.*

10 μl of the bacterial solution cultured from the method of Analysis 1 is added to a medium (e.g., above-mentioned Special Medium C), incubated at 37°C for 3 hours. The medium is washed with a wash buffer (e.g., above Wash Buffer A) for three times, then left still about 15 minutes with the last wash buffer. The wash buffer is removed, and again the medium is washed with Wash Buffer A for once, then a buffer containing a Gram-positive bacteria staining reagent (e.g., above Buffer 1) is added and left still for 1 minute. It is washed with the wash buffer for three times, and a buffer containing a mordanting agent (e.g., above Buffer 2) is added. If the color of the medium was changed, it is determined to be *S. mutans*-positive, if the color of the medium is unchanged, it is determined to be *S. mutans*-negative. A reagent in which a staining reagent and a mordanting agent are already combined may also be used.

In Analysis 3, detection of PA-deleted *S. mutans* is carried out by following procedures using for example instruments and reagents in the aforementioned kit for detecting PA-deleted *S. mutans.*

(1) Sample preparation

To the bacterial solution cultured by the method of Analysis 1 above a suitable buffer (e.g., above-mentioned Buffer 3) is added, which is then immersed in boiling water for 10 minutes, and frozen if it is to be stored.

(2) Detection of PA-deleted *S. mutans*

1) The sample produced from (1) above is added to a plate, left still overnight at 4°C.
2) The plate is washed three times with a wash buffer (e.g., above Wash Buffer B), then skimmed milk (e.g., above Buffer 4) is added thereto, and left still at room temperature for 1 hour.
3) The plate is washed three times with the wash buffer, then a primary antibody (e.g., above Buffer 5) is added, reacted at room temperature for 1 hour.
4) The plate is washed three times with the wash buffer, then a labeled secondary antibody (e.g., above Buffer 6) is added, reacted at room temperature for 1 hour.
5) The plate is washed three times with the wash buffer, then a color-developing reagent (e.g., above Buffer 7) is added, and after appropriate time period, changes in the color of the solution are observed. When the color of the solution is changed, it is determined to be PA-positive, when the color of the solution is not changed, it is determined to be PA-negative.

In Analysis 4, detection of CBP-carrying *S. mutans* is carried out by following procedures using for example instruments and reagents in the aforementioned kit for detecting CBP-carrying *S. mutans.*

(1) The medium (e.g., above Special Medium D) is washed three times with a wash buffer (e.g., above Wash Buffer A), then albumin-containing buffer (e.g., above Buffer 8) is added, and left still at 37°C for 1 hour.
(2) After washing three times with a wash buffer containing a surfactant (e.g., above Wash Buffer C), bacterial solution cultured by the method of Analysis 1 above is added, and incubated at 37°C for 2 hours.
(3) After washing three times with the wash buffer (e.g., above Wash Buffer A), the fixative solution (e.g., above Buffer 9) is added and left still at room temperature for 30 minutes.
(4) After washing three times with the wash buffer, the Gram-positive bacteria staining reagent (e.g., above Buffer 1) is added and left still for 1 minute.
(5) After washing three times with Wash Buffer A, the mordanting agent (e.g., above Buffer 2) is added.

It is determined to be CBP-positive then the color of the solution is changed, and it is determined to be CBP-negative when the color of the solution is not changed.

In any of the detecting methods described above, the detection is possible if bacterial concentration is 1 CFU or more.

Moreover, a culture of e.g., *S. sobrinus, S. sanguinis, S. oralis, S. gordonii,* and *S. salivarius* may be used as a control to confirm in Analysis 1 that any bacterium other than *S. mutans* and *S. sobrinus* grows; in Analysis 3 that any bacterium other than PA-carrying *S. mutans* shows a positive reaction; and in Analysis 4 that any bacterium other than CBP-carrying *S. mutans* shows a positive reaction, respectively.

A skilled person in the art may appropriately modify the method of the present invention according to its object. For example, for detecting PA-deleted *S. mutans,* a substrate to which a specific antibody for PA or CBP is attached may be contacted with a bacterial solution, washed to remove the bacteria which are not attached to the substrate, then only the bacterial cells that are attached to the substrate can be detected by the Gram-positive bacteria staining reagent. Alternatively, primers or probes for a PA or CBP-coding nucleic acid may be used to detect whether the cultured bacterium has the gene of PA or CBP.

In preferred embodiment of the present invention, *S. mutans* MT8148 strain may be used as a positive control for detection of a PA-deleted oral bacterium, and/or as a negative control for detection of a CBP-carrying oral bacterium. As a positive control for detection of a PA-deleted oral bacterium, depending on the detection method, an isolated PA protein, a nucleic acid or vector comprising a DNA encoding PA or its fragment, a cell transformed with said vector may also be used. As a negative control for detection of a CBP-carrying oral bacterium, CND strain, which is a TW295 strain in which CBP-encoding gene has been knocked out, and a Gram-positive bacterium that does not express CBP may also be used.

The present invention provides, in one embodiment, a hemostatic agent comprising an oral bacterial PA protein or a nucleic acid encoding the PA protein. When the subject has been infected with a PA-deficient, highly virulent bacterium, a hemostatic effect through the induction of platelet aggregation will be provided by supplying PA protein or expressing PA in the subject or bacterium.

Accordingly, the present invention also provides a use of an oral bacterial PA protein or a nucleic acid encoding the PA protein for the production of a hemostatic agent, as well as a method of hemostatic method comprising a step of administering an oral bacterial PA protein or a nucleic acid encoding the PA protein.

The present invention provides, in another embodiment, an inhibitor of platelet aggregation caused by a PA-expressing oral bacterium, the inhibitor comprising a substance that binds to an oral bacterial PA protein or a nucleic acid encoding the PA protein. When the subject has been infected with a PA-expressing oral bacterium, PA in the bacterial cell surface layer may be blocked by a substance that binds to PA protein, or the production of PA by the bacterial cell may be inhibited by a substance that inhibits the expression of PA protein, thereby inhibiting the platelet aggregation effect of the bacterium can be inhibited.

Accordingly, the present invention also provides a use of a substance that binds to an oral bacterial PA protein or a nucleic acid encoding the PA protein for the production of an inhibitor of platelet aggregation caused by a PA-expressing oral bacterium, as well as a method of inhibiting platelet aggregation caused by a PA-expressing oral bacterium comprising a step of administering a substance that binds to an oral bacterial PA protein or a nucleic acid encoding the PA protein.

The present invention provides, in another embodiment, an inhibitor of hemorrhage aggravation comprising a substance that binds to an oral bacterial CBP or a nucleic acid encoding the CBP protein. When the subject has been infected with a CBP-expressing hemorrhage-aggravating oral bacterium, using a substance that binds to CBP, e.g., a CBP-specific antibody, the CBP protein in the bacterial cell surface layer may be blocked and the binding of the bacterial cell to collagen-denuded site (i.e., the damaged site of vascular endothelia) may be inhibited, thereby treating or preventing hemorrhage aggravation. Alternatively, by using a nucleic acid encoding a substance that binds to CBP protein (e.g., an siRNA, antisense nucleic acid), CBP production by a bacterial cell can be inhibited, thereby inhibiting the binding of the bacterial cell to collagen-denuded site.

Accordingly, the present invention also provides a use of a substance that binds to an oral bacterial CBP or a nucleic acid encoding the CBP protein for the production of a hemorrhage aggravation inhibitor, as well as a method of inhibiting hemorrhage aggravation comprising a step of administering a substance that binds to an oral bacterial CBP or a nucleic acid encoding the CBP protein.

The present invention provides, in another embodiment, an agent for detecting collagen-denuded site in tissue comprising CBP of an oral bacterium. When connective tissue collagen is denuded due to vascular endothelia injury, the damaged site can be detected using the detecting agent of the present invention. Particularly, the detecting agent of the present invention allows noninvasive detection of the damaged site even if the hemorrhage site is in an area difficult to be detected, e.g., in head. Various labels may be added to the detecting agent for the convenience of detection. The label may be selected from any known labels, e.g., any radioisotopes, magnetic bodies, a substance that binds to the above-mentioned components (e.g., an antibody), biotin, fluorescent substances, fluorophores, chemiluminescent substances, elements that induce nuclear magnetic resonance (e.g., hydrogen, phosphorus, sodium and fluorine) and enzymes.

Accordingly, the present invention also provides a use of oral bacterial CBP for the production of an agent for detecting collagen-denuded site in tissue, as well as a method of detecting a collagen-denuded site in tissue comprising a step of administering an oral bacterial CBP.

Furthermore, the present invention provides, in another embodiment, a carrier for delivering a substance to the collagen-denuded site comprising an oral bacterial CBP. The hemostatic agent of the present invention or other drugs (e.g., an antibiotic or an anti-inflammatory agent) can be incorporated into the delivering carrier and administering it to an organism to target the hemostatic agent and the drugs to the damaged site, thereby expecting a damaged site-specific therapy. The carrier may be, for example, a liposome fused with a CBP protein or its collagen binding domain (CBD). To the carrier of the present invention, the hemostatic agent of the present invention or other drugs may be incorporated. Alternatively, the carrier of the present invention may be the CBP protein itself, and in this case, the therapeutic agent can directly be bound to the CBP protein or CBD.

The present invention provides, in another embodiment, a therapeutic agent for hemorrhage comprising an oral bacterial CBP and a hemostatic agent. The therapeutic agent for hemorrhage of the present invention is particularly useful in a subject having low platelet sensitivity to collagen. A subject having low platelet sensitivity to collagen includes a subject suffering such as aplastic anemia, acute leukemia, thrombocytopenic purpura, disseminated intravascular coagulation, thrombotic thrombocytopenic purpura, systemic lupus erythematosus, thrombasthenia or storage pool syndrome. Also, the therapeutic agent for hemorrhage of the present invention is particularly useful in a subject having a disease caused by a disorder of coagulation factor, such as hemophilia.

The CBP to be used for the carrier for substance delivery to the collagen-denuded site and therapeutic agent for hemorrhage of the present invention may be obtained, for example, by incorporating a nucleic acid construct comprising CBP gene into a suitable expression vector, and expressing CBP protein in the suitable host cell. Such techniques are well known in the art. For example, a plasmid, cosmid, phage, virus, YAC or BAC vector system comprising CBP gene can be incorporated into a host cell by various nucleic acid introducing method, e.g., calcium phosphate method, lipofection method, ultrasonic introduction method, electroporation method, particle gun method, microinjection method, liposome method (e.g., by cationic liposome), competent cell method or protoplast method to express CBP gene. CBP may also be the CBP-positive bacterium itself, or the CBP-containing component of the CBP-positive bacterium. Such component may be isolated by, for example, lysing and/or homogenizing CBP-positive bacteria and exposing to a substrate coated with Type I collagen. If the CBP-positive bacterium itself is to be used, said bacterium may be inactivated by a conventional method.

Moreover, the present invention relates to, in another embodiment, a prophylactic agent for hemorrhage aggravation comprising an agent for removing an oral bacterium. According to the method of the present invention, in a case if a hemorrhage-aggravating oral bacterium has been detected, the hemorrhage-aggravating oral bacterium should be removed from the subject in order to alleviate the risk of hemorrhage aggravation and prevent it. As an oral bacterium-removing agent e.g., beta-lactam antibiotic may be used. A beta-lactam antibiotic includes, e.g., penicillin, methicillin, cephalosporin, cephamycin and carbapenems.

The hemostatic agent, platelet aggregation inhibitor, hemorrhage aggravation inhibitor, prophylactic agent for hemorrhage aggravation, therapeutic agent for hemorrhage, collagen-denuded site detecting agent and the carrier for substance delivery to the collagen-denuded site of the present invention may be administered by various routes encompasses oral and parenteral routes, such as, for example, oral, buccal, intravenous, intramuscular, subcutaneous, topical, rectal, intraarterial, intraportal, intraventricular, transmucosal, transdermal, intranasal, intraperitoneal, intrapulmonary and intrauterine routes, and may be formulated into a dosage form suitable for each administration route. Any known dosage form and method for formulation may be employed as appropriate (see, e.g., Watanabe et al., eds., 2003, HYOJUN YAKUZAIGAKU, Nanzando).

For example, formulations suitable for oral administration include, a powder, granule, tablet, capsule, liquid, suspension, emulsion, gel and syrup. Formulations suitable for parenteral administration include injections such as an injectable solution, injectable suspension, injectable emulsion, and preparation-at-use injection. A formulation for parenteral administration may be in a form of aqueous or nonaqueous isotonic sterile solution or suspension. Specifically, for example, it may be formulated into a suitable unit dosage form, by combining appropriately with a pharmacologically acceptable carrier or medium such as, in specific, sterile water or physiological saline, vegetable oil, emulsifier, surfactant, stabilizing agent, excipient, vehicle, preservative or a binder. The amount of the effective ingredient in these formulations may be determined as appropriate so that a therapeutically effective amount can be provided to the subject in the defined dosage frequency.

Injectable aqueous solutions include, for example, a physiological saline, an isotonic solution comprising glucose and other adjuvant, e.g., D-sorbitol, D-mannose, D-mannitol and sodium chloride. Appropriate solubilizing agent such as alcohol, specifically ethanol, a polyalcohol such as propyleneglycol, polyethyleneglycol, or a nonionic surfactant such as polysorbate 80 or HCO-50 may be used in combination.

Oily solutions includes e.g., a sesame oil and soy bean oil, which may be used in combination with a solubilizer such as benzyl benzoate or benzyl alcohol. Moreover, a buffering agent, e.g., a phosphate buffer, sodium acetate buffer, soothing agent, e.g., procaine hydrochloride, stabilizing agent, e.g., benzyl alcohol, phenol or antioxidant may be mixed. The injection prepared is usually filled in an appropriate container such as an ampoule, vial, tube, bottle or a pack.

Administration of hemostatic agent, platelet aggregation inhibitor, hemorrhage aggravation inhibitor, prophylactic agent for hemorrhage aggravation, therapeutic agent for hemorrhage, collagen-denuded site detecting agent and the carrier for substance delivery to the collagen-denuded site of the present invention into the body of subject may be via any of the above-mentioned routes, though, preferably, it is parenteral administration, more preferably topical or intravenous administration, particularly preferably intraportal or intratumoral administration. The frequency of dosage is preferably at once, though plurality of dosage may be used depending on the situation. The duration of dosage may be short, or may be sustained for a long time. More specifically, the composition of the present invention may be administered by injection or transdermally. The examples of administration by injection include local injection, intravenous injection, intra-arterial injection, selective arterial infusion, portal vein injection, intramuscular injection, intraperitoneal injection, subcutaneous injection, intratumoral injection, intrathecal injection, intra-articular injection, intraventricular injection. An intravenous injection allows an administration in a manner of an ordinal blood transfusion, requiring neither a surgical operation to the subject nor local anesthesia, thus enabling alleviating the burden of both the subject and the operator. Moreover, it is advantageous that administration can be carried out elsewhere out of an operation room.

Furthermore, the present invention relates to, in one embodiment, a method of treating hemorrhage comprising administering an effective amount of the hemostatic agent, hemorrhage aggravation inhibitor, prophylactic agent for hemorrhage aggravation and/or therapeutic agent for hemorrhage described above to a subject. The present invention also relates to, in one embodiment, a method of treating a disease condition caused by platelet aggregation comprising administering an effective amount of the platelet aggregation inhibitor described above to a subject. Disease conditions caused by platelet aggregation include thrombosis and disseminated intravascular coagulation.

Moreover, the present invention relates to, in one embodiment, a method for diagnosing the site of hemorrhage comprising administering the collagen-denuded site detecting agent described above to a subject. Furthermore, the present invention relates to, a method of treating a disease associated with hemorrhage comprising administering an effective amount of the carrier for delivering a substance to the collagen-denuded site to a subject.

In the method of treatment or diagnosis of the present invention, the administration of the composition for treatment or diagnosis of the present invention to a subject may appropriately performed according to, for example, above-mentioned administration method. Also, a physician or veterinarian may appropriately modify the administration method described above to administrate the agent of the invention to a subject. Here, an effective amount is an amount of the hemostatic agent, hemorrhage aggravation inhibitor and/or therapeutic agent for hemorrhage described above that inhibits, alleviates or prevents the hemorrhage, or an amount of the platelet aggregation inhibitor that decreases the onset of, alleviates the symptoms or preventing the progress of a disease condition caused by platelet aggregation. It is preferably an amount that does not cause an adverse effect that exceeds the benefit by the administration. Such amount may be determined as appropriate by an *in vitro* examination using cultured cell, etc., or an examination in an animal model of such as a mouse, rat, dog or pig.

Specific amount of the composition for treatment or diagnosis of the present invention to be administered in the method of treatment or diagnosis of the present invention may be determined in consideration of various conditions associated with the subject in need of such treatment, e.g., the severity of the symptom, general health conditions of the subject, age, body weight and sexuality of the subject, diet, timing and frequency of administration, combination therapies, reactivity to the treatment, and the compliance to the treatment, etc., and thus may differ from the general effective amount, though, even in such cases, these methods are still encompassed within the scope of the present invention.

Routes of administration include various routes encompassing both oral and parenteral routes, e.g., oral, buccal, intravenous, intramuscular, subcutaneous, topical, intratumoral, rectal, intraarterial, intraportal, intraventricular (cardiac), transmucosal, transdermal, intranasal, intraperitoneal, intrathecal, intraarticular, intraventricular (brain), intrapulmonary and intrauterine routes.

The frequency of administration may vary depending on the characteristics of the composition to be used and the conditions of the subject as described above, though, for example, it may be plurality of times a day (namely, twice, three times, four times or five times or more a day), or once a day, once per several days (namely, e.g., every 2, 3, 4, 5, 6 or 7 days), once a week, once per several weeks (namely, e.g., every 2, 3 or 4 weeks).

Moreover, in the method of treating hemorrhage of the present invention, a drug other than the hemostatic agent, hemorrhage aggravation inhibitor and/or therapeutic agent for hemorrhage of the present invention which is effective for the treatment of a hemorrhage-associated disease described above may be used in combination. Also, in the method of treating a disease condition caused by platelet aggregation of the present invention, a drug other than the platelet aggregation inhibitor of the present invention which is effective for the treatment of a disease condition caused by platelet aggregation may be used in combination.

The term "subject" in the present invention means any living organism, preferably an animal, still more preferably a mammal, still more preferably a human individual.

## EXAMPLES

Hereinafter, the present invention is more specifically illustrated by way of examples, though the present invention is not to be limited by these examples.

### Materials and Methods

Animal experiments and human subject

All animal experiments in the present study has been carried out in accordance with the Guide for the Care and Use of Laboratory Animals published by US National Institutes of Health (NIH), and approved by the Institutional Animal Care and Use Committee of the Graduate School of Dentistry Osaka University and Hamamatsu University School of Medicine.

Study protocols using human samples have been approved by the ethics committee of the Graduate School of Dentistry Osaka University, Hamamatsu University School of Medicine and Suita Municipal Hospital (Suita City, Osaka, Japan). Before entry, all subjects were asked to sign to a consent form after the explanation about the protocols.

*S. mutans* bacterial strains and culture conditions

Major *S. mutans* strains used in the present study are shown in Table 1 (Reference 11, 21, 24, 25 and 29). Furthermore, 58 clinical *S. mutans* strains (strains isolated from blood: n=13, strains isolated from oral cavity: n=45) were used in the present study. All strains were cultured in Brain Heart Infusion (BHI) broth (Difco Laboratories, Detroit, MI, USA), and erythromycin was added for the selection of the mutant strains. For each assay, bacterial cells were washed with PBS, and diluted to adjust the cell number.

**Table 1**

**Table 1. *S. mutans* used in this study.**

| Strains | Serotypes | Protein expression | Features | References |
|---|---|---|---|---|
| TW295 | *k* | PA(-) Cnm (+) | Blood isolate from Japanese subject with bacteremia after tooth extraction | Fujiwara et al. (2001) |
| TW295-CND | *k* | PA (-) Cnm (-) | Isogenic mutant with defect of Cnm constructed by TW295 | Nomura et al. |
| TW871 | *k* | PA (+) Cnm (+) | Blood isolate from Japanese subject with Infective endocarditis complicated with subarachnoid hemorrhage | Fujiwara et al. (2001) |
| MT8148 | *c* | PA (+) Cnm (-) | Oral isolate from Japanese subject | Ooshima et al. (1983) |
| MT8148-PD | *c* | PA (-) Cnm (-) | Isogenic mutant with defect of PA constructed by MT8148 | Nakano et al. (2006) |
| SA53 | *k* | PA (-) Cnm (+) | Oral isolate from Finnish subject | Nakano et al. (2008) |
| LJ32 | *f* | PA (-) Cnm (+) | Oral isolate from Japanese subject | Nakano et al. (2008) |

Collagen binding assay

The collagen binding properties of the mutant strain and parent strain were assessed by a modified version of the method of Reference 27 (Reference 22). The result for each strain was shown in a percentage relative to the binding of TW871.

Platelet aggregation assay

Platelet aggregation assay were carried out using mouse whole blood by the impedance method with an aggregometer (Whole-blood aggregometer C540, Baxter Ltd., Tokyo, Japan). In brief, whole blood were taken from mice (ICR, male, 8 weeks old, body weight 35 to 40g, CLEA Japan, Inc., Tokyo, Japan), and the mixture of the whole blood and various amount ($10^3$, $10^5$ or $10^7$ CFU) of the bacterial cells were incubated at 37°C for 5 minutes, then 4.0 $\mu$g collagen (native collagen fibril (Type I), Chrono-log Co., Havertown, PA, USA) were added. The aggregation rate for each strain were calculated by the impedance ($\Omega$) values in the presence or absence of the bacterial cells, and expressed as a percentage to that of the vehicle (where only collagen were added). Also, the platelet aggregation properties of 58 clinical strains and 3 MT8148 isogenic mutant strains were analyzed in the presence of $10^7$ bacterial cells.

Assessment of bacterial cell surface charge (zeta potential)

The cell surface charge of the bacteria tested was measured using zeta potential analyzer (ELSZ-2, Otsuka Electronics, Co., Ltd., Hirakawa, Osaka, Japan). Said analyzer automatically calculates the zeta potential from the electrophoretic mobility using Smoluchowski equation. The bacterial cells cultured overnight were washed with PBS, adjusted to be $10^7$ CFU, loaded onto the analyzer, which automatically measured the zeta potential of the cells at five standard points. The results are shown as the mean values.

Mouse model of brain artery injury

In mice, an injury was induced in vascular endothelial cells of the middle cerebral artery using a modified version of the already-described photochemical method (References 12, 28, Fig. 17). BALB/c mice (8 weeks old, male, body weight 20 to 30g) were infected with the suspension of the test bacteria at 1 x $10^7$ CFU/mouse. Subsequently, Rose Bengal was administered, and a damage was given to the middle cerebral artery at one side via photosensitization for 10 minutes using a xenon lamp equipped with a heat absorption filter (0.04 W/$cm^2$, wavelength at 540nm, Hamamatsu Photonics, Hamamatsu, Japan) and an optic fiber of 1.5mm diameter installed onto the micromanipulator, thereby inducing the onset of a mild cerebral hemorrhage. The animals were euthanized 24 hours after the bacterial infection, and the resected brain tissue was sliced at certain intervals, and the area of total hemorrhage site from all brain slices was quantified in $mm^2$ unit by computer analysis according to the already-described method (References 12, 28, DP controller, Model DP70, OLYMPUS).

Activation of matrix-metalloprotease (MMP-9)

Gelatin gel zymography was carried out by a modified version of already-described method (Reference 13). In brief, the tissue sample collected 24 hours after the administration of either tested bacteria or a vehicle was homogenized in a buffer containing 50mM Tris-HCl, 150mM NaCl, 1% Nonidet P-40, 0.1% SDS and 0.1% deoxycholic acid, pH7.4, supplemented with a protease inhibitor. Subsequently, the sample was separated using gelatin-zymo electrophoresis kit (Cosmo Bio., Tokyo, Japan).

*in vivo* electron microscopic observation

Three hours after the induction of cerebral hemorrhage, the brain tissue was resected from the mouse, and the region of cerebral hemorrhage was observed with an electron microscope. In brief, the brain having a hemorrhage was fixed with 2% glutaraldehyde and dissected so that the section included a part of the obstacle, which was then fixed again with 1% osmium tetraoxide and dehydrated through an ethanol series. The sample was frozen, fractured into 2 to 4 pieces using a freeze-fracturing device filled with liquid nitrogen. The torn surface was perpendicular to the cerebral surface and included the hemorrhage site. Fractured samples were desiccated with a freeze-drying apparatus using t-butyl alcohol, then attached to the sample stage using a conductive paste so that the section came on top, and coated with osmium in order to confer conductance. The samples were observed with SEM.

Three-dimensioned computerized tomography of bacterial cells using transmission electron microscopy

Bacterial cell membranes were compared using three-dimensioned reconstructed images generated by a TEM CT (JEM 1220: JEOL Co., Tokyo). The TEM images of the bacterial cells were taken at x 150,000 magnification, at every 1° in a tilt range from -60° to +60°. The three-dimensioned reconstructed CT images were generated using Radon transform software. These CT images can be displayed in any direction.

Detection of bacteria in a tissue sample

Detection of bacterial infection in several organs was carried out as follows using PCR. Total DNA was extracted from resected tissues such as the damaged and undamaged hemispheres of the brain, lung, liver and intestine, and examined by PCR method using *S. mutans*-specific primers (Reference 9) below.

*S. mutans*-specific primers:

Forward: 5’ -GGC ACC ACA ACA TTG GGA AGC TCA GTT-3’ (SEQ ID NO.: 11)
Reverse: 5’ -GGA ATG GCC GCT AAG TCA ACA GGA T-3’ (SEQ ID NO.: 12)

The detection limit of bacteria was from 5 to 50 cells in each sample. In order to confirm the presence of the viable cells in tissue, each tissue resected was compressed in PBS, then the stock and diluted solutions were streak-cultured on a bacitracin (100 units/ml; Sigma-Aldrich, St. Louis, MO. USA)-containing Mitis-Salivariusagar plate (Difco) which is an agar plate for selective culture.

Production of CBP gene knockout strain (CND strain): TW295CD

TW295 strain *cnm* gene fragment was amplified using following primers designed based on the full length sequence of *cnm* gene encoding CBP of TW295 strain (SEQ ID NO.: 4: DDBJ Accession No.AB469913)

Primers for amplification of *cnm*:

*cnm*1F 5'-GAC AAA GAA ATG AAA GAT GT-3'(SEQ ID NO.: 13)
*cnm*1R 5'-GCA AAG ACT CTT GTC CCT GC-3'(SEQ ID NO.: 14)

Amplified fragment was incorporated into pGEM-T Easy vector (Promega, Madison, WI, USA) to generate the plasmid pTN11. pTN11 was treated with the restriction enzyme BsmI to digest the middle part of the open reading flame of *cnm* and generated the plasmid pTN12, in which an erythromycin-resistant gene fragment obtained from the plasmid pKN100 was incorporated. pTN12 was disassembled into single strands using the restriction enzyme PstI, and homologously recombined into TW295 strain by a chemical procedures using horse serum. The screening of a strain having an erythromycin resistant gene in the middle part of *cnm* gene (CND strain) was carried out using an erythromycin-containing *S. mutans*-selection medium. Generated strain was confirmed by Southern hybridization and measurement of collagen binding ability.

Production of PA gene knockout strain (PD strain): MT8148PD

According to the method described in Nakano et al. Microbes Infect. 2006 8(1):114-21, PD strain was generated and confirmed by a similar method as the CND strain above using primers based on the full sequence of *pac* gene encoding PA of MT8148 strain (SEQ ID NO.: 2: DDBJ Accession No.X14490).

Primers for the amplification of *pac*:

*pac*-F 5' -GCG CGC ATG CTT TAT TCA GAT TTG GAG GAT-3' (SEQ ID NO.: 15)
*pac*-R 5' -GCG AAA GCG CAT GCT GTG ATT TAT CGC TTC-3' (SEQ ID NO.: 16)

Statistical Analysis

Statistical Analysis was performed using Prism 4 software (GraphPad Software Inc., San Diego, CA., USA). Fisher's PLSD, Student's t-test, regression analysis and ANOVA was performed. The result was considered significant if $p<0.05$.

Statistics of the frequency of carrying *S. mutans* bacterial surface protein

For 170 strains of *S. mutans* isolated from 170 child patients who consulted to Osaka University Graduate School of Dentistry, Department of Pediatric Dentistry from 2002 to 2003, frequencies of carrying PA and CBP. Furthermore, the malignancy of hemorrhage aggravation in representative bacterial strains was determined in mouse cerebral hemorrhage model.

**Results**

**Example 1.** Examination of the virulence of *Streptococcus mutans* in mouse cerebral hemorrhage model:

At 24 hours after the onset of cerebral hemorrhage, in the control group which had not been given the bacteria at all, a mild cerebral hemorrhage was confirmed in the downstream of middle cerebral artery in the vessel-damaged hemisphere (Fig. 4a および 4b). This cerebral hemorrhage induces cerebral infarction (Reference 12). In the group which had been infected with MT8148 strain, no exacerbation of cerebral hemorrhage was confirmed as compared to vehicle control (Fig. 4c). On the other hand, in the group which had been given a serotype *k* TW871 strain isolated from a patient with subarachnoid hemorrhage, a dramatic exacerbation of cerebral hemorrhage was confirmed (Fig. 4c). Similarly, in groups of mice which had been infected with other serotype *k* strains TW295, SA53 or LJ32 strain, a significant increase in hemorrhage area was observed as compared to the control group and the group infected with MT8148 strain (Fig. 4). TW295 and SA53 strain induced the maximum increase in cerebral hemorrhage area. However, administration of TW295 strain itself did not cause any changes in blood pressure, heart rate and cerebral blood flow (Tables 2 and 3). Microscopic observation confirmed an evident hemorrhage 3 hours after the induction of cerebral hemorrhage in a mouse infected with TW295 strain (Fig. 4d).

**Table 2. Parameters of circulation.**

| Parameters | Time after administration (min) | | | | | | |
|---|---|---|---|---|---|---|---|
| | Pre | 5 | 10 | 15 | 20 | 30 | 40 |
| Systolic blood pressure (mmHg) | | | | | | | |
| Control | 83.0 ± 2.5 | 77.3 ± 3.7 | 78.0 ± 2.0 | 79.7 ± 2.8 | 80.3 ± 2.6 | 79.0 ± 1.5 | 78.0 ± 2.5 |
| TW295 | 75.5 ± 1.3 | 74.3 ± 4.8 | 76.0 ± 1.0 | 74.3 ± 1.4 | 73.3 ± 1.4 | 74.5 ± 0.9 | 73.8 ± 0.6 |
| Diastolic blood pressure (mmHg) | | | | | | | |
| Control | 73.3 ± 2.4 | 68.7 ± 3.5 | 68.7 ± 1.9 | 70.7 ± 2.8 | 70.0 ± 2.3 | 68.7 ± 1.9 | 66.7 ± 2.2 |
| TW295 | 66.0 ± 1.4 | 66.3 ± 4.1 | 66.8 ± 1.1 | 66.3 ± 1.5 | 64.8 ± 1.3 | 65.5 ± 0.3 | 64.5 ± 0.3 |
| Mean blood pressure (mmHg) | | | | | | | |
| Control | 76.0 ± 2.1 | 71.7 ± 3.4 | 72.7 ± 2.4 | 72.7 ± 2.3 | 73.3 ± 1.8 | 72.3 ± 1.5 | 71.0 ± 2.1 |
| TW295 | 69.0 ± 1.0 | 68.8 ± 4.7 | 70.0 ± 0.4 | 68.5 ± 1.2 | 67.3 ± 1.3 | 68.8 ± 0.9 | 67.3 ± 1.1 |
| Heart rate (beats/min) | | | | | | | |
| Control | 428.3 ± 30.6 | 403.0 ± 33.6 | 409.3 ± 32.7 | 409.3 ± 29.8 | 407.7 ± 29.6 | 403.0 ± 28.0 | 399.3 ± 28.1 |
| TW295 | 439.0 ± 14.5 | 426.0 ± 9.3 | 425.5 ± 10.9 | 424.8 ± 11.6 | 421.3 ± 11.3 | 414.5 ± 12.4 | 411.5 ± 14.5 |
| Mean ± SEM (n = 3-4). | | | | | | | |

**Table 3. Cerebral blood flow.**

| Parameters | Control | TW295 |
|---|---|---|
| Occlusion time (sec) | 396.7 ± 126.7 | 469.0 ± 101.8 |
| Total flow time (sec) | 1612.0 ± 644.9 | 1749.3 ± 287.6 |
| Mean ± SEM(n = 3-4). | | |

Moreover, the effect of TW295 strain on activation of matrix-metalloprotease (MMP)-9 was investigated. Destruction of vascular obstacle by activated MMP-9 is an important amplifying route that causes further hemorrhage (References 12, 13). As shown in Fig. 4e, in the vessel-damaged hemisphere of the damaged mouse brain, an activation of MMP-9 was confirmed. The administration of TW295 strain stimulated further activation of MMP-9 in the vessel-damaged hemisphere as compared to the control. However, it should be noted that the administration of TW295 strain in a mouse having no cerebral artery injury did not cause any damage to the tissue of the mouse even at 24 hours after the onset of cerebral hemorrhage(Fig. 4a and 4b). These results suggest that both a cerebrovascular event and the presence of a serotype kbacterium are necessary for aggravation of cerebral hemorrhage.

<u>Example 2. Examination of the relation between collagen binding activity and cerebral hemorrhage</u>

In order to testify the hypothesis that the administered bacteria are localized specifically to the damaged site, the localization of *S. mutans* in the damaged tissue after bacterial administration was investigated. The transfer of the bacteria to each organ was examined by PCR method, and transfer of the administered TW295 strain was observed only the ipsilateral hemisphere of the vascular injury, but not in other parts of the brain or in other organs (Fig. 5a and 5b). Furthermore, *in vivo* electron microscopic observation (Fig. 5c) and optical microscopy observation (Fig. 6) confirmed the localization of the bacterial cells in the vessels in the damaged hemisphere in which vascular endothelia had been damaged and collagen fibers had been denuded. Moreover, attachment of the bacterial cells to collagen fibers in the damaged vessels (Fig. 5d). These results suggest that the administered bacteria specifically interact *in vivo* with the damaged vessels, especially via denuded collagen fibers.

Accordingly, the inventors focused on the direct interaction of serotype *k S. mutans* and collagen fibers. It has been known that denuded collagen fibers are present in the vascular surface of the vessel damaged by the disruption of endothelial cells, and that the interaction of the collagen fibers and platelets is important for platelet aggregation. Recently, a cell surface collagen binding protein of 120kDa on (CBP, also known as collagen binding adhisin and Cnm) has been identified in *S. mutans*, and its coding gene (*cnm*) has been cloned and its sequence has been disclosed (Reference 14). Among *S. mutans* clinical strain, about 10% are carrying CBP, and their distribution is dominant in serotype *k* or *f* strain (Reference 15 and 16). Interestingly, all of the highly virulent strain observed in the cases of human cerebral hemorrhage described hereinbelow (TW871, TW295, SA53 and LJ32, see, Fig. 4c) have this surface protein (Fig. 7). In fact, it was shown that the collagen binding activity *in vitro* of the highly virulent strain was dramatically higher than MT8148 strain (Fig. 8a). Furthermore, it was evidenced that *in vitro* treatment of blood with highly virulent bacterium decreases the level of platelet aggregation as compared to the case when MT8148 strain is used (Fig. 9a).

The inventors generated a mutant strain (TW295CND, Table 1) that is deficient in expression of collagen binding adhisin, from TW295 strain. Suppression of platelet aggregation observed in TW295 strain was completely recovered in TW295CND strain (Fig. 9a). These results indicate collagen binding protein is necessary for collagen binding activity and platelet aggregation inhibitory activity of TW295 strain.

Subsequently, the inventors administered TW295CND strain to a mouse cerebral hemorrhage model. As shown in Fig. 8b, the area of cerebral hemorrhage in the TW295CND-administered mouse was dramatically lower than those in the TW295 strain-administered mice. There was an evident interaction between collagen binding activity and hemorrhage area (Fig. 8c), as well as between collagen binding activity and platelet aggregation inhibitory ability (Fig. 9b), respectively. These results indicate that collagen binding protein in serotype *k S. mutans* is a major cause of the high virulence of the bacterium of this serotype in cerebral hemorrhage. Also, the PA-knockout mutant strain (MT8148PD) derived from the *S. mutans* standard strain MT8148 exacerbated cerebral hemorrhage as compared to the control (Fig. 8b). This result indicates that the deficiency in PA expression is involved in aggravation of cerebral hemorrhage by a highly virulent strain that expresses collagen binding protein.

3. Inhibition of platelet aggregation by serotype *k S. mutans*

Platelet aggregation is the most important step to hemostasis after a vessel injury. Effects of *S. mutans* of various serotypes on platelet aggregation induced by collagen were examined using mouse whole blood. The standard strain MT8148 did not show any platelet aggregation inhibitory effect in whole blood as compared to the vehicle control (Fig. 10a). On the contrary, serotype *k* TW871 strain showed a significant inhibition of platelet aggregation when $10^7$ cells were added to the whole blood (Fig. 10a).

Also, the effects of clinically isolated 58 other *S. mutans* strains on platelet aggregation were investigated. The platelet aggregation rate in the presence of a serotype *k* strain was significantly lower than other serotypes ($p<0.05$; Fig. 10b). Among these, TW295 strain showed the most potent platelet aggregation inhibition. Interestingly, the platelet aggregation rate in the presence of a blood-isolated strain was significantly lower than an oral cavity-isolated strain ($p<0.001$; Fig. 10c). A similar result was observed when TW871 strain was added to a platelet aggregation using platelet-rich plasma. Moreover, it was observed that the duration of hemorrhage tends to be longer after administrating TW295 strain to a mouse (data not shown).

However, arachidonic acid-induced platelet aggregation was not inhibited by administration of TW295 strain (Fig. 10e). The inventors made a hypothesis that TW295 strain inhibits collagen thereby inhibiting platelet aggregation. As shown in the scanning electron microscope (SEM) images in Fig. 11a, in the platelet fraction collected after the stimulation by collagen, an interaction between platelets and collagen which results in platelet activation was observed (left panel, vehicle). In the vehicle control group, morphological changes associated with platelet aggregation such as pseudopodia and platelet adhesion were observed. Addition of MT8148 strain did not show any effects on the interaction between platelets and collagen as compared to the control (Fig. 11a right panel). On the contrary, it was shown that addition of TW295 strain clearly suppressed the interaction between platelets and collagen, and resulted in the inhibition of platelet activation (Fig. 11a, middle panel). These results clearly indicate that the attachment of TW295 to collagen inhibits the interaction between platelets and collagen, thereby inhibiting the aggregation.

4. Assessment of bacterial surface ionic charge

The ionic charge of the platelet surface is an important factor that induces an interaction with the denuded collagen fibers of the damaged vessel. The anionicity of the platelet surface provides an interaction with the cation charge of collagen (References 17 to 19). Accordingly, the ionic charge of bacterial cell surface which may influence the interaction with collagen was measured. The mean value of the zeta potential (which is used as an index of the cell surface ionic charge) of MT8148 cells lysed in physiological saline was - 0.75 mV, which is almost nonionic (Fig. 11b). On the contrary, the zeta potential of serotype *k* strains such as TW295 strain and TW871 strain was - 13.51 mV and - 8.42 mV, respectively, showing much lower values than that of MT8148 strain, indicating that the cell surface condition of a serotype *k* strain is anionic (Fig. 11b). The regression analysis between the zeta potential value and the platelet aggregation rate indicated a significant positive correlation (Fig. 12).

Studies have been done in order to elucidate the role of *S. mutans* surface protein antigen as a virulence factor of dental caries, and a 190kDa protein antigen (PA) has been known to be relevant to the initial attachment to dental surface (Reference 20). It has been shown that a PA-knockout strain has a decreased antigenicity as compared to a strain with a normal expression of PA, and thus maintains a prolonged duration of bacteremia (Reference 21). The cell surface condition of the PA-knockout isogenic mutant strain generated from MT8148 (MT8148PD, Reference 21) was measured. The mean value of the zeta potential of MT8148PD was much lower than that of MT8148 strain (Fig. 11b). The presence or absence of these molecules is considered to be involved in the determination of the surface ionic charge of the serotype *k S. mutans.*

The transmission electron microscopy observation (TEM, Fig. 11c) and SEM observation (Fig. 11d) showed a remarkable difference in the cell surface between MT8148 strain and a serotype *k* strain such as TW295 strain. The PA-knockout strain generated from MT8148 strain showed a quite similar surface condition to that of TW295 strain (Fig. 11c). Bacterial membrane structure observed by TEM using ultra-thin sections may change depending on the direction of observation. In addition, it is necessary to consider the thickness of the section in ultrastructure observation. Accordingly, the bacterial membranes were compared using three-dimensional images reconstructed from computerized tomography (CT) of TEM. The peptidoglycan layer of MT8148 was observed as a transparent and smooth layer in a three-dimensional TEM image (Fig. 13a), whereas the peptidoglycan layer of TW295 was opaque and its boundary was obscure (Fig. 13b). These results clearly indicate that the bacterial surface containing the peptidoglycan layer greatly differ depending on the deficiency or the presence, and this difference results in the condition of the ionically charged surface of serotype *k S. mutans.*

5. *S. mutans* strain isolated from human stroke patients

In order to prove the hypothesis that the infection of CBP gene-expressing *S. mutans* is a risk factor of stroke, the frequency of the occurrence of *S. mutans* carrying the collagen binding protein among stroke patients using oral cavity samples. The results are shown in Table 4.

Table 4. Detection frequency of CBP-carrying *S. mutans* in stroke patients.

| No. | Age | Gender | Diagnosis | Major Complications | *S. mutans* | *cnm* |
|---|---|---|---|---|---|---|
| 1 | 64 | M | Cerebral Hemorrhage | Hypertension, Hyperlipidemia | + | ND |
| 2 | 75 | M | Cerebral Hemorrhage | Hypertension, Angina | + | + |
| 3 | 58 | M | Infarction | Stomach cancer | ND | ND |
| 4 | 84 | M | Infarction | Anemia, Rental Failure | + | + |
| 5 | 67 | M | Cerebral Hemorrhage | Cerebral aneurysm | ND | ND |
| 6 | 83 | M | Infarction | Hypertension | ND | ND |
| 7 | 75 | M | Cerebral Hemorrhage | Hypertension | ND | ND |
| 8 | 58 | M | Infarction | Hypertension, Diabetes | + | ND |
| 9 | 63 | F | Infarction | Hyperlipidemia | + | ND |
| 10 | 80 | F | Infarction | Hypertension, Diabetes | + | ND |
| 11 | 81 | M | subarachnoid hemorrhage | | ND | ND |
| 12 | 76 | F | Infarction | Hypertension | + | + |
| 13 | 70 | F | Infarction | Hypertension, Diabetes | + | ND |
| 14 | 51 | F | Cerebral Hemorrhage | Hypertension, Hyperlipidemia | ND | ND |
| 15 | 67 | M | Cerebral Hemorrhage | Hypertension, Hyperlipidemia | + | |
| 16 | 62 | M | Infarction | Hypertension | + | ND |
| 17 | 49 | M | Cerebral Hemorrhage | | + | + |

ND: not detected because of lower than detection limit (10 CFU/ml).

Among 17 cases of stroke patients, *S. mutans* was isolated from the patients in 11 cases. 5 cases among those were infected with CBP gene-expressing *S. mutans* (5/11, 45.5%, Table 4). This is much higher than the frequency of detecting collagen binding protein-carrying *S. mutans* in healthy subjects (10%). These results suggest that the infection with CBP gene-expressing *S. mutans* is likely to be a risk factor of stroke.

Furthermore, the virulence of isolated CBP gene-expressing *S. mutans* was examined in mice. Among the CBP-expressing *S. mutans* strains isolated from stroke patients, two strains (SMH4 and SMH6, Fig. 14a and 14b) in which both collagen binding activity and platelet aggregation inhibitory activity are higher as compared to those of wild-type MT8148 strain were investigated in a mouse cerebral hemorrhage model. In a mouse to which either SMH4 or SMH6 strain had been administrated, a dramatic aggravation of cerebral hemorrhage was observed as compared to the control (wild-type MT8148 strain) (Fig. 15a, 15b and 15c). These results clearly indicate that the *S. mutans* strain isolated from a stroke patient is a risk factor that causes stroke.

6. Correlation between the frequency of occurrence of PA and CBP-carrying strain and the malignancy of hemorrhage aggravation.

Table 5 summarizes the results of the investigation of the frequency of carrying bacterial surface protein by *S. mutans* for 170 strains of *S. mutans* isolated from 170 child patients. Malignancy was estimated from the area of hemorrhage region caused by each bacterial strain in the mouse cerebral hemorrhage model.

Table 5. Expression frequency of cell surface proteins for *S. mutans.*

| Frequency in the oral cavity | S. mutans | PA | CBP | malignancy |
|---|---|---|---|---|
| 1.8% | + | - | + | 100% |
| 1.2% | + | - | - | 50-70% |
| 8.2% | + | + | + | 40-60% |
| 88.8% | + | + | - | 0 |

Strains that do not express PA shared 3% of the overall, while strains that do not carry CBP occupy 90% of the total. Malignancy in cerebral hemorrhage was determined to be the highest in 1.8% of strains that do not carry PA and that carries CBP from the area of hemorrhage region caused by each bacterial strain, and which was defined as 100% malignancy. According to this definition, the malignancy of the strains that do not express PA and that do not carry CBP (frequency = about 1.2%) and the strains that express PA and that carry CBP (frequency = about 8.2%) were determined about 50 to 70% and about 40 to 60%, respectively. This result agrees to the experimental results using PA and CBP gene knockout strains described above.

Discussion

In the present study, it is first shown that a CBP-expressing and/or PA-deficient *S. mutans* is potential risk factor of a disease associated with hemorrhage, especially hemorrhagic stroke.

In the present study, an aggravation of cerebral hemorrhage by serotype *k S. mutans* strain was confirmed. Furthermore, since infectious bacteria were detected only in the vessel-damaged hemisphere but not in the contralateral hemisphere, it was shown that the interaction between the serotype *k S. mutans* and the damaged vessel is an important event in the onset of cerebral hemorrhage. These strains show the expression of the collagen binding protein (CBP) and/or the deficiency in the protein antigen (PA) as a common protein expression pattern, which are shown to be important in aggravation of cerebral hemorrhage (Fig. 7). The hypothesis by the inventors that the collagen binding protein of a serotype *k S. mutans* is involved in the onset of cerebral hemorrhage is supported by the present result that the collagen binding protein-deficient mutant strain TW295CND did not induce an aggravation of cerebral hemorrhage. The highly virulent strains TW295, TW871, SA53 and LJ32 all express on their surface the collagen binding protein, and have a potent collagen binding property. Accordingly, due to the accumulation of a bacterial strain having a potent collagen binding property to the denuded collagen layer, activating MMP-9 and inhibiting platelet aggregation, further bleeding is brought about. Therefore, a strain having the collagen binding protein should be considered as a highly virulent strain of cerebral hemorrhage. In fact, an *in vivo* SEM imaging of damaged vessel in a mouse brain demonstrated that although there were infectious bacteria, no platelet aggregation had been occurred.

Another potential virulent factor of cerebral hemorrhage is the deficiency in protein antigen (PA) expression. The highly virulent strains TW295, SA53 and LJ32 all were shown to be deficient in PA expression. On the other hand, TW871 expresses PA antigen (Fig. 7 and Table 1), and therefore the cerebral hemorrhage area in TW871 strain-treated mouse was much smaller than the cerebral hemorrhage area in mice treated with other highly virulent strains (Fig. 4c). Moreover, PA-knockout strain derived from the *S. mutans* standard strain MT8148 (MT8148PD) showed cerebral hemorrhage aggravation as compared to the control (Fig. 8b). Furthermore, the platelet aggregation rate was significantly lower in strains showing no PA expression as compared to in strains showing PA expression (data not shown). These results indicate the relevance of PA deficiency to cerebral hemorrhage aggravation by a highly virulent strain expressing the collagen binding protein.

In general, collagen is cationic under physiological conditions, and therefore the ionic properties of bacterial surface are considered to be important in their interaction with denuded collagen fibers. In fact, PA-deficient isogenic mutant shows the lowest zeta potential value, and other PA-knockout strains also tend to have a low zeta potential value. This indicates that PA influences zeta potential value. Because there was a positive correlation between the zeta potential value and collagen-induced platelet aggregation rate, a strain having a low zeta potential value can also be categorized as a highly virulent strain. From these results, it can be considered that a strain expressing *S. mutans* collagen binding protein possesses a high affinity to denuded collagen fibers, and a low level expression of PA in *S. mutans* inclines the cell surface condition to be anionic, which further increases the affinity with cationic collagen fibers. The synergic effect of the presence of the collagen binding protein and the deficiency in 190kDa protein results in a strong bound to collagen fibers and an accumulation of highly virulent bacteria to collagen-denuded vessels. Bacterial accumulation subsequently leads the activation of MMP-9 and inhibition of platelet aggregation in the damaged vessels, resulting in an acceleration of hemorrhage and hemorrhagic infarction (Fig. 16).

Among the patients infected with *S. mutans*, the rate of those who has been infected with strains expressing collagen binding protein is estimated to be 8 to 10% (Reference 16, 22). On the other hand, PA is normally expressed in most strains, and the strains as little as 4% do not express it (Reference 21). Accordingly, a *S. mutans* strain that expresses collagen binding protein and that is deficient in PA expression, i.e., a strain with an extremely high virulence is quite rare, and a limited number of strains become a potential risk factor of cerebral hemorrhage aggravation due to *S. mutans* bacteremia. Because the therapeutic approaches for cerebral hemorrhage are limited after its onset, prophylaxis is considered to be the most important approach (Reference 23). Accordingly, it is important to identify a patient who has been infected with a highly virulent *S. mutans* strain for the prevention of cerebral hemorrhage. In fact, the inventors has isolated CBP-expressing, highly virulent TW295-type *S. mutans* from stroke patients with an extremely high frequency. Moreover, some of such strains also induced cerebral hemorrhage aggravation in a mouse model of hemorrhagic infarction, which indicates the relevance of a highly virulent *S. mutans* in the onset of hemorrhagic stroke.

From these results, it can be concluded that infection by a highly virulent, stroke-inducing *S. mutans* is a potential risk factor of stroke. Two important virulent factors of cerebral hemorrhage are the presence of collagen binding protein and the deficiency in PA expression, which are the common features shared by many of clinically isolated serotype *k* strains. Accordingly, the possession or deficiency of PA and/or CBP by a *S. mutans* strain can be an index for the determination of the risk at the hemorrhage in a carrier, which can be useful in prevention of cerebral hemorrhage.

Detection Example 1. Detection of *Streptococcus mutans* having a cell surface layer structure which may becomes a risk at hemorrhage

Materials and methods

| | |
|---|---|
| Tested bacteria: | Following bacteria were used in the establishment of the detection system. |
| *S. mutans* | MT8148 strain (PA+/CBP-) /TW295 strain (PA-/CBP+) |
| *S. sobrinus* | B13 strain /6715 strain |
| *S. sanguinis* | ATCC10556 strain |
| *S. oralis* | ATCC10557 strain |
| *S. gordonii* | ATCC10558 strain |
| *S. salivarius* | HHT strain |

Analysis 1. Method for culturing *S. mutans* (mutans streptococci) (operation time: about 5 minutes, waiting time (such as during culturing of a bacterium):2 days)

Culturing of *S. mutans* employs following things:

- spitz for collecting saliva (not particularly limited as long as it is sterilized and suitable for collecting and seeding)
- a special dropper capable of collecting saliva of 10 μl
- Special Medium A (agar medium) (24-well plate (it is not particularly limited as long as it is a plate of about 24-well, e.g., 24 well with Lid MICROPLATE (IWAKI)) coated with MSB agar medium e.g., Mitis-salivariusagar medium (Difco Laboratories) is supplemented with bacitracin (100 unit/ml; SIGMA-ALDRICH) and 15% sucrose (Wako Pure Chemical Industries, Ltd.). It is preferred to be provided with Anaero Pack®.)

- a sterilized toothpick for picking up bacterial colonies
- Special Medium B (liquid medium) (sterilized Brain Heart Infusion (BHI) liquid medium (Difco Laboratories) contained in a disposal test tube).

Culturing of *S. mutans* is carried out as follows:

The saliva of the subject is collected in a small amount using the spitz for collecting saliva. 10 μl of the saliva is taken from the spitz using the special dropper, plated onto Special Medium A, then cultured at 37°C for 48 hours, preferably in an anaerobic condition. After culturing, the presence of bacterial colonies is confirmed on gloss, colonies (rough colonies are desirable) are picked up and added into Special Medium B, cultured at 37°C for 18 hours, and used in following Analyses 2, 3 and 4. Cultures of *S. sobrinus*, *S. sanguinis, S. oralis*, *S. gordonii*, and *S. salivarius* are used as controls, and in Analysis 1, it is confirmed that no bacterium other than *S. mutans* and *S. sobrinus* grows.

Analysis 2. Method for detecting *S. mutans* (mutans streptococci)

(operation time: about 15 minutes, waiting time (such as during culturing of a bacterium):about 3 hours)

Although the method of culturing mutans streptococci of above Analysis 1 is provided with conditions in which the mutans streptococci group (*S. mutans* /*S. sobrinus*) can preferably grow, a bacterium having bacitracin-resistance other than mutans streptococci may grow. Therefore, confirmation is done in this step.
Detection employs following things:

- a special dropper capable of collecting bacterial solution of 10 μl
- Special Medium C (96-well plate (e.g., MULTI WELL PLATE for ELISA (SUMIRON)) containing 100 μl of BHI solution containing 1% sucrose (Wako Pure Chemical Industries, Ltd.))
- Wash Buffer A (PBS solution)
- Buffer 1(a solution in which 0.5% crystal violet (Wako Pure Chemical Industries, Ltd.) is added to sterile distilled water)
- Buffer 2 (7% acetate (Wako Pure Chemical Industries, Ltd.) solution)

Detection is carried out as follows:

10 μl of the bacterial solution cultured according to the method of Analysis 1 is added to Special Medium C, incubated at 37°C for 3 hours. The Special Medium C is washed 3 times with Wash Buffer A, then left still for approximately 15 minutes after the last Wash Buffer A is added. Wash Buffer A is removed, and the Special Medium C is washed once again with the Wash Buffer A, then 100 μl Buffer 1 is added to the Special Medium C, left still for 1 minute. This is washed 3 times with Wash Buffer A, and 200 μl of Buffer 2 is added thereto.

It is determined to be *S. mutans*-positive if the color of the medium is changed, *S. mutan*-negative if the color of the medium is unchanged.

Analysis 3. Method for detecting PA-deleted *S. mutans*

(operation time: about 30 minutes, waiting time (such as during culturing of a bacterium):about 11 hours and 30 minutes)

Detection of PA-deleted *S. mutans* employs following things:

- Special Plate (96-well plate; MICROTEST U-Bottom (BECTON DICKINSON))
- Wash Buffer B (a PBST solution in which 0.05% of Triton X-100 (Wako Pure Chemical Industries, Ltd.) is added to Wash Buffer A used in Analysis 2)
- Buffer 3 (a mixture of Tris buffered saline, pH6.8, 100mM dithiothreitol (Wako Pure Chemical Industries, Ltd.) and 20% glycerin (Wako Pure Chemical Industries, Ltd.))
- Buffer 4 (a PBST solution supplemented with 5%skimmed milk (BECTON DICKINSON))
- Buffer 5 (a PBST solution supplemented with 0.1%rabbit anti-PA antiserum (stored in our laboratory))
- Buffer 6 (a PBST solution supplemented with 0.1% porcine anti-rabbit immunoglobulin antibody (Dakopatts))
- Buffer 7 (a solution in which AP (100mM 2-amino-2-hydroxymethyl-1,3-propanediol, 5mM magnesium chloride, 100mM sodium chloride) buffer is supplemented with NBT solution (Wako Pure Chemical Industries, Ltd.) at 0.6% final concentration and BCIP solution (Wako Pure Chemical Industries, Ltd.) at 0.33%final concentration.)

Detection of PA-deleted *S. mutans* is carried out as follows:

(1) Sample preparation

To 100 μl of the bacterial solution cultured according to the method of Analysis 1 above, Buffer 3 is added, and immersed in boiling water for 10 minutes, and frozen if it is to be stored.

(2) Detection of PA-deleted *S. mutans*

1) (1) 100 μl of the sample prepared as above is added to the Special Plate, left still overnight at 4°C.
2) The Special Plate was washed 3 times in Wash Buffer B, then 100 μl of Buffer 4 is added thereto, left still at room temperature for 1 hour.
3) The Special Plate was washed 3 times in Wash Buffer B, then 100 μl of Buffer 5 is added thereto, reacted at room temperature for 1 hour.
4) The Special Plate was washed 3 times in Wash Buffer B, then 100 μl of Buffer 6 is added thereto, reacted at room temperature for 1 hour.
5) The Special Plate was washed 3 times in Wash Buffer B, then 100 μl of Buffer 7 is added thereto, and after 15 minutes changes in the color of the solution are observed. It is determined to be PA-positive if the color of the solution is changed, PA-negative if the color of the solution is not changed. Cultures of *S. sobrinus*, *S. sanguinis, S. oralis*, *S. gordonii*, and *S. salivarius* are used as controls, and in Analysis 3, it is confirmed that no bacterium other than PA-carrying *S. mutans* shows a positive reaction.

**<u>Analysis 4. Detection method of CBP-carrying *S. mutans*</u>**

(operation time: about 30 minutes, waiting time (such as during culturing of a bacterium): about 3 hours and 30 minutes)

Detection of CBP-carrying *S. mutans* employs the followings:

- Special Medium D (the Special Plate used in Analysis 3, to which a mixed solution of sterile distilled water supplemented with 0.6% acetate and Type I collagen (Sigma) in 9: 1 ration was added.)
- Wash Buffer A (the same buffer as that used in above Analysis 2 (detection method of *S. mutans*))
- Buffer 8 (Wash Buffer A supplemented with 5% bovine albumin (Sigma))
- Wash Buffer C (Wash Buffer A which is a PBST solution supplemented with 0.01% Tween 20 (Wako Pure Chemical Industries, Ltd.))
- Buffer 9 (sterile distilled water supplemented with 25% formaldehyde (Wako Pure Chemical Industries, Ltd.))
- Buffer 1 (the same buffer as that used in above Analysis 2)
- Buffer 2 (the same buffer as that used in above Analysis 2)

Detection of CBP-carrying *S. mutans* is carried out as follows:

(1) Special Medium D is washed three times with Wash Buffer A, then 200 μl of Buffer 8 is added thereto, and left still at 37°C for 1 hour.
(2) Washed three times with Wash Buffer C, then 200 μl of the bacterial solution cultured according to the method of 1 described above is added thereto, and incubated at 37°C for 2 hours.
(3) Washed three times with Wash Buffer A, then 200 μl of Buffer 9 is added thereto, and left still at room temperature for 30 minutes.
(4) Washed three times with Wash Buffer A, then 200 μl of Buffer 1 is added to the 96-well plate, and left still for 1 minute.
(5) Washed three times with Wash Buffer A, then 200 μl of Buffer 2 is added thereto.

It is determined to be CBP-positive if the color of the solution is changed, CBP-negative if the color of the solution is not changed. Cultures of *S. sobrinus*, *S. sanguinis*, *S. oralis*, *S. gordonii*, and *S. salivarius* are used as controls, and in Analysis 4, it is confirmed that no bacterium other than CBP-carrying *S. mutans* shows a positive reaction.

**Analysis Example 1**

Fig. 11 is an example of the result of an analysis on whether the *S. mutans* in saliva samples (A, B and C) collected from 3 subjects are PA and/or CBP-carrying strains. As results of culturing saliva samples in Special Medium A (bacitracin-selection agar medium) in steps in Analysis 1, colony formation was confirmed in all of A, B and C. Formed colonies are picked up and cultured in Special Medium B at 37°C for 18 hours. Moreover, cultures of *S. sobrinus*, *S. sanguinis*, *S. oralis*, *S. gordonii*, and *S. salivarius* were cultured similarly as controls, and in Analysis 1, it was confirmed that no bacterium other than *S. mutans* and *S. sobrinus* grew.

Subsequently, in steps in Analysis 2, the bacterial solution cultured in Analysis 1 was added to Special Medium C, incubated at 37°C for 3 hours, washed with Wash Buffer A, then stained with Buffer 1 containing crystal violet. Since the buffer was changed to blue-violet in the medium in which samples A and B has been cultured, the presence of *S. mutans* was determined. As the buffer remained transparent in the medium in which sample C has been cultured, no presence of *S. mutans* was determined.

In steps in Analysis 3, Buffer 3 was added to each of the bacterial solutions of the samples A and B cultured in Analysis 1 and boiled for 10 minutes, and stored frozen. This was added to Special Plate (96-well plate: MICROTEST U-Bottom(BECTON DICKINSON)), left still overnight at 4°C. After washing with Wash Buffer B, Buffer 4 was added and blocked at room temperature for 1 hour, then Buffer 5 containing rabbit anti-PA antiserum was added and reacted at room temperature for 1 hour. After washing with Wash Buffer B, Buffer 6 containing porcine anti-rabbit immunoglobulin antibody was added and reacted at room temperature for 1 hour. After washing with Wash Buffer B, Buffer 7 which contained an alkaline phosphatase reaction-detecting reagent was added, and after 15 minutes changes in the color of the solution were observed. Since the solution was changed to pink in the plate of the sample A, the presence of PA-carrying *S. mutans* was determined. As the color of the solution remained transparent for the sample B, no presence of PA-carrying *S. mutans* was determined. Similar analysis was performed using cultures of *S. sobrinus*, *S. sanguinis*, *S. oralis*, *S. gordonii*, and *S. salivarius* as controls, confirming that no bacterium other than the PA-carrying *S. mutans* showed a positive reaction.

In steps in Analysis 4, Buffer 8 containing 5% bovine albumin was added to the Special Medium D coated with Type I collagen (Sigma), and left still at 37°C for 1 hour. After washing with Wash Buffer C, bacterial solution cultured in Analysis 1 was added and incubated at 37°C for 2 hours. After washing with Wash Buffer A, Buffer 9 containing 25% formaldehyde was added, left still at room temperature for 30 minutes. After washing with Wash Buffer A, Buffer 1 was added and left still for 1 minute. After washing with Wash Buffer A, Buffer B was added and changes in the color of the solution were observed. Since the color of the solution remained transparent in the plate containing the sample A, no presence of CBP-carrying *S. mutans* was determined. As the color of the solution changed to blue-violet in the plate containing the sample B, the presence of CBP-carrying *S. mutans* was determined. Similar analysis was performed using cultures of *S. sobrinus*, *S. sanguinis*, *S. oralis*, *S. gordonii*, and *S. salivarius* as controls, confirming that no bacterium other than the CBP-carrying *S. mutans* showed a positive reaction.

**Example 4. Optimal conditions for culturing *S. mutans***

In order to obtain a determination with higher accuracy in Analyses 2 to 4 above, it is considered to be important to culture *S. mutans* as many as possible in Analysis 1 and to ensure the contamination of bacteria other than *S. mutans* as little as possible. As conditions for culturing, (1) culturing in an aerobic condition/anaerobic condition, (2) antibiotics (bacitracin) concentration, and (3) nutrient (sucrose) concentration were investigated. Fig. 12 is a graph showing the percentage of *S. mutans* to total bacteria isolated when bacitracin was added to the MSB medium at (a) 1 eq. or (b) 5 eq. (assuming the amount of bacitracin in a conventional MSB medium is 1 eq.) and sucrose was added to the MSB medium at 1 to 4 eq.(assuming the amount of sucrose in a conventional MSB medium is 1 eq.). It was shown that *S. mutans* could be isolated at the highest concentration in an anaerobic condition, when 1 eq. of bacitracin and 1 eq. of sucrose were used. Accordingly, it was shown that in order to obtain a determination with higher accuracy, it is necessary to culture in a sealable container in an anaerobic condition (e.g., in a sealed pack to which Anaero Pack® is attached) in a medium supplemented with bacitracin and sucrose at the same concentration (approx. 100 unit/ml and 15%, respectively) contained in a conventional MSB medium.

**Example 5. Stock period of the sample**

We investigated the stock period of saliva usable for detection of a virulent *S. mutans* under the optimal conditions shown in Example 4 using saliva that has been kept for a certain time after being sampled.

Fig. 13 is a graph showing the separation rate of *S. mutans* when the saliva that had been kept for 0 to 6 months after being sampled was used to perform Analysis 1, assuming the separation rate of *S. mutans* that could be separated when a saliva serially diluted with a sterile physiological saline on the day of being collected was plated onto a MSB agar medium is 100%. Saliva that had been stored frozen at - 20°C after being sampled was used. Sample number: N=8, except the 1 to 2 months-aged sample (N=6). The result shows that it is desired to use the saliva as a sample preferably within 3 months, preferably within 2 months most preferably within 1 month.

The sequences of the protein, polypeptide and nucleic acid used herein are described in the attached sequence listings, as follows:

Table 6. Table of sequences

| SEQ ID No. | Species or strain | content of the sequence |
|---|---|---|
| 1 | S. mutans MT8148 | PA-amino acid |
| 2 | S. mutans MT8148 | PA-DNA |
| 3 | TW295 | CBP-amino acid |
| 4 | TW295 | CBP-DNA-ORF |
| 5 | S. mutans TW295 | CBD-amino acid |
| 6 | S. mutans TW295 | CBD-DNA |
| 7 | S. mutans TW871 | CBP-amino acid |
| 8 | S. mutans TW871 | CBP-DNA-ORF |
| 9 | S. mutans TW871 | CBD-amino acid |
| 10 | S. mutants TW871 | CBD-DNA |
| 11 | Artificial | S. mutans-primer F |
| 12 | Artificial | S.mutans-primerR |
| 13 | Artificial | S. mutans-CBD-primerF (cnm1F) |
| 14 | Artificial | S. mutans-CBP-primer R (cnm1R) |
| 15 | Artificial | S. mutans-PAC- primerF (pac-F) |
| 16 | Artificial | S. mutans-PAC- primer R (pac-R) |
| 17 | S. mutans LJ23 | PA-amino acid |
| 18 | S. mutants LJ23 | PA-DNA |
| 19 | S. mutans SA98 | PA-amino acid |
| 20 | S. mutans SA98 | PA-DNA |
| 21 | S. mutans | antigenI/II-amino acid |
| 22 | S. mutans | antigenI/II gene (spa)-DNA |
| 23 | Neisseria meningitidis | iron-binding protein-amino acid |
| 24 | Neisseria meningitidis | iron-binding protein gene (fbp) DNA |
| 25 | S. mutans SA53 | CBP-amino acid |
| 26 | S. mutans SA53 | CBP-DNA-ORF |
| 27 | S. mutans A53 | CBD-amino acid |
| 28 | S. mutans SA53 | CBD-DNA |
| 29 | S. mutans LJ32 | CBP-amino acid |
| 30 | S. mutans LJ32 | CBP-DNA-ORF |
| 31 | S. mutans LJ32 | CBD-amino acid |
| 32 | S. mutans LJ32 | CBD-DNA |

Table 7

**References**

1. Murray. C. J. & Lopez. A. D.Mortality by cause for eight regions of the world: Global Burden of Disease Study. Lancet 349, 1269-1276 (1997).
2. Donnan. G. A., Fisher. M.. Macleod. M. & Davis. S. M. Stroke. Lancet 371, 1612-1623 (2008).
3. Broderick, J. et al. A guideline from the American Heart Association. American Stroke Association Stroke Council. High Blood Pressure Research Council, and the Quality of Care and Outcomes in Research Interdisciplinary Working Group. Stroke 38, 2001-2023 (2007).
4. Woo. D. et al. Genetic and environmental risk factors for intracerebral hemorrhage: preliminary results of a population-based study. Stroke 33, 1190-1195 (2002).
5. Emsley. H. C. & Tyrrell. P. J. Inflammation and infection in clinical stroke. J. Cereb. Blood Flow. Metab. 22, 1399-1419 (2002).
6. Moreillon, P. & Que. Y. A. Infective endocarditis. Lancet 363, 139-149 (2004).
7. Mylonakis. E. & Calderwood. S. B. Infective endocarditis in adults. N. Engl. J. Med. 345, 1318-1330 (2001).
8. Bayer. A. S. et al. Diagnosis and management of infective endocarditis and its complications. Circulation 98, 2936-2948 (1998).
9. (Nakano. K., (Nomura. R.. Nakagawa. I.. Hamada, S. and Ooshima, T. Demonstration of Streptococcus mutans with a cell wall polysaccharide specific to a new serotype. k. in the human oral cavity. J. Clin. Microbiol. 42, 198-202 (2004).
10. Eishi. K. et al. Surgical management of infective endocarditis associated with cerebral complications. Multi-center retrospective study in Japan. J. Thorac. Cardiovasc. Surg. 110, 1745-1755 (1995).
11. Fujiwara. T. et al. Biochemical and genetic characterization of serologically untypable Streptococcus mutans Strains isolated from patients with bacteremia. Eur. J. Oral Sci. 109, 330-334 (2001).


**Table 8**

12. Zhao. B. Q. et al. Essential role of endogenous tissue plaminogen activator through matrix metalloprotease 9 induction and expression on heparin-produced cerebral hemorrhage after cerebral ischemia in mice. Blood 103, 2610-2616 (2004).
13. Gursoy-Ozdemir. Y. et al. Cortical spreading depression activates and upregulates MMP-9. J. Clin. Invest. 113, 1447-1455 (2004).
14. Sato. Y. et al. Streptococcus mutans strains harboring collagen-binding adhesin. J. Dent. Res. 83, 534-539 (2004).
15. Nakano. K. et al. Detection of novel serotype k Streptococcus mutans in infective endocarditis patients. J. Med. Microbiol. 56, 1413-1415 (2007).
16. Nakano. K. et al. Streptococcus mutans clonal variation revealed by multilocus sequence typing. J. Clin. Microbiol. 45, 2616-2625 (2007).
17. Hampton. J. R. & Mitchell. J. R. A. Effect of aggregating agents on the electrophoretic mobility of human platelets. Br. Med. J. 1, 1074-1077 (1966).
18. Hampton. J. R. & Mitchell. J. R. A. Modification of the electrokinetic response of blood platelets to aggregating agents. Nature 210, 1000-1002 (1966).
19. Boisseau. M. R.. Lorient. M. F.. Born. G. V. R. & Michal. F. Change in electrophoretic mobility associated with the shape change of human blood platelets. Proc. R. Soc. Lond. B Biol Sci. 196, 471-474 (1977).
20. Okahashi. N.. Sasakawa. C.. Yoshikawa. M.. Hamada. S. & Koga. T. Cloning of a surface protein antigen gene from serotype c Streptococcus mutans. Mol. Microbiol. 3, 221-228 (1989).
21. Nakano. K.. Tsuji. M.. Nishimura. K.. Nomura. R. & Ooshima. T. Contribution of cell surface protein antigen PAc of Streptococcus mutans to bacteremia. Microbes Infect. 8, 114-121 (2006).
22. Nomura. R. et al. Molecular and clinical analyses of the gene encoding the collagen-binding adhesin of Streptococcus mutans. J. Med. Microbiol. 58, 469-475 (2009).


**Table 9**

23. Ariesen. M. J.. Clans. S. P.. Rinkel. G. J. & Alegra. A. Risk factors for intracerebral hemorrhage in the general population: a systematic review. Stroke 34, 2060-2065 (2003).
24. Ooshima. T.. Izunitani. A.. Sobue. S.. Okahashi. N. & Hamada. S. Non-cariogenicity of the disaccharide palatinose in experimental dental caries of rats. Infect. Immun. 39, 43-49 (1983).
25. (Nakano. K ef al. Protein antigens in serotypes k Strepococcus mutans clinical isolates. J. Dent. Res. 87, 964-968 (2008).
26. Nakano. K. et al. Molecular characterization of Streptococcus mutans strains containing the cmn gene encoding a collagen-binding adhesin. Arch. Oral Biol. (in press).
27. Waterhouse. J. C. & Russell R. R. B. Dispensable genes and foreign DNA in Streptococcus mutans. Microbiology 152, 1777-1788 (2006).
28. Suzuki. Y.. Nagai. N. & Collen. D. Comparative effects of microplasmin and tissue-type plasminogen activator (tPA) on cerebral hemorrhage in a middle cerebral artery occlusion model in mice. J. Thromb. Haemost. 2, 1617-1621 (2004).
29. Nakano K, (Nomura R, Taniguchi N, Lapirattanakul J, Kojima A, Naka S, Senawongse P, Srisatjaluk R, Gr?nroos L, Alaluusua S, Matsumoto M, Ooshima T. Molecular characterization of Streptococcus mutans strains containing the cnm gene encoding a collagen-binding adhesin. Arch Oral Biol. 55(1):34-9, 2010.

Provided is a method of screening high risk of hemorrhage aggravation in a subject comprising determining if a hemorrhage aggravating Collagen Binding Protein (CBP) carrying *Streptococcus mutans* is detected in a biological sample of the subject.

## Claims

1. A method of screening a subject at a high risk of hemorrhage aggravation, comprising a step of determining as the subject is at a high risk of hemorrhage aggravation if a hemorrhage aggravating Collagen Binding Protein (CBP) carrying *Streptococcus mutans* is detected in a biological sample obtained from a subject.

2. A method of judging the risk of hemorrhage aggravation in a subject, comprising a step of determining as the subject is at a high risk of hemorrhage aggravation if a hemorrhage aggravating CBP-carrying *Streptococcus mutans* is detected in a biological sample obtained from a subject.

3. The method according to Claim 1 or 2, wherein a step further comprises detecting Protein Antigen (PA) of *Streptococcus mutans* in a sample and determining as a hemorrhage aggravating oral bacterium is present if PA is not detected.

4. The method according to any one of Claims 1 to 3, wherein the hemorrhage is hemorrhage by diabrosis.

5. The method according to Claim 4, wherein the hemorrhage by diabrosis is caused by the occurrence of damage on the vascular wall due to traumatic injury, an ulcer, or a ruptured aneurysm.

6. The method according to any one of Claims 1 to 4, wherein CBP is selected from the group consisting of:

   (1) a polypeptide comprising an amino acid sequence according to SEQ ID NO.: 5, 9, 27 or 31;
   (2) a polypeptide comprising one or more mutations in the polypeptide of (1), but having an equal function to the polypeptide of (1);
   (3) a polypeptide comprising an amino acid sequence encoded by a nucleic acid sequence that hybridizes with a nucleic acid sequence according to SEQ ID NOs.: 6, 10, 28 or 32 or its complementary sequence or its fragment under stringent condition, and having an equal function as the polypeptide of (1).

7. The method according to Claim 5, wherein CBP comprises a polypeptide consisting of an amino acid sequence according to SEQ ID NO.: 5, 9, 27 or 31.

## Patentansprüche

1. Verfahren zum Screenen eines Subjekts mit einem hohen Risiko einer Verschlimmerung der Blutung, umfassend einen Schritt des Bestimmens, wenn das Subjekt einem hohen Risiko einer Verschlimmerung der Blutung ausgesetzt ist, ob ein die Blutung verschlimmerndes Kollagenbindendes Protein (CBP)-tragendes *Streptococcus mutans* in einer biologischen Probe, erhalten von einem Subjekt, nachgewiesen wird.

**2.** Verfahren zum Beurteilen des Risikos einer Verschlimmerung der Blutung bei einem Subjekt, umfassend einen Schritt des Bestimmens, wenn das Subjekt einem hohen Risiko einer Verschlimmerung der Blutung ausgesetzt ist, ob ein die Blutung verschlimmerndes CBP-tragendes *Streptococcus mutans* in einer biologischen Probe, erhalten von einem Subjekt, nachgewiesen wird.

**3.** Verfahren nach Anspruch 1 oder 2, wobei ein Schritt weiter das Nachweisen von Proteinantigen (PA) von *Streptococcus mutans* in einer Probe und das Bestimmen umfasst, wenn ein die Blutung verschlimmerndes orales Bakterium vorhanden ist, ob PA nicht nachgewiesen wird.

**4.** Verfahren nach einem der Ansprüche 1 bis 3, wobei die Blutung eine Blutung durch Diabrose ist.

**5.** Verfahren nach Anspruch 4, wobei die Blutung durch Diabrose hervorgerufen ist durch das Auftreten einer Beschädigung der Gefäßwand aufgrund von traumatischen Verletzungen, einem Geschwür oder einem gerissenen Aneurysma

**6.** Verfahren nach einem der Ansprüche 1 bis 4, wobei CBP ausgewählt ist aus der Gruppe, bestehend aus:

(1) einem Polypeptid, umfassend eine Aminosäuresequenz gemäß SEQ ID Nr.: 5, 9, 27 oder 31;
(2) einem Polypeptid, umfassend eine oder mehrere Mutationen im Polypeptid von (1), jedoch mit einer gleichen Funktion wie dem Polypeptid von (1);
(3) einem Polypeptid, umfassend eine Aminosäuresequenz, codiert durch eine Nukleinsäuresequenz, die mit einer Nukleinsäuresequenz gemäß SEQ ID Nr.: 6, 10, 28 oder 32 oder ihrer komplementären Sequenz oder ihrem Fragment unter stringenter Bedingung hybridisiert und eine gleiche Funktion wie das Polypeptid von (1) aufweist.

**7.** Verfahren nach Anspruch 5, wobei CBP ein Polypeptid umfasst, bestehend aus einer Aminosäuresequenz gemäß SEQ ID Nr.: 5, 9, 27 oder 31.

## Revendications

**1.** Procédé de dépistage d'un sujet présentant un risque élevé d'aggravation de l'hémorragie, comprenant une étape consistant à déterminer, lorsque le sujet présente un risque élevé d'aggravation de l'hémorragie, si un *Streptococcus mutans* porteur d'une protéine de liaison du collagène (CBP) aggravant l'hémorragie est détecté dans un échantillon biologique obtenu d'un sujet.

**2.** Procédé d'évaluation du risque d'aggravation de l'hémorragie chez un sujet, comprenant une étape consistant à déterminer, lorsque le sujet présente un risque élevé d'aggravation de l'hémorragie, si un *Streptococcus mutans* porteur d'une CBP aggravant l'hémorragie est détecté dans un échantillon biologique obtenu d'un sujet.

**3.** Procédé selon la revendication 1 ou 2, dans lequel une étape comprend en outre la détection d'antigène protéinique (PA) de *Streptococcus mutans* dans un échantillon et la détermination, lorsqu'une bactérie orale aggravant l'hémorragie est présente, si le PA n'est pas détecté.

**4.** Procédé selon l'une quelconque des revendications 1 à 3, dans lequel l'hémorragie est une hémorragie par diabrose.

**5.** Procédé selon la revendication 4, dans lequel l'hémorragie par diabrose est provoquée par la survenue de dégradations sur la paroi vasculaire dues à une lésion traumatique, un ulcère, ou une rupture d'anévrisme.

**6.** Procédé selon l'une quelconque des revendications 1 à 4, dans lequel la CBP est choisie dans le groupe constitué de :

(1) un polypeptide comprenant une séquence d'acides aminés selon SEQ ID NO : 5, 9, 27 ou 31 ;
(2) un polypeptide comprenant une ou plusieurs mutations dans le polypeptide de (1), mais ayant une fonction égale au polypeptide de (1) ;
(3) un polypeptide comprenant une séquence d'acides aminés codée par une séquence d'acide nucléique qui s'hybride avec une séquence d'acide nucléique selon SEQ ID NO : 6, 10, 28 ou 32 ou sa séquence complémentaire ou son fragment dans des conditions stringentes, et ayant une fonction égale au polypeptide de (1).

**7.** Procédé selon la revendication 5, dans lequel la CBP comprend un polypeptide constitué d'une séquence d'acides aminés selon SEQ ID NO : 5, 9, 27 ou 31.

# Figure 1. Flow-chart of analyses

Analysis 1: Culture of *S. mutans*
Analysis 2: Detection of *S. mutans*
Analysis 3: Detection of PA-deficient *S. mutans*
Analysis 4: Detection of CBP-carrying *S. mutans*

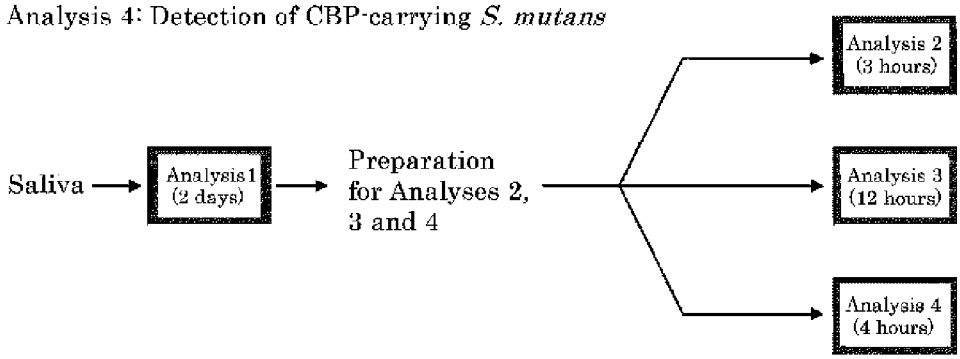


# Figure 2. Analytical procedures

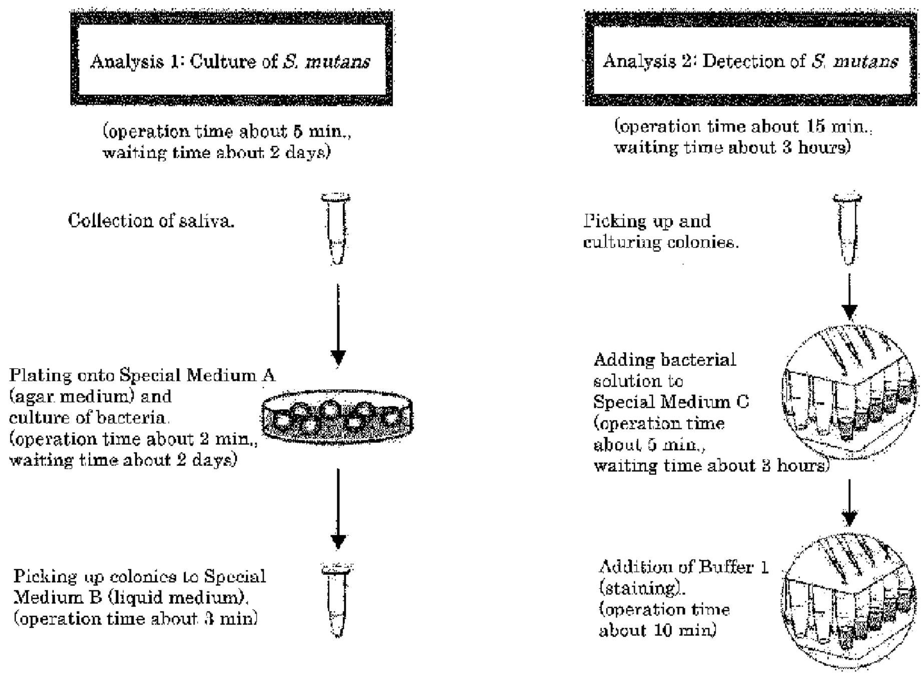

Figure 3.

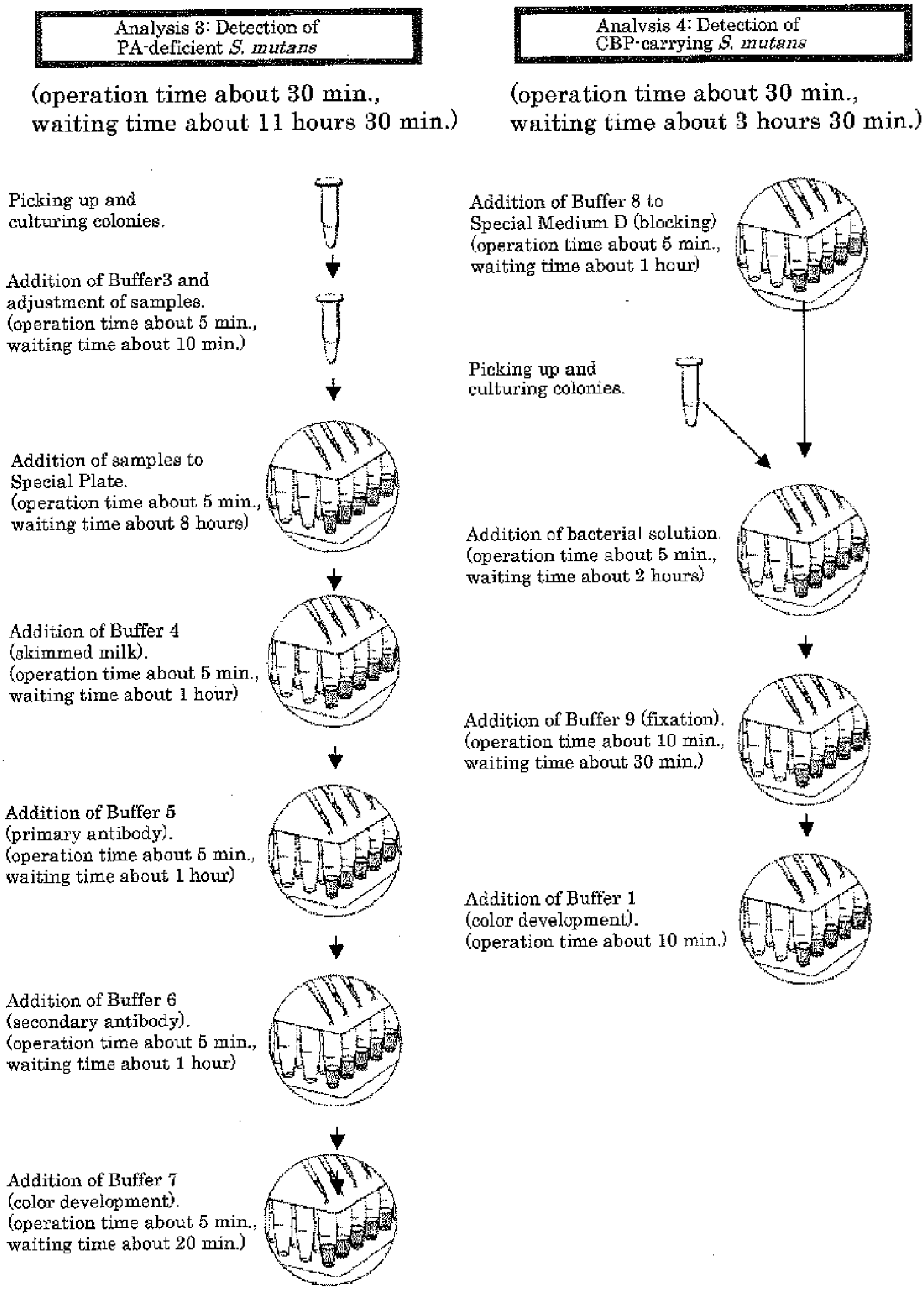

Analysis 3: Detection of PA-deficient *S. mutans*
(operation time about 30 min., waiting time about 11 hours 30 min.)
Picking up and culturing colonies.
Addition of Buffer3 and adjustment of samples. (operation time about 5 min., waiting time about 10 min.)
Addition of samples to Special Plate. (operation time about 5 min., waiting time about 8 hours)
Addition of Buffer 4 (skimmed milk). (operation time about 5 min., waiting time about 1 hour)
Addition of Buffer 5 (primary antibody). (operation time about 5 min., waiting time about 1 hour)
Addition of Buffer 6 (secondary antibody). (operation time about 5 min., waiting time about 1 hour)
Addition of Buffer 7 (color development). (operation time about 5 min., waiting time about 20 min.)
Analysis 4: Detection of CBP-carrying *S. mutans*
(operation time about 30 min., waiting time about 3 hours 30 min.)
Addition of Buffer 8 to Special Medium D (blocking) (operation time about 5 min., waiting time about 1 hour)
Picking up and culturing colonies.
Addition of bacterial solution. (operation time about 5 min., waiting time about 2 hours)
Addition of Buffer 9 (fixation). (operation time about 10 min., waiting time about 30 min.)
Addition of Buffer 1 (color development). (operation time about 10 min.)

Figure 4

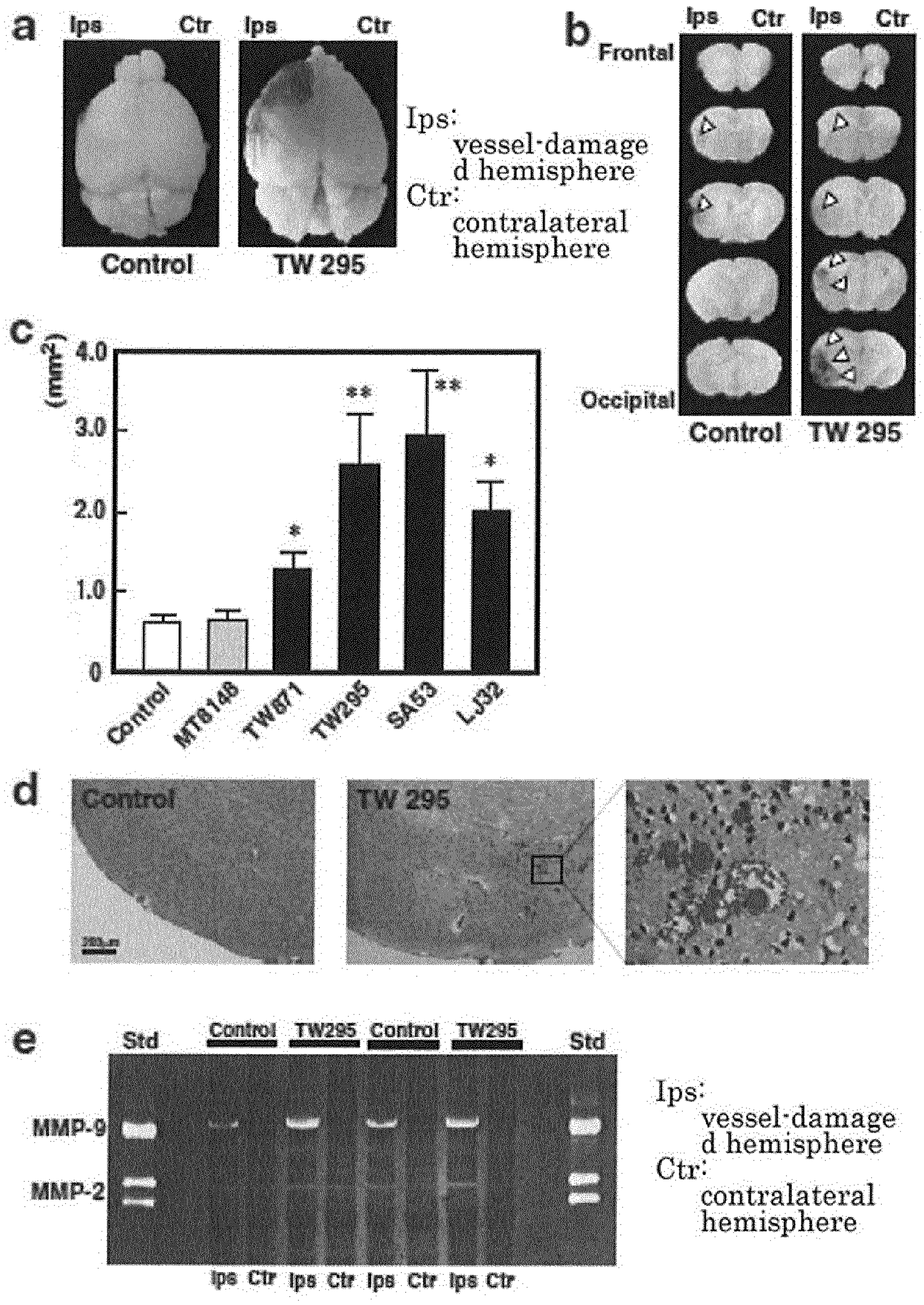
a
Ips
Ctr
Ips
Ctr
Control
TW 295
Ips:
vessel-damaged hemisphere
Ctr:
contralateral hemisphere
b
Ips
Ctr
Ips
Ctr
Frontal
Occipital
Control
TW 295
c
(mm2)
4.0
3.0
2.0
1.0
0
**
**
*
*
Control
MT8148
TW871
TW295
SA53
LJ32
d
Control
TW 295
e
Std
Control
TW295
Control
TW295
Std
MMP-9
MMP-2
Ips
Ctr
Ips
Ctr
Ips
Ctr
Ips
Ctr
Ips:
vessel-damaged hemisphere
Ctr:
contralateral hemisphere

Figure 5

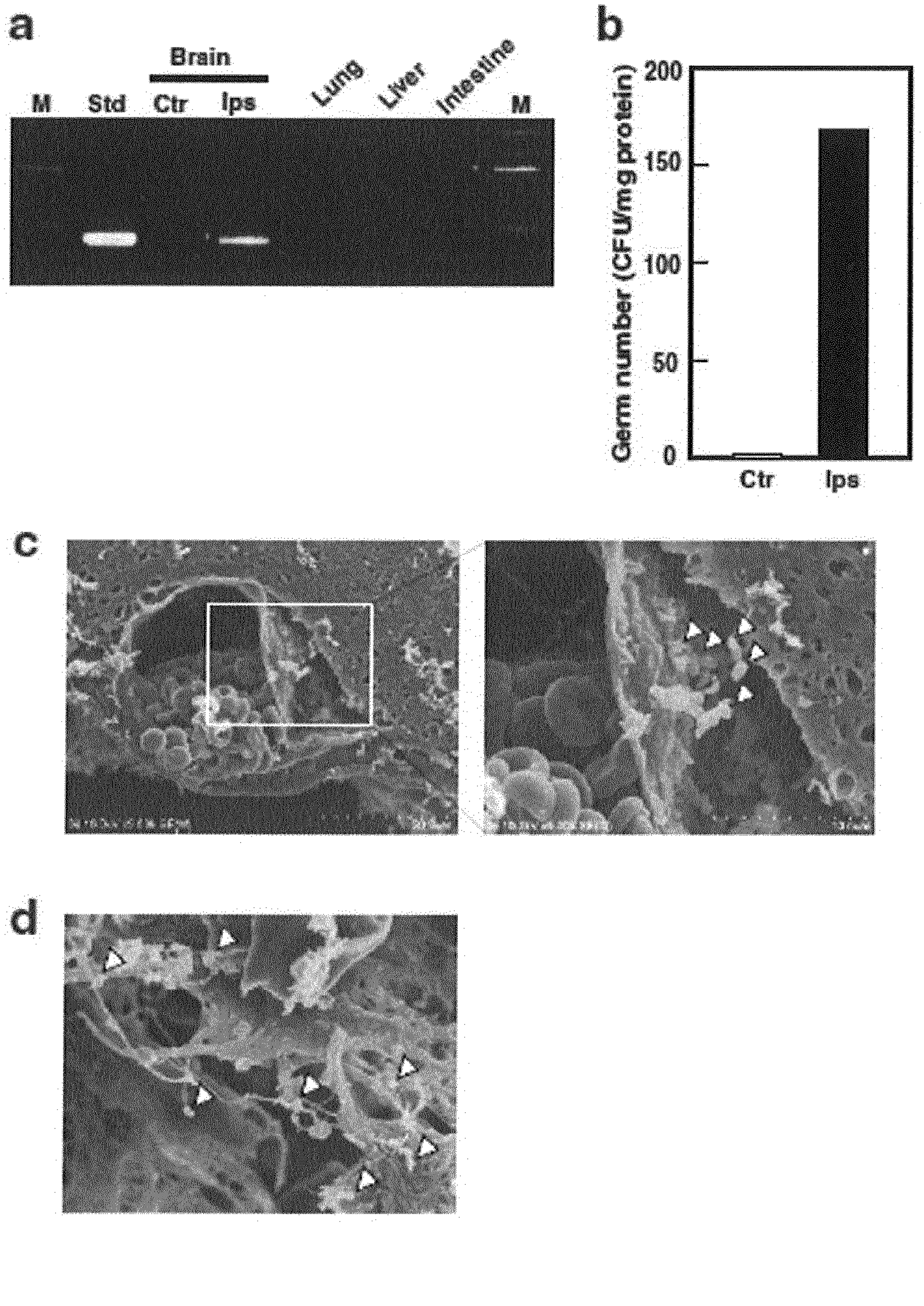
a
Brain
M
Std
Ctr
lps
Lung
Liver
Intestine
M
b
Germ number (CFU/mg protein)
200
150
100
50
0
Ctr
lps
c
d

Figure 6

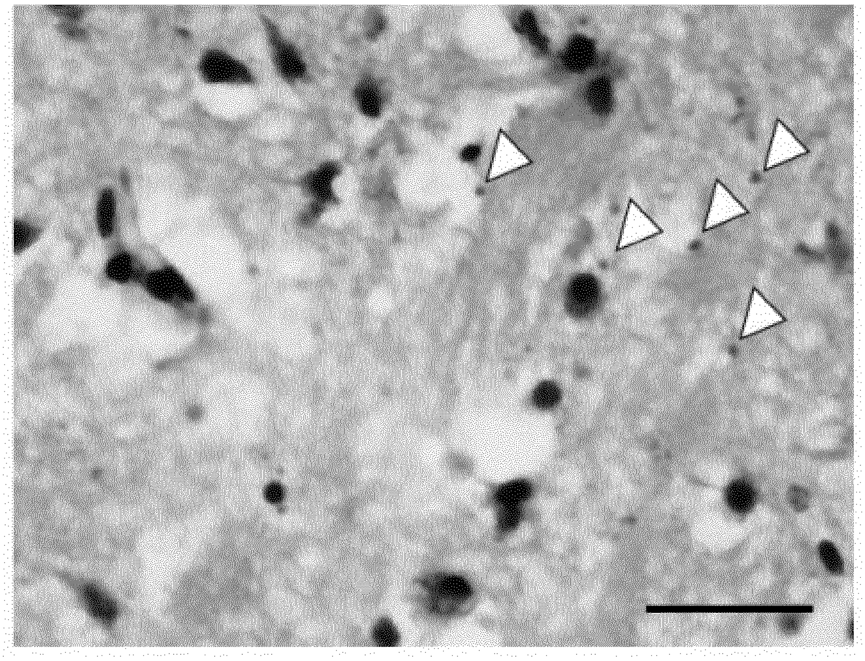

Figure 7

| Strain | CBP | PA |
|---|---|---|
| MT 8148 | — | + |
| TW 871 | + | + |
| TW 295 | + | — |
| SA 53 | + | — |
| LJ 32 | + | — |

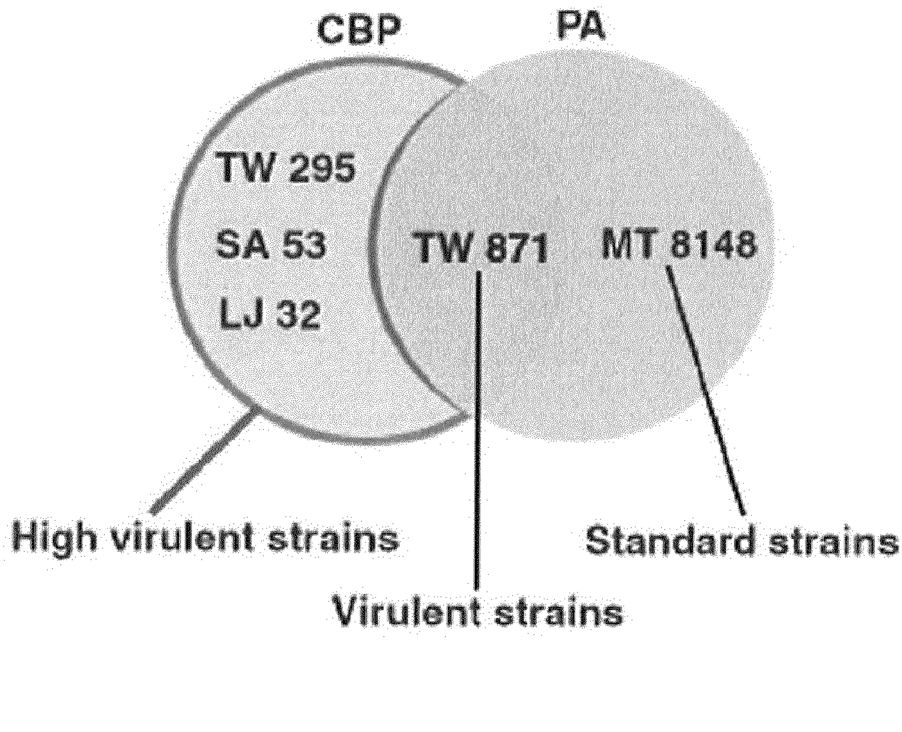

Figure 8

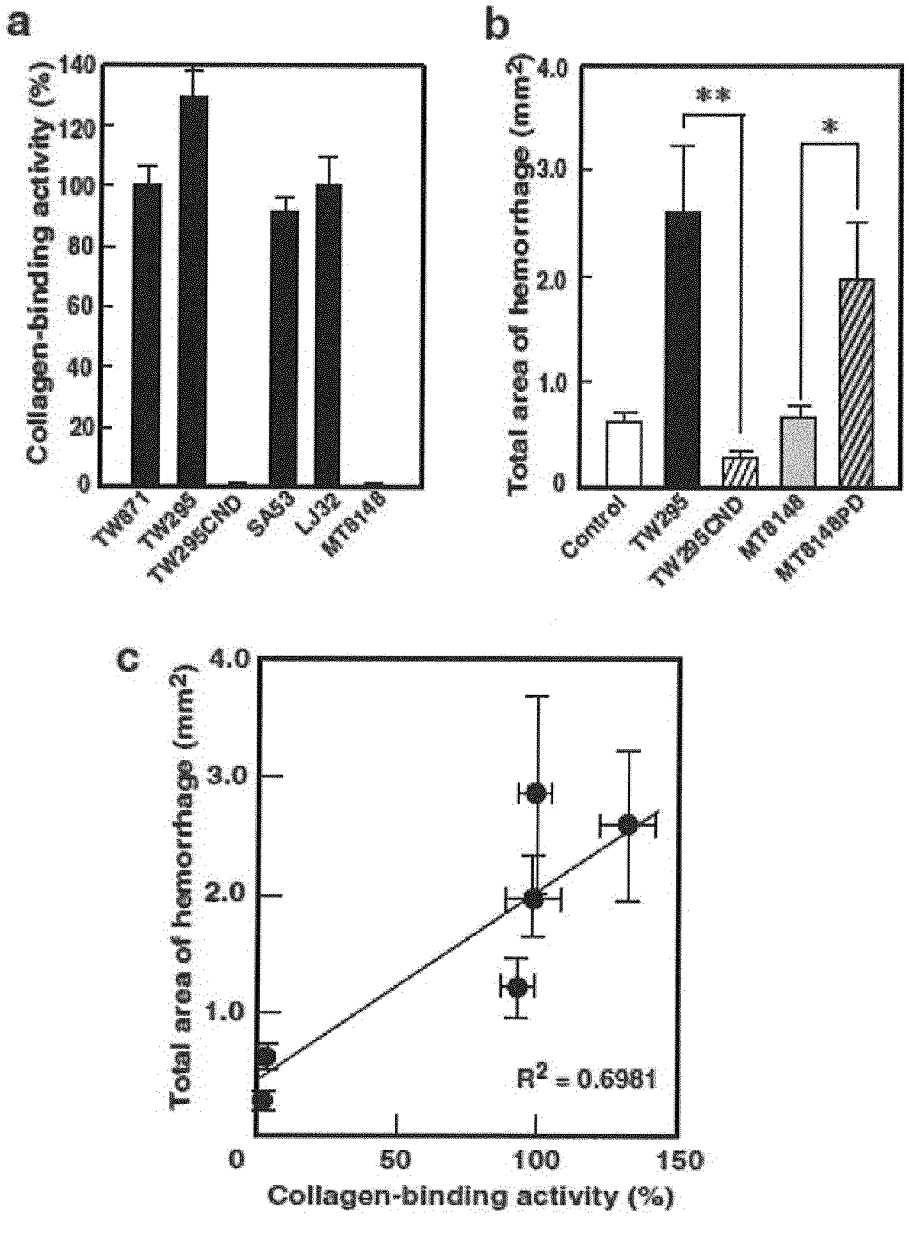
a
Collagen-binding activity (%)
140
120
100
80
60
40
20
0
TW871
TW295
TW295CND
SA53
LJ32
MT8148
b
Total area of hemorrhage (mm2)
4.0
3.0
2.0
1.0
0
**
*
Control
TW295
TW295CND
MT8148
MT8148PD
c
Total area of hemorrhage (mm2)
4.0
3.0
2.0
1.0
R2 = 0.6981
0
50
100
150
Collagen-binding activity (%)

Figure 9

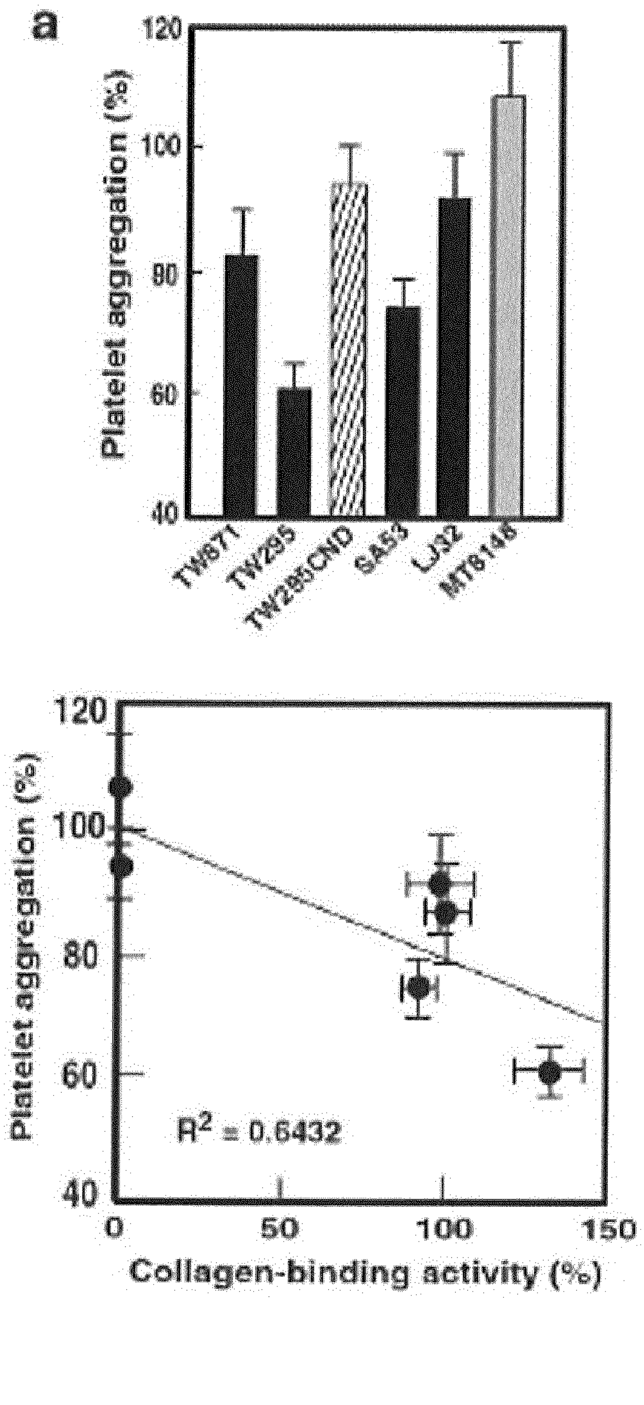
a
Platelet aggregation (%)
120
100
80
60
40
TW671
TW295
TW295CND
SA53
LJ32
MT8148
b
Platelet aggregation (%)
120
100
80
60
40
R2 = 0.6432
0
50
100
150
Collagen-binding activity (%)

Figure 10

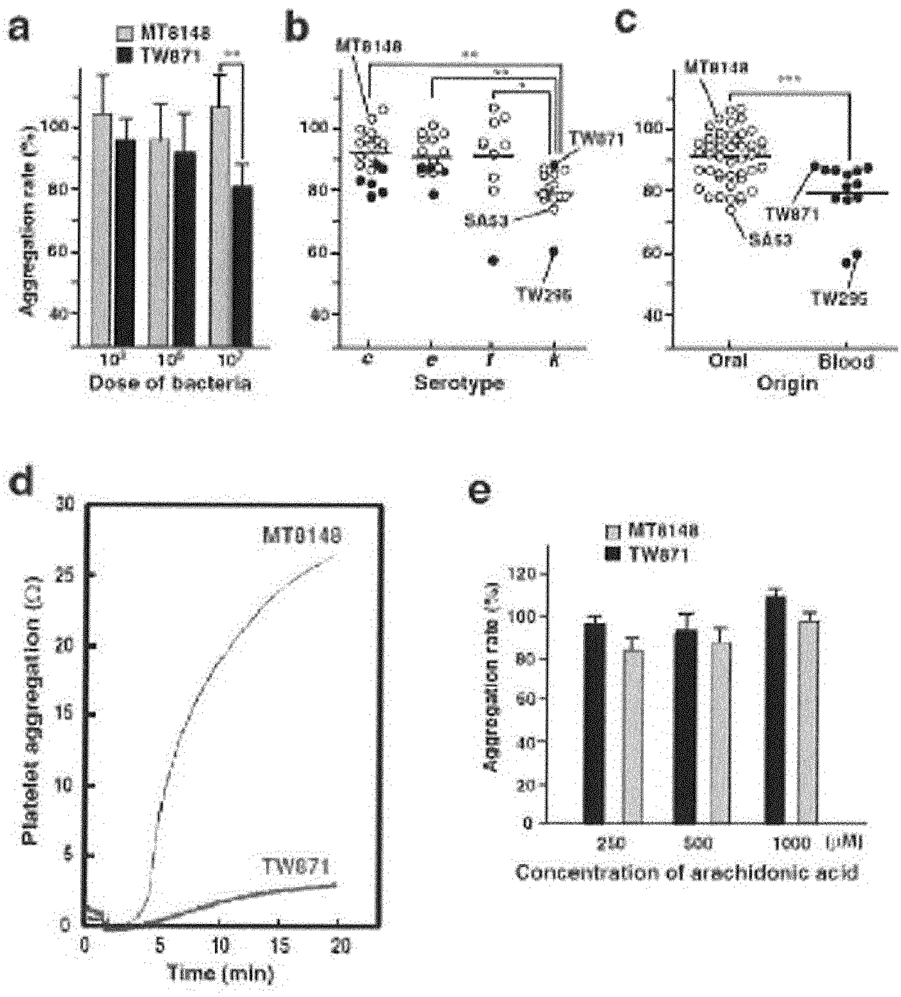
a
MT8148
TW871
Aggregation rate (%)
Dose of bacteria
b
MT8148
TW871
SA53
TW295
Serotype
c
MT8148
TW871
SA53
TW295
Oral
Blood
Origin
d
Platelet aggregation (Ω)
MT8148
TW871
Time (min)
e
MT8148
TW871
Aggregation rate (%)
Concentration of arachidonic acid

# Figure 11

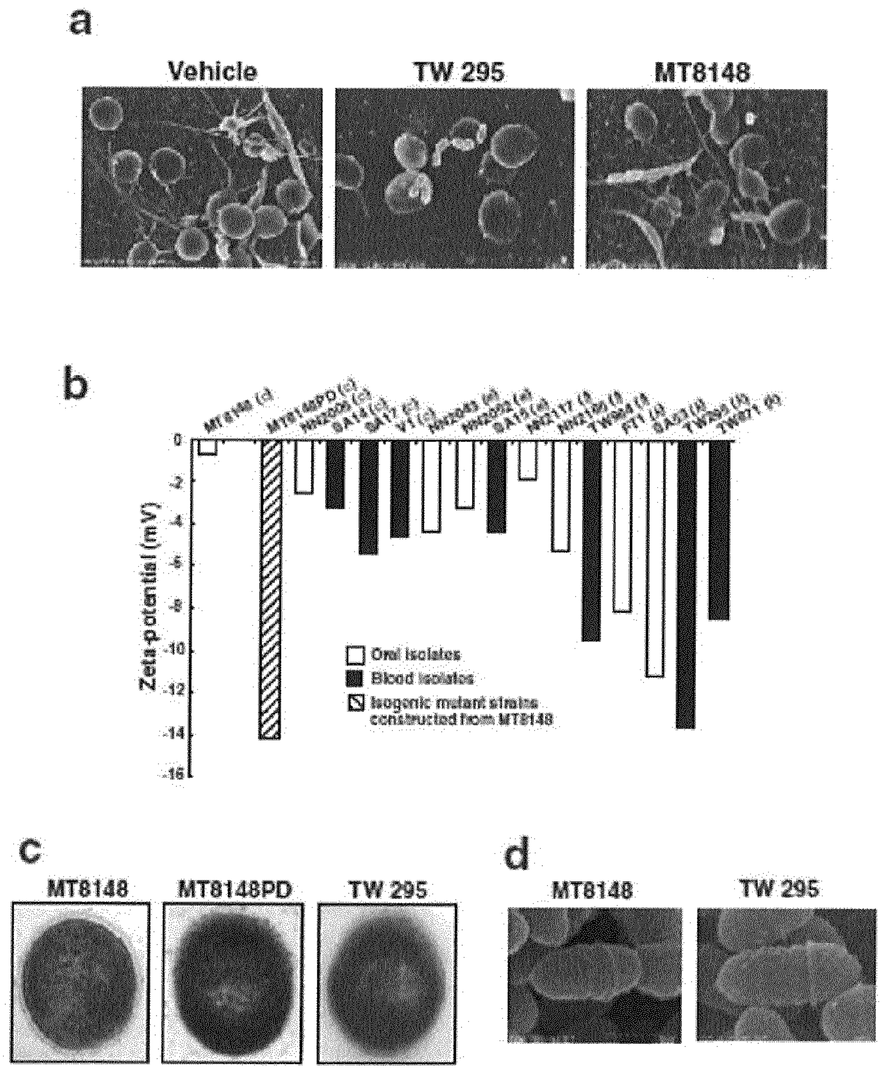
a
Vehicle
TW 295
MT8148
b
Zeta-potential (mV)
0
-2
-4
-6
-8
-10
-12
-14
-16
Oral isolates
Blood isolates
Isogenic mutant strains constructed from MT8148
c
MT8148
MT8148PD
TW 295
d
MT8148
TW 295

Figure 12

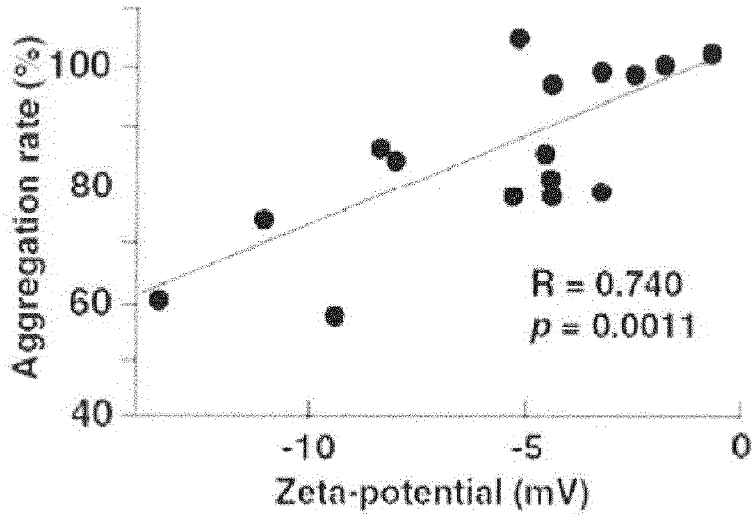


[Fig. 13]

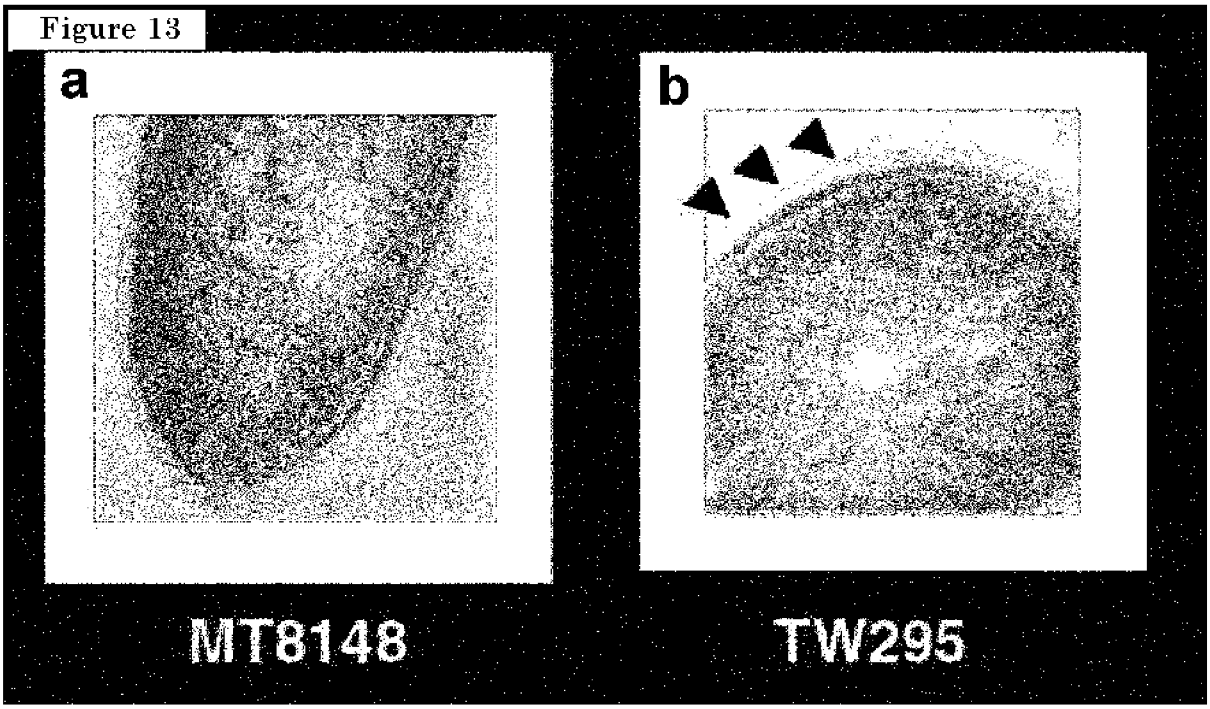

# Figure 14

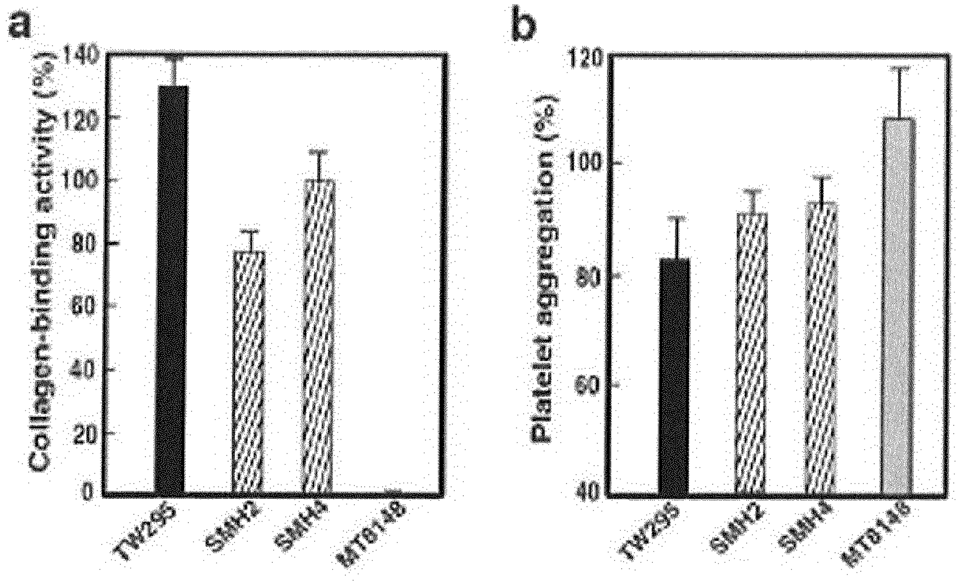
a
Collagen-binding activity (%)
140
120
100
80
60
40
20
0
TW295
SMH2
SMH4
MT8148
b
Platelet aggregation (%)
120
100
80
60
40
TW295
SMH2
SMH4
MT8148

Figure15

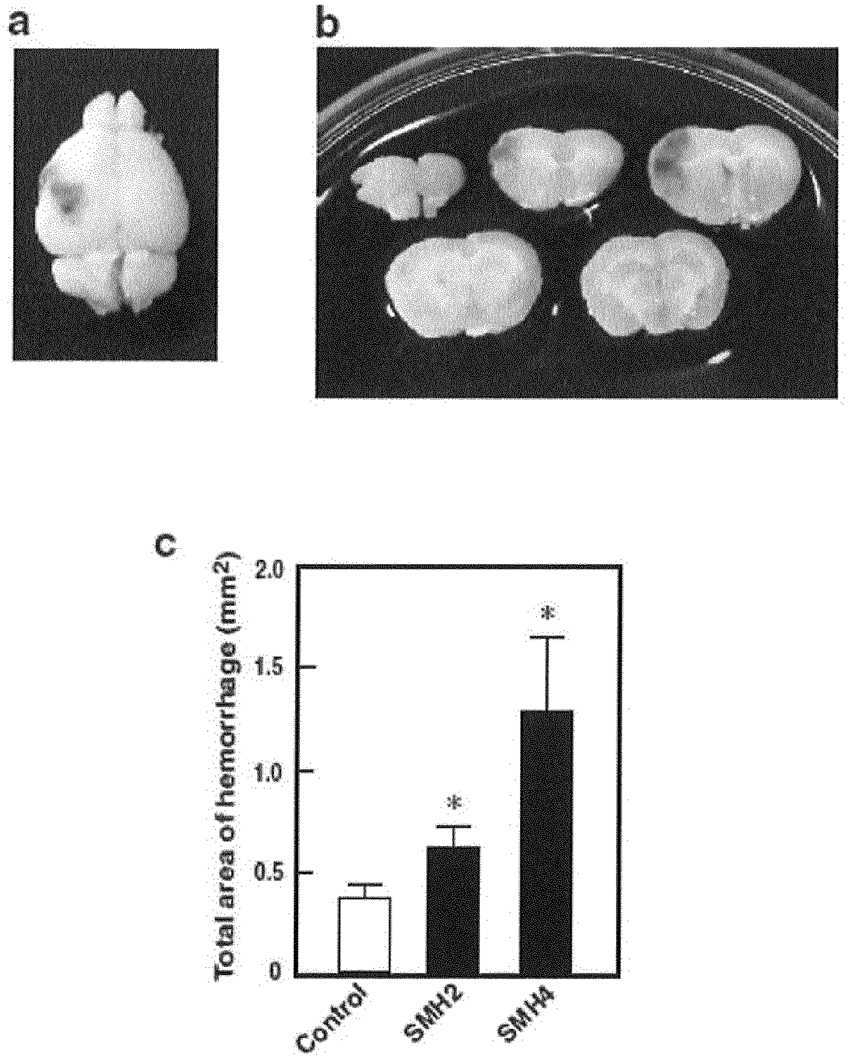
a
b
c
Total area of hemorrhage (mm2)
2.0
1.5
1.0
0.5
0
*
*
Control
SMH2
SMH4

Figure16

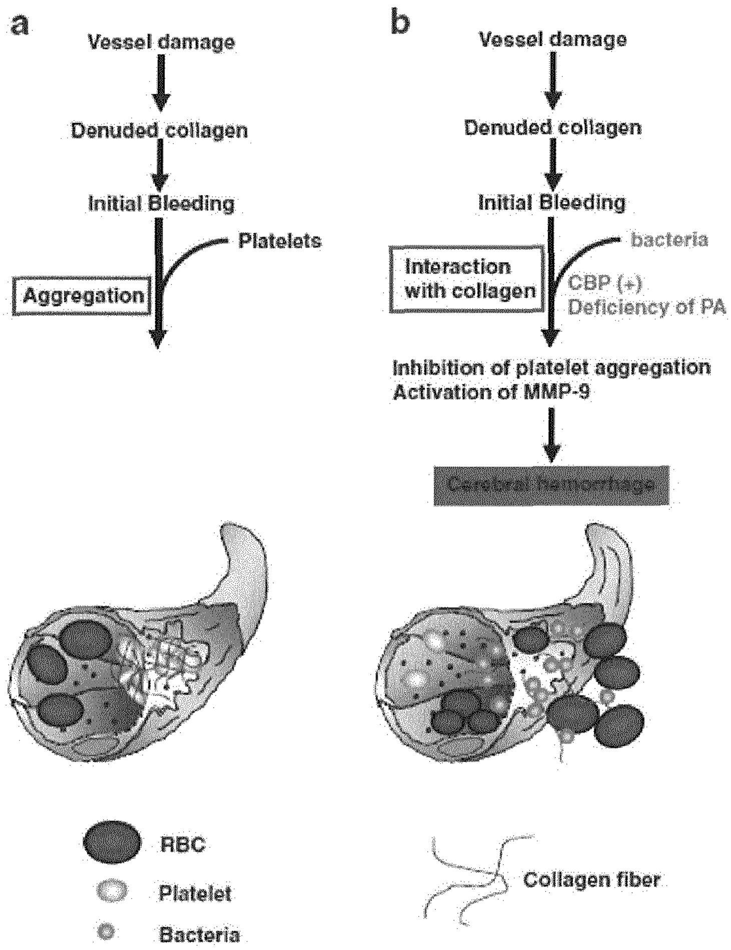
a
Vessel damage
Denuded collagen
Initial Bleeding
Platelets
Aggregation
b
Vessel damage
Denuded collagen
Initial Bleeding
bacteria
Interaction with collagen
CBP (+)
Deficiency of PA
Inhibition of platelet aggregation
Activation of MMP-9
Cerebral hemorrhage
RBC
Platelet
Bacteria
Collagen fiber

Figure 17

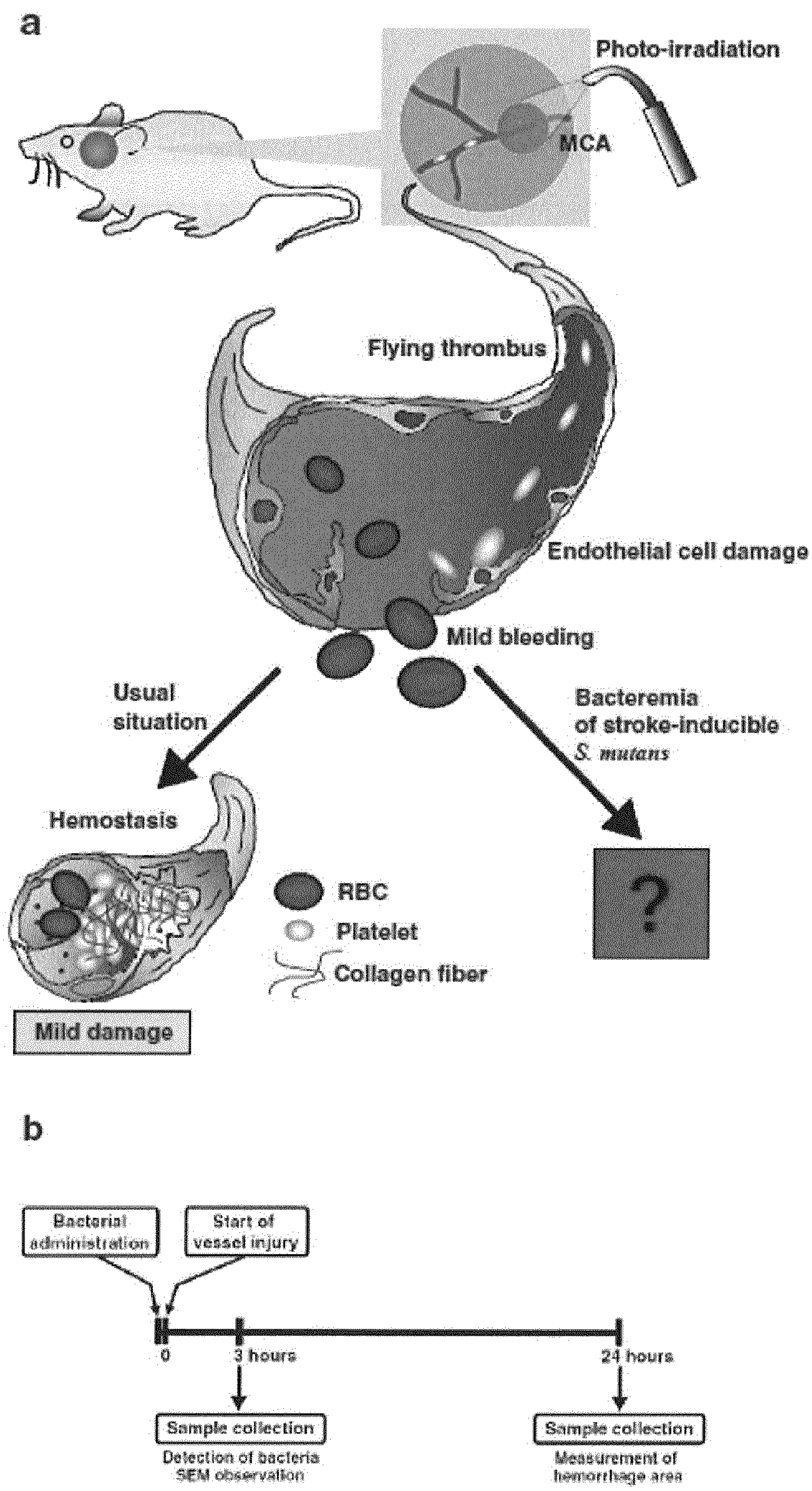
a
Photo-irradiation
MCA
Flying thrombus
Endothelial cell damage
Mild bleeding
Usual situation
Bacteremia of stroke-inducible S. mutans
Hemostasis
?
RBC
Platelet
Collagen fiber
Mild damage
b
Bacterial administration
Start of vessel injury
0
3 hours
24 hours
Sample collection
Detection of bacteria
SEM observation
Sample collection
Measurement of hemorrhage area

Figure 18. An example of analytic results

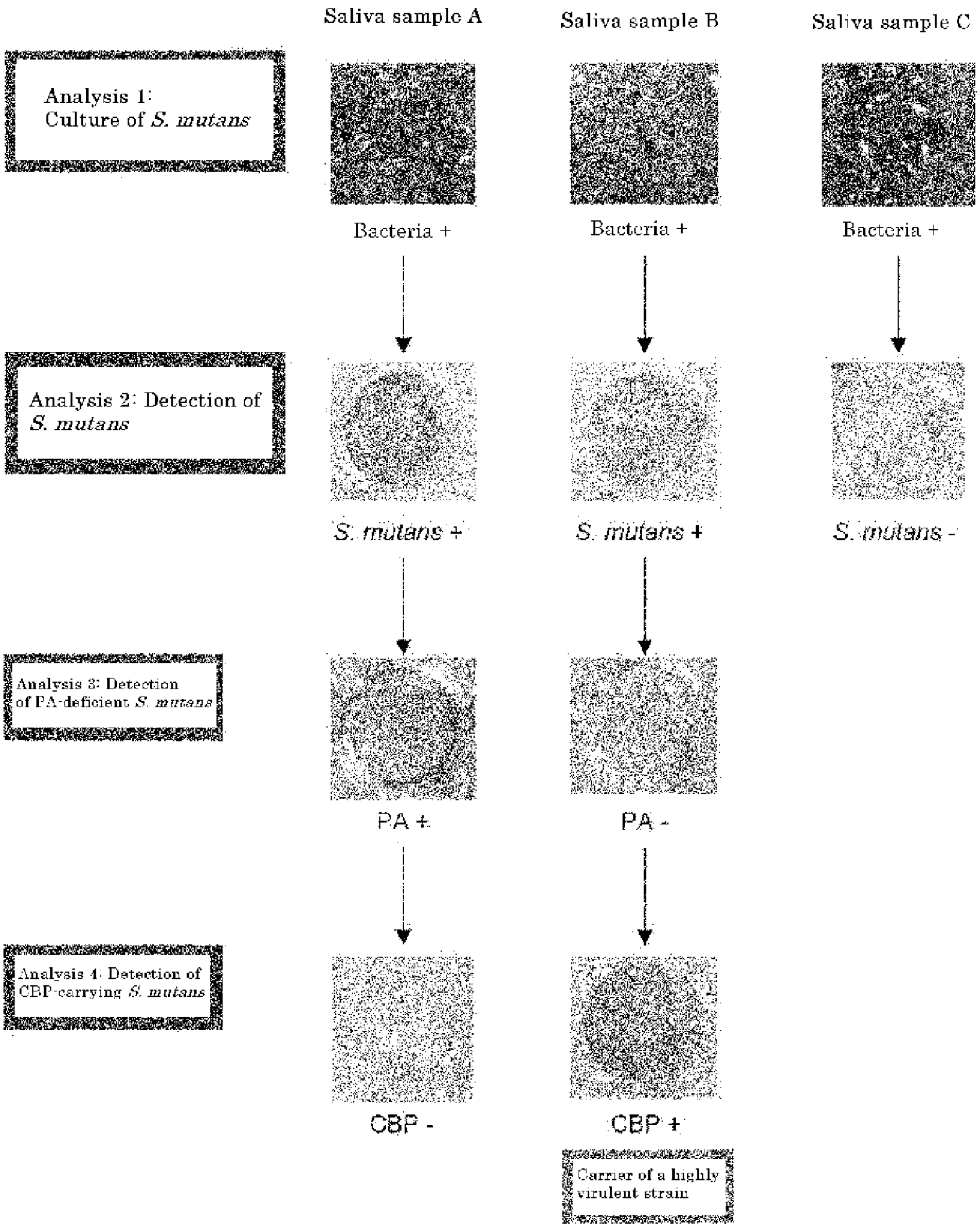

Figure 19. Consideration of optimal conditions for culturing *S. mutans* in Analysis 1

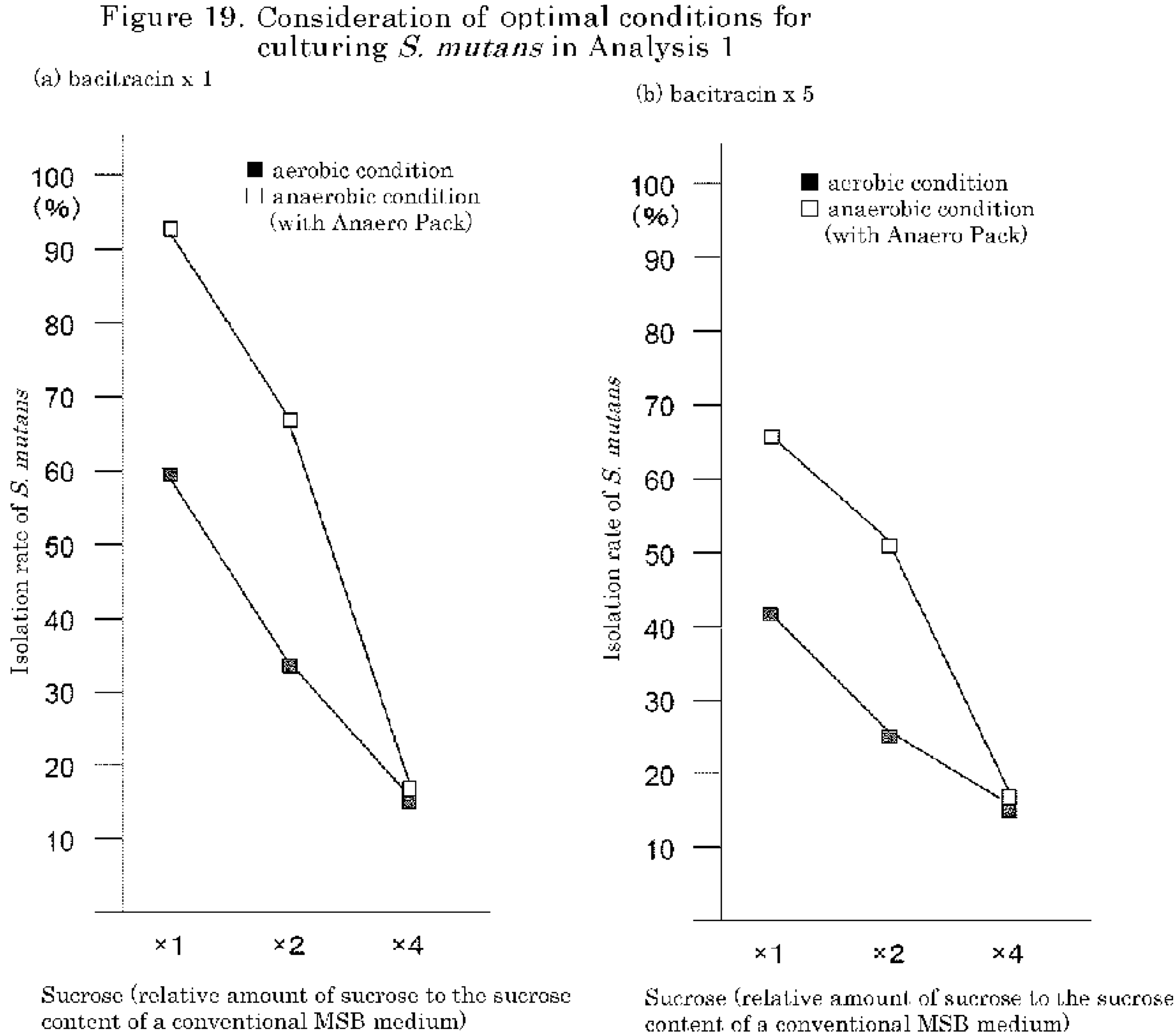

Figure 20. Consideration of possible stock period of saliva sample

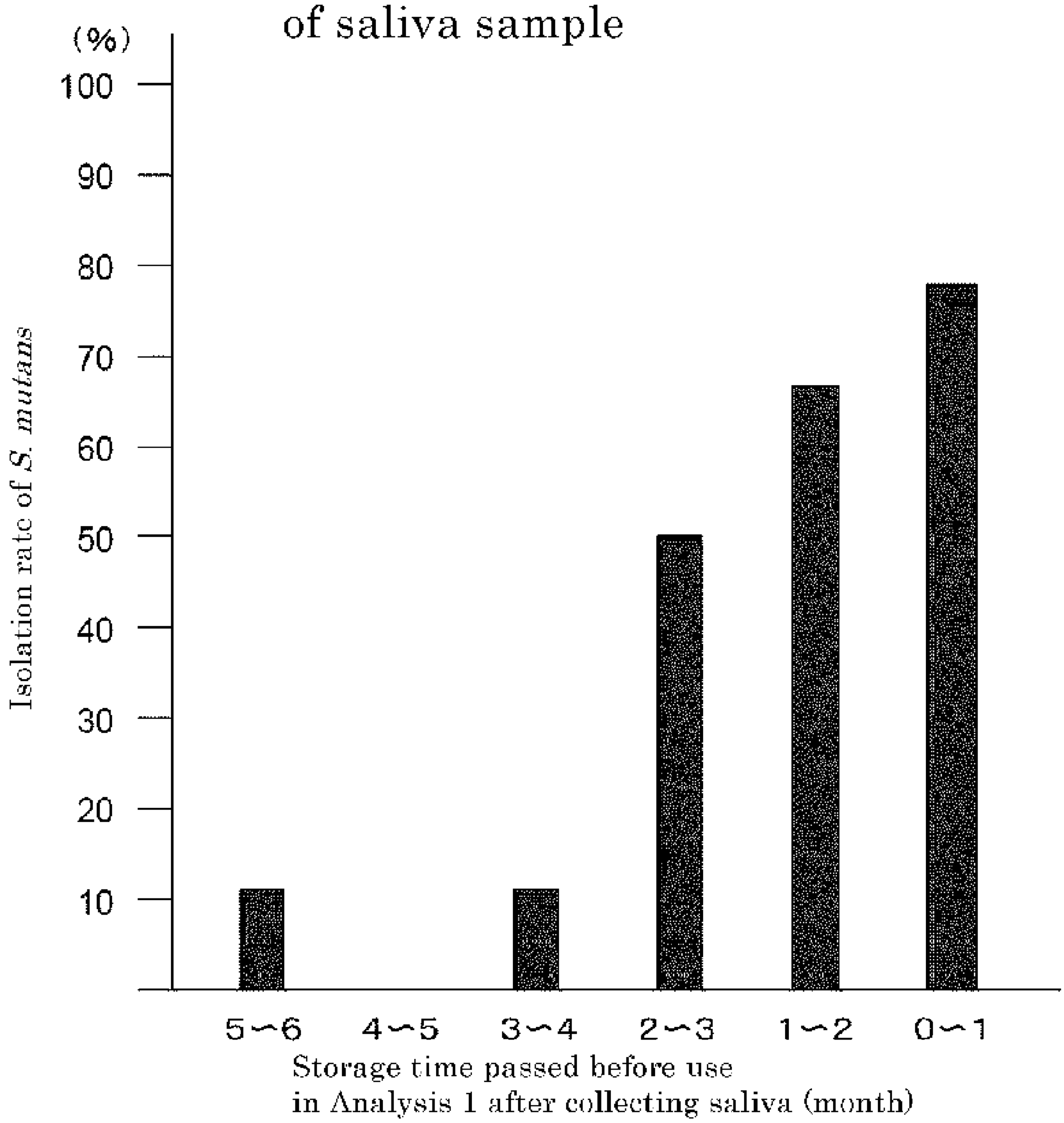

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2007502401 A
- JP 2005522669 A
- JP 2009088239 A


### Non-patent literature cited in the description


- **SATO Y et al.** *Journal of Dental Resarch, International Association for Dental Research,* 01 July 2004, vol. 83 (7), 534-539
- **NAKANO et al.** *Japanese Dental Science Review,* 2008, vol. 44, 29-37
- **MINAMI et al.** *Oral Microbiol. Immunol.,* 1990, vol. 5, 189-194
- **NAKANO et al.** *Journal of Clinical Microbiology,* 2004, vol. 42 (1), 198-202
- **NAKANO et al.** *J. Clin. Microbiol.,* 2007, vol. 45, 2614-2625
- **NAKANO, K. et al.** *J.Dent. Res.,* 2008, vol. 87, 964-968
- **MATSUMOTO-NAKANO et al.** *Oral Microbiology and Immunology,* 2008, vol. 23, 265-270
- **KAWATO et al.** *Oral Microbiology and Immunology,* 2008, vol. 23, 14-20
- **OKAHASHI et al.** *Molecular Microbiology,* 1989, vol. 3 (2), 221-228
- **NAKANO et al.** *Journal of Dental Research,* 2008, vol. 87 (10), 964-968
- **KELLY et al.** *FEBS Lett.,* 1989, vol. 258 (1), 127-132
- **ERISH et al.** *Nucleic Acid Research,* 1990, vol. 18 (15), 4596-4596
- **SATO et al.** *Journal of Dental Research,* 2004, vol. 83 (7), 534-539
- **NAKANO et al.** *J. Clin. Microbiol.,* 2007, vol. 45, 2616-2625
- **NOMURA.** *J. Med. Microbiol.,* 2009, vol. 58, 469-75
- **NOMURA et al.** *J. Med. Microbiol.,* 2009, vol. 58 (4), 469-475
- **NAKANO et al.** *Microbes and Infection,* 2006, vol. 8, 114-121
- HYOJUN YAKUZAIGAKU. Nanzando, 2003
- **NAKANO et al.** *Microbes Infect.,* 2006, vol. 8 (1), 114-21
- **MURRAY. C. J. ; LOPEZ. A. D.** Mortality by cause for eight regions of the world: Global Burden of Disease Study. *Lancet,* 1997, vol. 349, 1269-1276
- **DONNAN. G. A. ; FISHER. M. ; MACLEOD. M. ; DAVIS. S. M. STROKE.** *Lancet,* 2008, vol. 371, 1612-1623
- **BRODERICK, J. et al.** A guideline from the American Heart Association. American Stroke Association Stroke Council. High Blood Pressure Research Council, and the Quality of Care and Outcomes in Research Interdisciplinary Working Group. *Stroke,* 2007, vol. 38, 2001-2023
- **WOO. D. et al.** Genetic and environmental risk factors for intracerebral hemorrhage: preliminary results of a population-based study. *Stroke,* 2002, vol. 33, 1190-1195
- **EMSLEY. H. C. ; TYRRELL. P. J.** Inflammation and infection in clinical stroke. *J. Cereb. Blood Flow. Metab.,* 2002, vol. 22, 1399-1419
- **MOREILLON, P. ; QUE. Y. A.** Infective endocarditis. *Lancet,* 2004, vol. 363, 139-149
- **MYLONAKIS. E. ; CALDERWOOD. S. B.** Infective endocarditis in adults. *N. Engl. J. Med.,* 2001, vol. 345, 1318-1330
- **BAYER. A. S. et al.** Diagnosis and management of infective endocarditis and its complications. *Circulation,* 1998, vol. 98, 2936-2948
- **NOMURA. R. ; NAKAGAWA. I. ; HAMADA, S. ; OOSHIMA, T.** Demonstration of Streptococcus mutans with a cell wall polysaccharide specific to a new serotype. k. in the human oral cavity. *J. Clin. Microbiol.,* 2004, vol. 42, 198-202
- **EISHI. K. et al.** Surgical management of infective endocarditis associated with cerebral complications. Multi-center retrospective study in Japan. *J. Thorac. Cardiovasc. Surg.,* 1995, vol. 110, 1745-1755
- **FUJIWARA. T. et al.** Biochemical and genetic characterization of serologically untypable Streptococcus mutans Strains isolated from patients with bacteremia. *Eur. J. Oral Sci.,* 2001, vol. 109, 330-334
- **ZHAO. B. Q. et al.** Essential role of endogenous tissue plaminogen activator through matrix metalloprotease 9 induction and expression on heparin-produced cerebral hemorrhage after cerebral ischemia in mice. *Blood,* 2004, vol. 103, 2610-2616

- **GURSOY-OZDEMIR. Y. et al.** Cortical spreading depression activates and upregulates MMP-9. *J. Clin. Invest.,* 2004, vol. 113, 1447-1455
- **SATO. Y. et al.** Streptococcus mutans strains harboring collagen-binding adhesin. *J. Dent. Res.,* 2004, vol. 83, 534-539
- **NAKANO. K. et al.** Detection of novel serotype k Streptococcus mutans in infective endocarditis patients. *J. Med. Microbiol.,* 2007, vol. 56, 1413-1415
- **NAKANO. K. et al.** Streptococcus mutans clonal variation revealed by multilocus sequence typing. *J. Clin. Microbiol.,* 2007, vol. 45, 2616-2625
- **HAMPTON. J. R. ; MITCHELL. J. R. A.** Effect of aggregating agents on the electrophoretic mobility of human platelets. *Br. Med. J.,* 1966, vol. 1, 1074-1077
- **HAMPTON. J. R. ; MITCHELL. J. R. A.** Modification of the electrokinetic response of blood platelets to aggregating agents. *Nature,* 1966, vol. 210, 1000-1002
- **BOISSEAU. M. R. ; LORIENT. M. F. ; BORN. G. V. R. ; MICHAL. F.** Change in electrophoretic mobility associated with the shape change of human blood platelets. *Proc. R. Soc. Lond. B Biol Sci.,* 1977, vol. 196, 471-474
- **OKAHASHI. N. ; SASAKAWA. C. ; YOSHIKAWA. M. ; HAMADA. S. ; KOGA. T.** Cloning of a surface protein antigen gene from serotype c Streptococcus mutans. *Mol. Microbiol.,* 1989, vol. 3, 221-228
- **NAKANO. K. ; TSUJI. M. ; NISHIMURA. K. ; NOMURA. R. ; OOSHIMA. T.** Contribution of cell surface protein antigen PAc of Streptococcus mutans to bacteremia. *Microbes Infect.,* 2006, vol. 8, 114-121
- **NOMURA. R. et al.** Molecular and clinical analyses of the gene encoding the collagen-binding adhesin of Streptococcus mutans. *J. Med. Microbiol.,* 2009, vol. 58, 469-475
- **ARIESEN. M. J. ; CLANS. S. P. ; RINKEL. G. J. ; ALEGRA. A.** Risk factors for intracerebral hemorrhage in the general population: a systematic review. *Stroke,* 2003, vol. 34, 2060-2065
- **OOSHIMA. T. ; IZUNITANI. A. ; SOBUE. S. ; OKAHASHI. N. ; HAMADA. S.** Non-cariogenicity of the disaccharide palatinose in experimental dental caries of rats. *Infect. Immun.,* 1983, vol. 39, 43-49
- **NAKANO. K.** Protein antigens in serotypes k Strepococcus mutans clinical isolates. *J. Dent. Res.,* 2008, vol. 87, 964-968
- **NAKANO. K. et al.** Molecular characterization of Streptococcus mutans strains containing the cmn gene encoding a collagen-binding adhesin. *Arch. Oral Biol.*
- **WATERHOUSE. J. C. ; RUSSELL R. R. B.** Dispensable genes and foreign DNA in Streptococcus mutans. *Microbiology,* 2006, vol. 152, 1777-1788
- **SUZUKI. Y. ; NAGAI. N. ; COLLEN. D.** Comparative effects of microplasmin and tissue-type plasminogen activator (tPA) on cerebral hemorrhage in a middle cerebral artery occlusion model in mice. *J. Thromb. Haemost.,* 2004, vol. 2, 1617-1621
- **NOMURA R ; TANIGUCHI N ; LAPIRATTANAKUL J ; KOJIMA A ; NAKA S ; SENAWONGSE P ; SRISATJALUK R ; GR?NROOS L ; ALALUUSUA S ; MATSUMOTO M.** Molecular characterization of Streptococcus mutans strains containing the cnm gene encoding a collagen-binding adhesin. *Arch Oral Biol.,* 2010, vol. 55 (1), 34-9